# EUROPÄISCHE PATENTSCHRIFT

(11) **EP 0 516 588 B1**
(45) Veröffentlichungstag und Bekanntmachung des Hinweises auf die Patenterteilung: **13.07.1994**
(21) Anmeldenummer: 92810385.2
(22) Anmeldetag: 21.05.1992
(51) Int. Cl.: C07D 209/48, C07D 209/46, C07D 487/04, C07D 401/14, C07D 403/14, A61K 31/40, A61K 31/55, A61K 31/505, A61K 31/44, A61K 31/495

(54) **Substituierte Diaminophthalimide und Analoga**
Substituted diaminophtalimides and analogues
Diaminophtalimides substitués et leurs analogues

(30) Priorität: 30.05.1991 CH 1601/91
(43) Veröffentlichungstag der Anmeldung: 02.12.1992
(73) Patentinhaber: CIBA-GEIGY AG, 4002 Basel (CH)
(72) Erfinder: Trinks, Uwe, Dr., CH-4103 Bottmingen (CH); Traxler, Peter, Dr., CH-4124 Schönenbuch (CH)

(56) Entgegenhaltungen:
- EP-A- 0 152 021
- US-A- 3 857 947
- JOURNAL OF CHEMICAL RESEARCH/SYNOPSES, Band 8, 1990, Seiten 246-247
- JOURNAL OF ORGANIC CHEMISTRY, Band 32, Nr. 6, 1967, Seiten 1921-1926
- CHEMICAL ABSTRACTS, Band 85, Nr. 24, 13. Dezember 1976, Columbus, Ohio, US, Zusammenfassung-Nr. 178962h, Seite 70
- CHEMICAL ABSTRACTS, Band 109, Nr. 23, 5. Dezember 1988, Columbus, Ohio, US, Zusammenfassung-Nr. 210674t, Seite 627
- CHEMICAL ABSTRACTS, Band 106, Nr. 24, 5. Juni 1987, Columbus, Ohio, US, Zusammenfassung-Nr. 201763g, Seite 356

## Beschreibung

Die Erfindung betrifft Verbindungen der Formel I, 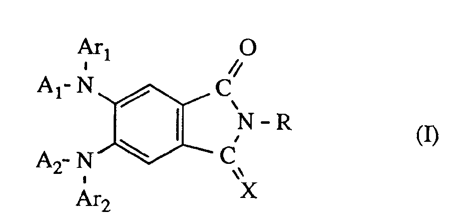
worin A₁ und A₂ unabhängig voneinander für Wasserstoff, C₁-C₇-Alkyl, C₂-C₇-Alkenyl, C₂-C₇-Alkinyl, Phenyl oder Naphthyl, welches unsubstituiert oder durch einen oder mehrere Substituenten aus der Gruppe bestehend aus C₁-C₇-Alkyl, C₂-C₇-Alkenyl, C₂-C₇-Alkinyl, C₃-C₄-Alkylen (angeknüpft an zwei benachbarten C-Atomen), C₃-C₈-Cycloalkyl, Phenyl-C₁-C₇-alkyl, Phenyl, C₁-C₇-Alkyl, das durch Hydroxy, C₁-C₇-Alkoxy, Phenyl-C₁-C₇-alkoxy, C₁-C₇-Alkanoyloxy, Halogen, Amino, C₁-C₇-Alkylamino, Di-C₁-C₇-alkylamino, Mercapto, C₁-C₇-Alkylthio, C₁-C₇-Alkylsulfinyl, C₁-C₇-Alkylsulfonyl, Carboxy, C₁-C₇-Alkoxycarbonyl, Carbamoyl, N-C₁-C₇-Alkylcarbamoyl, N,N-Di-C₁-C₇-alkylcarbamoyl und/oder Cyano substituiert ist; Hydroxy, C₁-C₇-Alkoxy, Halogen-C₁-C₇-alkoxy, Phenyl-C₁-C₇-alkoxy, Phenyloxy, C₂-C₇-Alkenyloxy, Halogen-C₂-C₇-alkenyloxy, C₂-C₇-Alkinyloxy, C₁-C₂-Alkylendioxy (angeknüpft an zwei benachbarten C-Atomen), C₁-C₇-Alkanoyloxy, Phenyl-C₁-C₇-alkanoyloxy, Phenylcarbonyloxy, Mercapto, C₁-C₇-Alkylthio, Phenyl-C₁-C₇-alkylthio, Phenylthio, C₁-C₇-Alkylsulfinyl, Phenyl-C₁-C₇-alkylsulfinyl, Phenylsulfinyl, C₁-C₇-Alkylsulfonyl, Phenyl-C₁-C₇-alkylsulfonyl, Phenylsulfonyl, Halogen, Nitro, Amino, C₁-C₇-Alkylamino, C₃-C₈-Cycloalkylamino, Phenyl-C₁-C₇-alkylamino, Phenylamino, Di-C₁-C₇-Alkylamino, N-C₁-C₇-Alkyl-N-phenylamino, N-C₁-C₇-Alkyl-N-phenyl-C₁-C₇-alkylamino, C₄-C₇-Alkylenamino, durch -O-, -S- oder -NR˝- unterbrochenes C₄-C₇-Alkylenamino (worin R˝ für Wasserstoff, C₁-C₇-Alkyl oder C₁-C₇-Alkanoyl steht), C₁-C₇-Alkanoylamino, Phenyl-C₁-C₇-alkanoylamino, Phenylcarbonylamino, C₁-C₇-Alkanoyl, Phenyl-C₁-C₇-alkanoyl, Phenylcarbonyl, Carboxy, C₁-C₇-Alkoxycarbonyl, Carbamoyl, C₁-C₇-Alkylcarbamoyl, N,N-Di-C₁-C₇-alkylcarbamoyl, N-Hydroxycarbamoyl, N-Phenylcarbamoyl, Cyano, Sulfo, C₁-C₇-Alkoxysulfonyl, Sulfamoyl, N-C₁-C₇-Alkylsulfamoyl, N,N-Di-C₁-C₇-alkylsulfamoyl oder N-Phenylsulfamoyl substituiert ist, wobei in den Substituenten vorkommende Phenylgruppen jeweils unsubstituiert oder durch C₁-C₇-Alkyl, C₁-C₇-Alkoxy, Hydroxy, Halogen und/oder Trifluormethyl substituiert sind; C₁-C₇-Alkanoyl, Halogen-C₁-C₇-alkanoyl, im Phenyl- oder Naphthylrest unsubstituiertes oder wie oben substituiertes Phenyl- oder Naphthyl-C₁-C₇-alkanoyl oder im Phenyl- oder Naphthylrest unsubstituiertes oder wie oben substituiertes Phenyl- oder Naphthyl-carbonyl, C₁-C₇-Alkylsulfonyl oder Phenyl- oder Naphthylsulfonyl, worin Phenyl oder Naphthyl unsubstituiert oder wie oben substituiert sind, stehen; oder worin A₁ und A₂ zusammen unsubstituiertes oder durch C₁-C₇-Alkyl oder Hydroxy substituiertes C₁-C₄-Alkylen bedeuten; Ar₁ und Ar₂ unabhängig voneinander unsubstituiertes oder wie oben substituiertes Phenyl oder Naphthyl; Imidazolyl, Triazolyl, Pyridyl, Pyrimidinyl oder Triazinyl, welches unsubstituiert oder durch C₁-C₇-Alkyl, Hydroxy, C₁-C₇-Alkoxy, Halogen, Cyano und/oder Trifluormethyl substituiert ist; oder unsubstituiertes oder durch C₁-C₇-Alkyl oder Hydroxy substituiertes C₃-C₈-Cycloalkyl bedeuten, die Gruppe -C(=X)- für -C(=O)-, -C(=S)-, -CH₂- oder -C(=CR₁R₂)- steht, wobei R₁ und R₂ unabhängig voneinander Wasserstoff oder C₁-C₇-Alkyl bedeuten, und R Wasserstoff, C₁-C₇-Alkyl, Aryl-C₁-C₇-alkyl oder Aryl, worin Aryl Phenyl oder Napthyl bedeutet, welches unsubstituiert oder wie oben substituiert ist, Amino, Hydroxy oder C₁-C₇-Alkoxy bedeutet, mit der Massgabe, dass R von der Bedeutung Phenyl verschieden ist, wenn A₁ und A₂ für Wasserstoff stehen, Ar₁ und Ar₂ Phenyl bedeuten und die Gruppe -C(=X)- für -C(=O)- steht, und Salze davon, sofern salzbildende Gruppen vorliegen; Verfahren zur Herstellung dieser Verbindungen, pharmazeutische Präparate, die diese Verbindungen enthalten, diese Verbindungen zur Anwendung in einem Verfahren zur therapeutischen Behandlung des menschlichen oder tierischen Körpers und die Verwendung dieser Verbindungen zur Herstellung pharmazeutischer Präparate.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7, insbesondere bis und mit 4, und vor allen Dingen mit 1 oder 2 Kohlenstoffatomen, sofern nichts anderes angegeben ist.

Niederalkyl ist vorzugsweise n-Propyl, Isopropyl, n-Butyl, Isobutyl, sek.-Butyl, tert.-Butyl, n-Pentyl, Neopentyl, n-Hexyl oder n-Heptyl, vorzugsweise Ethyl und vor allem Methyl.

Niederalkenyl hat 2 bis 7, vorzugsweise 2 bis 4 Kohlenstoffatome, und ist z. B. Allyl oder Crotyl.

Niederalkinyl hat 2 bis 7, vorzugsweise 2 bis 4 Kohlenstoffatome, und ist z. B. Propin-1- oder Propin-2-yl oder 2-Butin-1-yl

Niederalkyl, das durch Halogen substituiert ist, ist z.B. Trifluormethyl.

Phenyl- und Naphthylreste können unsubstituiert oder substituiert sein, wie nachfolgend für Phenyl angegeben. Bevorzugt ist Phenyl, das unsubstituiert oder durch einen oder mehrere, insbesondere einen oder zwei, Substituenten aus der Gruppe bestehend aus Niederalkyl, Niederalkenyl, Niederalkinyl, Niederalkylen (angeknüpft an zwei benachbarten C-Atomen), Cycloalkyl, Phenylniederalkyl oder Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy; Niederalkoxy, Halogenniederalkoxy, Phenylniederalkoxy, Phenyloxy, Niederalkenyloxy, Halogenniederalkenyloxy, Niederalkinyloxy oder Niederalkylendioxy (angeknüpft an zwei benachbarten C-Atomen); Niederalkanoyloxy, Phenylniederalkanoyloxy oder Phenylcarbonyloxy (≙ Benzoyloxy); Mercapto; Niederalkylthio, Phenylniederalkylthio, Phenylthio, Niederalkylsulfinyl [-S(=O)-Niederalkyl], Phenylniederalkylsulfinyl, Phenylsulfinyl, Niederalkylsulfonyl [-S(O₂)-Niederalkyl], Phenylniederalkylsulfonyl oder Phenylsulfonyl; Halogen, Nitro, Amino; Niederalkylamino, Cycloalkylamino, Phenylniederalkylamino oder Phenylamino; Diniederalkylamino, N-Niederalkyl-N-phenylamino, N-Niederalkyl-N-phenylniederalkylamino, Niederalkylenamino oder durch -O-, -S- oder -NR˝- unterbrochenes Niederalkylenamino (worin R˝ für Wasserstoff, Niederalkyl oder Niederalkanoyl steht); Niederalkanoylamino, Phenylniederalkanoylamino oder Phenylcarbonylamino (≙ Benzoylamino); Phenylniederalkanoyl oder Phenylcarbonyl ( = Benzoyl); Carboxy; Niederalkoxycarbonyl; Carbamoyl, N-Niederalkylcarbamoyl, N,N-Diniederalkylcarbamoyl, N-Hydroxycarbamoyl oder N-Phenylcarbamoyl; Cyano, Sulfo (SO₃H); Niederalkoxysulfonyl; Sulfamoyl (SO₂NH₂), N-Niederalkylsulfamoyl, N,N-Diniederalkylsulfamoyl oder N-Phenylsulfamoyl, substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind.

R ist vorzugsweise aus allen vorgenannten Bedeutungen ausser den im letzten Absatz genannten unsubstituierten oder substituierten Phenyl- oder Naphthylresten ausgewählt.

Von den Resten Imidazolyl, Triazolyl, Pyridyl, Pyrimidinyl oder Triazinyl ist vor allen Dingen Pyridyl bevorzugt. Diese Reste können unsubstituiert oder durch Niederalkyl, Hydroxy, Niederalkoxy, Halogen, Cyano und/oder Trifluormethyl substituiert sein.

Pyridyl ist z.B. 2-, 3- oder 4-Pyridyl.

Imidazolyl ist z.B. 2- oder 4(5)-Imidazolyl.

Triazolyl ist z.B. 1,2,4-Triazol-3- oder -4-yl oder 1,2,3-Triazol-4-yl.

Pyrimidinyl ist z.B. 2-, 4- oder 5-Pyrimidinyl.

Triazinyl ist z.B. 1,3,5-Triazin-2-yl.

Niederalkylen, gebildet aus A₁ und A₂ zusammen, ist unverzweigt und ist eine Gruppe (CH₂)ₙ, worin n für 1 bis 4, bevorzugt 2 bis 3, steht. Es ist vorzugsweise unsubstituiert, kann aber auch substituiert sein durch Niederalkyl oder Hydroxy.

Niederalkylen, angeknüpft an zwei benachbarten C-Atomen eines Benzolrings, ist C₃-C₄-Alkylen, z.B. 1,3-Propylen oder 1,4-Butylen.

Niederalkylendioxy, angeknüpft an zwei benachbarten C-Atomen, bedeutet C₁-C₂-Alkylendioxy, z.B. Methylen- oder 1,2-Ethylendioxy.

Niederalkylenamino ist C₄-C₇- und insbesondere C₄-C₅-Alkylenamino, z.B. Piperidino. Durch -O-, -S- oder -NR˝- unterbrochenes Niederalkylenamino steht für solches C₄-C₇-und insbesondere C₄-C₅-Alkylenamino, bei dem ein Ringkohlenstoffatom durch die entsprechende Heterogruppe ersetzt ist, und ist insbesondere Morpholino, Thiomorpholino, Piperazino oder 4-Niederalkyl- oder 4-Niederalkanoylpiperazino.

Niederalkanoyl, Halogenniederalkanoyl, im Phenyl- oder Naphthylrest unsubstituiertes oder wie oben substituiertes Phenyl- oder Naphthyl-niederalkanoyl oder im Phenyl- oder Naphthylrest unsubstituiertes oder wie oben substituiertes Phenyl- oder Naphthyl-carbonyl sind Acylreste. Bevorzugt hiervon ist Niederalkanoyl.

Niederalkanoyl ist vorzugsweise Formyl, Acetyl, Propionyl, n-Butyryl, Pivaloyl oder Valeroyl, insbesondere Acetyl.

Phenylniederalkyl ist insbesondere Benzyl.

Cycloalkyl ist C₃-C₈- und insbesondere C₅-C₇-Cycloalkyl, was bedeuten soll, dass es 3 bis 8 bzw. 5 bis 7 Ringkohlenstoffe enthält. Es kann aber auch substituiert sein durch Niederalkyl oder Hydroxy.

Halogen steht insbesondere für Fluor, Chlor und Brom, kann aber auch Iod bedeuten.

Salze von erfindungsgemässen Verbindungen mit salzbildenden Gruppen sind in erster Linie pharmazeutisch verwendbare, nicht-toxische Salze. Beispielsweise können Verbindungen der Formel I mit basischen Gruppen Säureadditionssalze bilden, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder mit Aminosäuren, wie Arginin oder Lysin. Verbindungen der Formel I mit einer sauren Gruppe, z.B. Carboxy, bilden z.B. Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wie Niederalkylaminen, z.B. Triethylamin, Hydroxyniederalkylaminen, z.B. 2-Hydroxyethylamin, Bis-(2-hydroxyethyl)-amin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylaminen, z.B. N,N'-Dibenzylethylendiamin, Dibenzylamin oder Benzyl-β-phenethylamin. Verbindungen der Formel I mit einer sauren und einer basischen Gruppe können auch in Form von inneren Salzen, d.h. in zwitterionischer Form, vorliegen.

Unter die Salze der erfindungsgemässen Verbindungen fallen weiterhin Komplexe von Verbindungen der Formel I (A₁, A₂ = Wasserstoff) mit Uebergangsmetallionen, z.B. Kupfer, Kobalt oder Mangan.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Die erfindungsgemässen Verbindungen weisen wertvolle, insbesondere pharmakologisch verwendbare, Eigenschaften auf. Insbesondere zeigen sie spezifische Hemmwirkungen, die von pharmakologischem Interesse sind. In erster Linie wirken sie als Protein-Tyrosinkinase-Hemmer und zeigen z.B. eine potente Hemmung der Tyrosinkinaseaktivität des Rezeptors für den epidermalen Wachstumsfaktor (EGF) und der c-erbB2-Kinase. Diese rezeptorspezifischen Enzymaktivitäten spielen eine Schlüsselrolle in der Signalübertragung in einer Vielzahl von Säugetierzellen einschliesslich Humanzellen, insbesondere von Epithelialzellen, Zellen des Immunsystems und Zellen des zentralen und peripheren Nervensystems. Die EGF-induzierte Aktivierung der rezeptorassoziierten Protein-Tyrosinkinase (EGF-R-PTK) ist bei verschiedenen Zelltypen eine Voraussetzung für die Zellteilung und damit für die Proliferation einer Zellpopulation. Durch die Zugabe von EGF-Rezeptor-spezifischen Tyrosinkinasehemmern wird somit die Vermehrung dieser Zellen gehemmt.

Die Hemmung der EGF-Rezeptor-spezifischen Protein-Tyrosinkinase (EGF-R-PTK) kann z.B. mit der Methode von C. House et al., Europ. J. Biochem. 140, 363-367 (1984) nachgewiesen werden. Die erfindungsgemässen Verbindungen hemmen die Enzymaktivität zu 50 % (IC50) in 0.1 bis 10 »M Konzentration. Sie zeigen ferner ebenfalls im mikromolaren Bereich z.B. auch eine Hemmung des Zellwachstums einer EGF-abhängigen Zellinie, etwa der epidermoiden Maus-Keratinocyten-Zellinie. Um diese Hemmung des Zellwachstums zu messen, wird die EGF-stimulierte Zellproliferation von epidermalen Balb/MK-Keratinocyten verwendet (Beschreibung des Verfahrens in Meyer, T., et al., Int. J. Cancer 43, 851 (1989). Diese Zellen sind zur Proliferation in hohem Masse auf die Gegenwart von EGF angewiesen (Weissmann, B. E., Aaronson, S. A, Cell 32, 599 (1983). Zur Durchführung des Tests werden Balb/MK-Zellen (10 000/Vertiefung) auf 96-Loch-Mikrotiterplatten übertragen und über Nacht inkubiert. Die Testsubstanzen (gelöst in DMSO) werden in in verschiedenen Konzentrationen (in Verdünnungsreihen) zugegeben, so dass die Endkonzentration an DMSO nicht grösser als 1 % ist. Nach der Zugabe werden die Platten für drei Tage inkubiert, während denen die Kontrollkulturen ohne Testsubstanz wenigstens drei Zellteilungscyclen durchlaufen können. Das Wachstum der MK-Zellen wird gemessen mittels Methylenblau-Färbung. IC₅₀-Werte werden definiert als die Konzentration der jeweiligen Testsubstanz, die zu einer 50 %-igen Abnahme im Vergleich zu den Kontrollkulturen ohne Inhibitor führt.

Die erfindungsgemässen Verbindungen hemmen neben der EGF-R-PTK auch andere Tyrosinkinasen, die in die durch trophische Faktoren vermittelte Signalübertragung involviert sind, z.B. die abl-Kinase, Kinasen aus der Familie der src-Kinasen und die c-erbB2-Kinase (HER-2), sowie Serin/Threonin-Kinasen, z.B. die Proteinkinase C, welche alle in der Wachstumsregulation und Transformation bei Säugetierzellen einschliesslich Humanzellen eine Rolle spielen.

Die Hemmung der c-erbB2-Tyrosinkinase (HER-2) kann z.B. analog der für EGF-R-PTK verwendeten Methode von C. House et al., Europ. J. Biochem. 140, 363-367 (1984) nachgewiesen werden. Die c-erbB2-Kinase kann nach bekannten Protokollen isoliert und ihre Aktivität bestimmt werden, z.B. nach T. Akiyama et al., Science 232, 1644 (1986).

Somit sind die erfindungsgemässen Verbindungen auch zur Hemmung der durch diese und verwandte Tyrosinkinasen vermittelten Prozesse geeignet.

Die erfindungsgemässen Verbindungen sind daher z.B. bei der Behandlung benigner oder maligner Tumoren nützlich. Sie sind in der Lage, Tumorregression zu bewirken und Tumormetastasierung und das Wachstum von Mikrometastasen zu verhindern. Insbesondere sind sie bei epidermaler Hyperproliferation (Psoriasis), bei der Behandlung von Neoplasien epithelialen Charakters, z.B. Mammakarzinomen, und bei Leukämien anwendbar. Ausserdem sind die Verbindungen bei der Behandlung von Erkrankungen des Immunsystems und von Entzündungen einzusetzen, soweit in diese Erkrankungen Proteinkinasen involviert sind. Auch bei der Behandlung von Erkrankungen des zentralen oder peripheren Nervensystems sind die Verbindungen einzusetzen, soweit Signalübertragung durch Proteinkinasen involviert ist.

Die erfindungsgemässen Verbindungen können sowohl alleine als auch in Kombination mit anderen pharmakologisch wirksamen Substanzen angewandt werden, z.B. zusammen mit (a) Inhibitoren der Enzyme der Polyaminsynthese, (b) Inhibitoren der Proteinkinase C, (c) Inhibitoren anderer Tyrosinkinasen, (d) Cytokinen, (e) negativen Wachstumsregulatoren, z.B. TGF-β oder IFN-β, (f) Aromatasehemmern, (g) Antiöstrogenen oder (h) Cytostatika.

Bevorzugt betrifft die Erfindung die Verbindungen der Formel I, worin A₁ und A₂ unabhängig voneinander für Wasserstoff, Niederalkyl; Niederalkenyl; Phenyl oder 1- oder 2-Naphthyl, wobei die drei letztgenannten Reste unsubstituiert oder durch Niederalkyl, Niederalkoxy, Hydroxy, Halogen und/oder Trifluormethyl substituiert sind; Niederalkanoyl, Niederalkylsulfonyl oder Phenylsulfonyl, worin die Phenylgruppe unsubstituiert oder durch Niederalkyl, Niederalkoxy, Hydroxy, Halogen und/oder Trifluormethyl substituiert ist, stehen; oder worin A₁ und A₂ zusammen Niederalkylen bedeuten; worin Ar₁ und Ar₂ unabhängig voneinander Phenyl oder Naphthyl, welches jeweils unsubstituiert oder durch einen oder mehrere Substituenten aus der Gruppe bestehend aus Niederalkyl, Niederalkenyl, Niederalkinyl, Niederalkylen (angeknüpft an zwei benachbarten C-Atomen), C₃-C₈-Cycloalkyl, Phenylniederalkyl, Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy, Niederalkoxy, Halogenniederalkoxy, Phenylniederalkoxy, Phenyloxy, Niederalkenyloxy, Halogenniederalkenyloxy, Niederalkinyloxy, Niederalkylendioxy (angeknüpft an zwei benachbarten C-Atomen), Niederalkanoyloxy, Phenylniederalkanoyloxy, Phenylcarbonyloxy, Mercapto, Niederalkylthio, Phenylniederalkylthio, Phenylthio, Niederalkylsulfinyl, Phenylniederalkylsulfinyl, Phenylsulfinyl, Niederalkylsulfonyl, Phenylniederalkylsulfonyl, Phenylsulfonyl, Halogen, Nitro, Amino, Niederalkylamino, C₃-C₈-Cycloalkylamino, Phenylniederalkylamino, Phenylamino, Diniederalkylamino, N-Niederalkyl-N-phenylamino, N-Niederalkyl-N-phenylniederalkylamino, Niederalkylenamino, durch -O-, -S- oder -NR˝- unterbrochenes Niederalkylenamino (worin R˝ für Wasserstoff, Niederalkyl oder Niederalkanoyl steht), Niederalkanoylamino, Phenylniederalkanoylamino, Phenylcarbonylamino, Niederalkanoyl, Phenylniederalkanoyl, Phenylcarbonyl, Carboxy, Niederalkoxycarbonyl, Carbamoyl, N-Niederalkylcarbamoyl, N,N-Diniederalkylcarbamoyl, N-Hydroxycarbamoyl, N-Phenylcarbamoyl, Cyano, Sulfo, Niederalkoxysulfonyl, Sulfamoyl, N-Niederalkylsulfamoyl, N,N-Diniederalkylsulfamoyl und N-Phenylsulfamoyl substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; Pyridyl oder Pyrimidinyl, das unsubstituiert oder durch Niederalkyl, Hydroxy, Niederalkoxy, Halogen, Cyano und/oder Trifluormethyl substituiert ist; oder C₃-C₈-Cycloalkyl bedeuten; die Gruppe -C(=X)- für -C(=O)-, -C(=S)-, CH₂- oder -C(=CR₁R₂)- steht, wobei R₁ und R₂ unabhängig voneinander Wasserstoff oder Niederalkyl bedeuten, und R Wasserstoff, Niederalkyl; Phenylniederalkyl, Phenyl, 1- oder 2-Naphthyl, wobei in den vier letztgenannten Resten die Phenyl- bzw. Naphthylgruppe jeweils unsubstituiert oder durch Niederalkyl, Niederalkoxy, Hydroxy, Halogen und/oder Trifluormethyl substituiert ist; Amino, Hydroxy oder Niederalkoxy bedeutet, mit der Massgabe, dass R von der Bedeutung Phenyl verschieden ist, wenn A₁ und A₂ für Wasserstoff stehen, Ar₁ und Ar₂ Phenyl bedeuten und die Gruppe -C(=X)- für -C(=O)- steht, und Salze davon, sofern salzbildende Gruppen vorliegen.

Von diesen zuletzt genannten Verbindungen der Formel I sind solche stark bevorzugt, worin R die genannten Bedeutungen ausser Phenyl hat, und die übrigen Reste die genannten Bedeutungen haben, und Salze davon, sofern salzbildende Gruppen vorliegen.

Stärker bevorzugt betrifft die Erfindung die Verbindungen der Formel I, worin A₁ und A₂ unabhängig voneinander für Wasserstoff, Niederalkyl; Phenyl oder 1- oder 2-Naphthyl, wobei die drei letztgenannten Reste unsubstituiert oder durch Niederalkyl, Niederalkoxy, Hydroxy, Halogen und/oder Trifluormethyl substituiert sind; Niederalkanoyl, Niederalkylsulfonyl oder Phenylsulfonyl, worin die Phenylgruppe unsubstituiert oder durch Niederalkyl, Niederalkoxy, Hydroxy, Halogen und/oder Trifluormethyl substituiert ist, stehen; oder worin A₁ und A₂ zusammen Niederalkylen bedeuten; worin Ar₁ und Ar₂ unabhängig voneinander Phenyl oder Naphthyl, welches jeweils unsubstituiert oder durch einen oder mehrere Substituenten aus der Gruppe bestehend aus Niederalkyl, Niederalkenyl, Niederalkinyl, Niederalkylen (angeknüpft an zwei benachbarten C-Atomen), C₃-C₈-Cycloalkyl, Phenylniederalkyl, Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy, Niederalkoxy, Halogenniederalkoxy, Phenylniederalkoxy, Phenyloxy, Niederalkenyloxy, Halogenniederalkenyloxy, Niederalkinyloxy, Niederalkylendioxy (angeknüpft an zwei benachbarten C-Atomen), Niederalkanoyloxy, Phenylniederalkanoyloxy, Phenylcarbonyloxy, Mercapto, Niederalkylthio, Phenylniederalkylthio, Phenylthio, Niederalkylsulfinyl, Phenylniederalkylsulfinyl, Phenylsulfinyl, Niederalkylsulfonyl, Phenylniederalkylsulfonyl, Phenylsulfonyl, Halogen, Nitro, Amino, Niederalkylamino, C₃-C₈-Cycloalkylamino, Phenylniederalkylamino, Phenylamino, Diniederalkylamino, N-Niederalkyl-N-phenylamino, N-Niederalkyl-N-phenylniederalkylamino, Niederalkylenamino, durch -O-, -S- oder -NR˝- unterbrochenes Niederalkylenamino (worin R˝ für Wasserstoff, Niederalkyl oder Niederalkanoyl steht), Niederalkanoylamino, Phenylniederalkanoylamino, Phenylcarbonylamino, Niederalkanoyl, Phenylniederalkanoyl, Phenylcarbonyl, Carboxy, Niederalkoxycarbonyl, Carbamoyl, N-Niederalkylcarbamoyl, N,N-Diniederalkylcarbamoyl, N-Hydroxycarbamoyl, N-Phenylcarbamoyl, Cyano, Sulfo, Niederalkoxysulfonyl, Sulfamoyl, N-Niederalkylsulfamoyl, N,N-Diniederalkylsulfamoyl und N-Phenylsulfamoyl substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; Pyridyl, das unsubstituiert oder durch Niederalkyl, Hydroxy, Niederalkoxy, Halogen, Cyano und/oder Trifluormethyl substituiert ist; oder C₃-C₈-Cycloalkyl bedeuten; die Gruppe -C(=X)- für -C(=O)-, -C(=S)-, -CH₂- oder -C(=CR₁R₂)- steht, wobei R₁ und R₂ unabhängig voneinander Wasserstoff oder Niederalkyl bedeuten, und R Wasserstoff, Niederalkyl; Phenylniederalkyl, Phenyl, 1- oder 2-Naphthyl, wobei in den vier letztgenannten Resten die Phenyl- bzw. Naphthylgruppe jeweils unsubstituiert oder durch Niederalkyl, Niederalkoxy, Hydroxy, Halogen und/oder Trifluormethyl substituiert ist; Amino, Hydroxy oder Niederalkoxy bedeutet, mit der Massgabe, dass R von der Bedeutung Phenyl verschieden ist, wenn A₁ und A₂ für Wasserstoff stehen, Ar₁ und Ar₂ Phenyl bedeuten und die Gruppe -C(=X)- für -C(=O)- steht, und Salze davon, sofern salzbildende Gruppen vorliegen.

Besonders bevorzugt betrifft die Erfindung die im vorigen Absatz erwähnten Verbindungen der Formel I, worin A₁ und A₂ unabhängig voneinander Wasserstoff oder Niederalkyl bedeuten, die Gruppe -C(=X)- für -C(=O)-, -C(=S)- oder -C(=CH₂)- steht, und R Wasserstoff oder Niederalkyl bedeutet, und Salze davon, sofern salzbildende Gruppen vorliegen.

In noch stärker bevorzugter Weise betrifft die Erfindung die Verbindungen der Formel I, worin A₁ und A₂ unabhängig voneinander für Wasserstoff; Niederalkyl; Niederalkenyl; oder Niederalkanoyl stehen; oder worin A₁ und A₂ zusammen Niederalkylen bedeuten; worin Ar₁ und Ar₂ unabhängig voneinander Phenyl oder Naphthyl, welches jeweils unsubstituiert oder durch einen oder mehrere Substituenten aus der Gruppe bestehend aus Niederalkyl, Niederalkylen (angeknüpft an zwei benachbarten C-Atomen), Hydroxy, Phenyloxy, Halogen, Nitro, Amino, Niederalkylamino, Diniederalkylamino, Niederalkanoylamino, Carboxy, Niederalkoxycarbonyl, Carbamoyl, N-Niederalkylcarbamoyl, N,N-Diniederalkylcarbamoyl und Cyano substituiert ist; Pyridyl; Pyrimidinyl; oder C₃-C₈-Cycloalkyl bedeuten; die Gruppe -C(=X)- für -C(=O)- oder -C(=S)- steht, und R Wasserstoff, Niederalkyl; Phenylniederalkyl, Amino oder Hydroxy bedeutet, und Salze davon, sofern salzbildende Gruppen vorliegen.

In erster Linie betrifft die Erfindung die Verbindungen der Formel I, worin A₁ und A₂ unabhängig voneinander für Wasserstoff oder Methyl stehen; oder worin A₁ und A₂ zusammen -(CH₂)₂- oder -(CH₂)₃- bedeuten; Ar₁ und Ar₂ unabhängig voneinander für Phenyl oder Naphthyl, welches jeweils unsubstituiert oder durch einen oder mehrere Substituenten aus der Gruppe bestehend aus Niederalkyl, Niederalkoxy, Phenylniederalkoxy, Hydroxy, Niederalkanoyloxy, Nitro, Amino, Niederalkylamino, Diniederalkylamino, Niederalkanoylamino, Halogen, Trifluormethyl, Carboxy, Niederalkoxycarbonyl, Carbamoyl, N-Niederalkylcarbamoyl, N,N-Diniederalkylcarbamoyl, Cyano, Niederalkanoyl, Benzoyl, Niederalkoxysulfonyl und Sulfamoyl, N-Niederalkylsulfamoyl und N,N-Diniederalkylsulfamoyl substituiert ist; oder für Cyclopentyl, Cyclohexyl oder Pyridyl stehen; die Gruppe -C(=X)- für -C(=O)-, -C(=S)- oder -C(=CH₂)- steht, und R Wasserstoff oder Niederalkyl bedeutet, und pharmazeutisch anwendbare Salze davon.

Vor allen Dingen betrifft die Erfindung die Verbindungen der Formel I, worin A₁ und A₂ für Wasserstoff stehen; Ar₁ und Ar₂ unabhängig voneinander Phenyl, das unsubstituiert oder durch Niederalkyl, Trifluormethyl, Phenyl, Hydroxy, Niederalkoxy, Benzyloxy, Amino, Diniederalkylamino, Niederalkanoylamino, Halogen, Carboxy, Niederalkoxycarbonyl, Carbamoyl, N,N-Diniederalkylcarbamoyl oder Cyano substituiert ist; oder Cyclohexyl bedeuten; die Gruppe -C(=X)- für -C(=O)-, -C(=S)- oder -C(=CH₂)- steht, und R Wasserstoff bedeutet, und pharmazeutisch anwendbare Salze davon.

Die Erfindung betrifft vor allem die in den Beispielen beschriebenen spezifischen Verbindungen und pharmazeutisch anwendbare Salze davon.

Die Verbindungen der Formel I können in an sich bekannter Weise hergestellt werden, indem man z.B.
(a) eine Verbindung der Formel II, 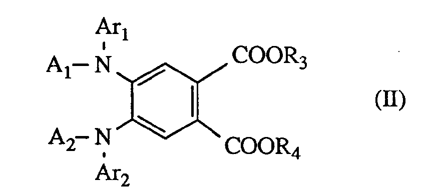 worin Ar₁, Ar₂, A₁ und A₂ die unter Formel I angegebene Bedeutung haben und R₃ und R₄ unabhängig voneinander Aryl oder Niederalkyl bedeuten, mit einer Verbindung der Formel III,

   H₂N-R (III)

   worin R die unter Formel I angegebene Bedeutung hat, umsetzt, oder
(b) eine Verbindung der Formel IV, 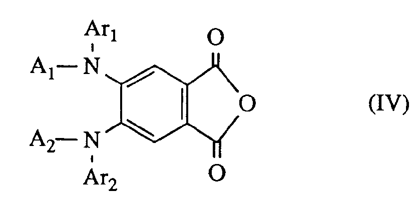 worin Ar₁, Ar₂, A₁ und A₂ die unter Formel I angegebene Bedeutung haben, mit einer Verbindung der Formel III,

   H₂N-R (III)

   worin R die unter Formel I angegebene Bedeutung hat, umsetzt;

und gewünschtenfalls eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I in ein Salz umwandelt und/oder ein erhaltenes Gemisch von isomeren Verbindungen der Formel I in die einzelnen Isomeren auftrennt.

In der folgenden näheren Beschreibung der Verfahren haben die Symbole Ar₁, Ar₂, A₁, A₂, X, R, R₃ und R₄ jeweils die unter Formel I und II angegebene Bedeutung, sofern nichts anderes angegeben ist.

Verfahren (a): Die Umsetzung nach dem Verfahren (a) entspricht der an sich bekannten Aminolyse von Phthalsäurediestern mit Ammoniak oder primären Aminen. Die Umsetzung verläuft mit aktivierten Phthalsäurediestern, z.B. dem Di(p-nitrophenyl)ester, normalerweise bei Raumtemperatur, mit Diniederalkylestern dagegen meist nur bei hohen Temperaturen. Bevorzugt wird der Dimethylester verwendet. Bevorzugt ist die Umsetzung von Diniederalkylestern in einem Lösungsmittel, insbesondere einem hochsiedenden Alkohol, z. B. einem Diol, wie Ethylenglykol, bei Temperaturen von 100 bis 150 °C, z.B. von etwa 120 °C, oder die Umsetzung der Niederalkylester mit Ammoniak oder dem betreffenden Amin der Formel II bei denselben Temperaturen in Anwesenheit eines Lösungsmittels, z. B. eines Alkoholes, wie eines Niederalkanoles, z. B. Methanol oder Ethanol, oder ferner in Abwesenheit eines Lösungsmittels, in einem Autoklaven bei erhöhtem Druck.

Die Ausgangsverbindungen der Formel II werden z.B. dadurch hergestellt, dass man ein Cyclohexadien der Formel V 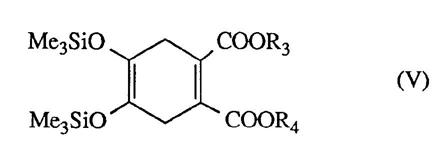
worin Me für Methyl steht, mit einem Anilin der Formel VI

AHN-Ar (VI)

worin A insbesondere Wasserstoff oder Niederalkyl bedeutet, unter Säurekatalyse umsetzt [s. Matlin, Stephen A. and Barron, Kenneth, J. Chem. Res. Synop. 8, 246-247 (1990)].

Die Herstellung der Verbindungen der Formel V erfolgt z.B. via einer Diels-Alder-Reaktion und ist ebenfalls in der angegebenen Literatur beschrieben.

Zur Herstellung von unsymmetrischen Verbindungen der Formel II, worin A₁ und A₂ und/oder Ar₁ und Ar₂ verschieden sind, können z.B. Verbindungen der Formel V mit zwei unterschiedlichen Verbindungen der Formel VI - z.B. stufenweise - umgesetzt und die gewünschten Verbindungen der Formel II durch eine chromatographische Trennung isoliert werden.

Weiterhin können z.B. Verbindungen der Formel II mit A₁ = A₂ = H im Verhältnis 1:1 mit einem Niederalkylierungsmittel, z.B. Methyliodid, umgesetzt werden, wobei man unsymmetrische Verbindungen der Formel II mit A₁ = Niederalkyl und A₂ = H erhält. Setzt man das Niederalkylierungsmittel im Ueberschuss, z.B. 10:1, ein, so erhält man symmetrische Verbindungen der Formel II mit A₁ = A₂ = Niederalkyl.

Verfahren (b): Die Umsetzung nach dem Verfahren (b) entspricht der an sich bekannten Aminolyse von Phthalsäureanhydriden z.B. mit Ammoniak oder primären Aminen bei höheren Temperaturen oder mit Hexamethyldisilazan und Methanol bei Raumtemperatur [Davis, Peter D. and Bit, Rino A., Tetrahedron Lett. 31, 5201-5204 (1990)].

Die Ausgangsverbindungen der Formel IV werden z.B. dadurch hergestellt, dass man eine Verbindung der Formel VII 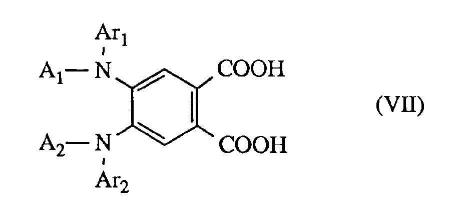
mit einem Säureanhydrid der Formel VIII, (R₅CO)(R₅'CO)O, umsetzt, worin R₅ und R₅' unabhängig voneinander für Wasserstoff oder Niederalkyl, nicht jedoch beide für Wasserstoff, stehen.

Die Verbindungen der Formel VII sind z.B. durch Hydrolyse, z.B. im sauren oder alkalischen Medium, einer entsprechenden Verbindung der Formel II zugänglich.

Verbindungen der Formel I können in an sich bekannter Weise in andere Verbindungen der Formel I umgewandelt werden.

Zum Beispiel kann eine Verbindung der Formel I, worin die Gruppe -C(=X)- für -C(=O)- steht, mit einem geeigneten Reagens umgesetzt werden, so dass eine andere Verbindung der Formel I, worin die Gruppe -C(=X)- für -C(=S)-, -CH₂- oder -C(=CR₁R₂)- steht, erhalten wird. Ein geeignetes Reagens für die Ueberführung von -C(=O)- in -C(=S)- ist z.B. das Lawesson-Reagens (= 2,4-Bis-(4-methoxyphenyl)-2,4-dithioxo-1,3,2,4-dithiaphosphetan), wobei die Reaktion beispielsweise in einem halogenierten Kohlenwasserstoff, wie Dichlormethan, bei Temperaturen von 30 °C bis zur Rückflusstemperatur, insbesondere bei Rückflusstemperatur, durchgeführt wird. Zur Umwandlung von -C(=O)- in -CH₂- sind beispielsweise die Systeme LiAlH₄/Tetrahydrofuran oder Zinkamalgam/HCl/Ethanol geeignet. Die Ueberführung von -C(=O)- in -C(=CR₁R₂)- gelingt z.B. durch die Umsetzung mit einer starken Base, z.B. LDA (Lithiumdiisopropylamid) und dann einem Grignard-Reagens der Formel HCR₁R₂MgHal (Hal = Halogen, z.B. Iod).

Weiterhin können z.B. Verbindungen der Formel I, worin R Wasserstoff bedeutet, durch Alkylierung, z.B. mit Niederalkyl- oder Arylniederalkylhalogeniden nach Behandlung mit geeigneten Basen, etwa Natriumhydrid oder Kalium-tert.-butoxid, in andere Verbindungen der Formel I, worin R für Niederalkyl oder Arylniederalkyl steht, überführt werden, wobei Aryl hier jeweils unsubstituiertes oder wie oben substituiertes Phenyl oder Naphthyl bedeutet.

Ferner können z.B. Verbindungen der Formel I, worin A₁ und/oder A₂ Wasserstoff bedeuten, durch Umsetzung mit geeigneten Reagentien in andere Verbindungen der Formel I, worin A₁ und/oder A₂ andere oben genannte Bedeutungen als Wasserstoff haben, überführt werden.

Zur Einführung von A₁ und/oder A₂ = Niederalkyl kommt z.B. die Behandlung mit der Base LDA und anschliessende Umsetzung mit einem Diniederalkylether oder einem Niederalkylhalogenid in Frage. Unter diesen Bedingungen wird eine gegebenenfalls im Molekül vorhandene Gruppe -N(-R)- = -NH- nur wenig oder gar nicht alkyliert.

Zur Einführung von A₁ und/oder A₂ = Acyl, Niederalkylsulfonyl oder unsubstituierte4s oder wie oben substituiertes Phenyl- oder Naphthylsulfonyl kann die Ausgangsverbindung z.B. ebenfalls zunächst mit LDA und dann mit einem Acylierungsmittel, z.B. Acetylchlorid, bzw. einem die Niederalkylsulfonyl- oder Arylsulfonylgruppe einführenden Mittel, z.B. Methylsulfonylchlorid oder p-Toluolsulfonsäurechlorid, umgesetzt werden.

Verbindungen der Formel I, worin Ar₁ und/oder Ar₂ durch Halogen, vorzugsweise Brom, substituiertes Phenyl oder Naphthyl bedeutet, können in die entsprechenden Derivate umgewandelt werden, in denen eines oder alle der in den genannten Resten Ar₁ und/oder Ar₂ vorhandenen Halogenatome durch Cyano ersezt sind, beispielsweise durch Umsetzung mit einem Cyanidsalz eines Übergangsmetalles, insbesondere CuCN, bei Temperaturen zwischen 50 und 150 °C, vorzugsweise zwischen 60 und 140 °C, in einem inerten polaren Lösungsmittel, wie einem N,N-Diniederalkylniederalkancarbonsäureamid, z. B. Dimethylformamid, ohne oder mit anschliessender Zugabe eines Katalysators, z. B. eines Übergangsmetallhalogenides, wie Eisen(III)chlorid, in wässriger Lösung, (siehe auch Rosenmund et al., Ber. **52**, 1749 (1916); von Braun et al., Ann. 488, 111 (1931).

In Verbindungen der Formel I können die Reste Ar₁ und/oder Ar₂, welche unsubstituiertes oder wie oben definiert substituiertes Phenyl oder Naphthyl, unabhängig voneinander unter Einführung einer oder mehrerer Nitrogruppen nitriert werden, beispielsweise unter üblichen Bedingungen zur Einführung einer Nitrogruppe in Aromaten, z. B. mit konzentrierter oder 100 % Salpetersäure bei Temperaturen ziwschen 0 und 100 °C, vorzugsweise zwischen 10 und 40 °C, in einem inerten Lösungsmittel, z. B. einem organischen Säureanhydrid, wie Acetanhydrid. Entstehen dabei mehrere verschiedene Produkte mit unterschiedlicher Position und Anzahl der Nitrogruppen, so können diese nach üblichen Methoden, beispielsweise säulenchromatographisch, getrennt werden.

Nitrosubstituenten innerhalb der Reste Ar₁ und/oder Ar₂ können zu Aminogruppen reduziert werden, beispielsweise durch Hydrierung unter üblichen Bedingungen, z. B. durch Hydrierung in Gegenwart eines zur selektiven Reduktion der Nitrogruppen geeigneten Hydrierkatalysators, wie Raney-Nickel, in einem inerten Lösungsmittel, z. B. einem cyclischen oder acyclischen Ether, wie Tetrahydrofuran, unter Normaldruck oder bis zu 5 bar erhöhtem Druck.

Verbindungen der Formel I mit veretherten Hydroxygruppen, beispielsweise mit Niederalkoxyresten als Substituenten innerhalb von Ar₁ und/oder Ar₂, können durch Etherspaltung in die entspechenden Hydroxy-substituierten Verbindungen der Formel I überführt werden. Die Etherspaltung erfolgt unter an sich bekannten Bedingungen, beispielsweise in Gegenwart von Halogenwasserstoffsäuren, wie Bromwasserstoff oder Iodwasserstoff, in An- oder Abwesenheit von Lösungsmitteln, wie Carbonsäuren, z. B. Niederalkancarbonsäuren, wie Essigsäure, bei Temperaturen zwischen 20 °C und der Rückflusstemperatur des Reaktionsgemisches, oder vorzugsweise unter schonenden Bedingungen mit Borhalogeniden, insbesondere Bortribromid, in einem inerten Lösungsmittel, wie einem chlorierten Kohlenwasserstoff, z. B. Methylenchlorid oder Chloroform, bei Temperaturen zwischen -80 und 0 °C, insbesondere zwischen - 50 und -20 °C.

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -80°C bis etwa 200°C, vorzugsweise von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen, sofern die Verbindungen salzbildende Gruppen, z.B. basische Gruppen, wie Amino- oder Iminogruppen, auch solche, die an nicht mehr als ein ungesättigtes Kohlenstoffatom, wie die Gruppen -NA₁Ar₁ und/oder -NA₂Ar₂ am C-Atom des zentralen Phenylringes Rest, worin Ar₁ und A₁ und/oder Ar₂ und A₂ nicht über ein ungesättigtes Kohlenstoffatom gebunden sind, und/oder saure Gruppen, wie Carboxy oder Sulfo (SO₃H), enthalten.

Die Verbindungen, einschliesslich ihrer Salze, können auch in Form von Hydraten erhalten werden, oder ihre Kristalle können z.B. das zur Kristallisation verwendete Lösungsmittel einschliessen.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Bevorzugt ist eine pharmazeutische Zusammensetzung, welche geeignet ist zur Verabreichung an einen Warmblüter, insbesondere Menschen, der an einer Erkrankung leidet, die auf eine Hemmung einer Proteinkinase anspricht, beispielsweise Psoriasis oder ein Tumor, umfassend eine zur Hemmung der Proteinkinase wirksame Menge einer Verbindung der Formel I oder eines Salzes davon, falls salzbildende Gruppen vorliegen, zusammen mit mindestens einem pharmazeutisch annehmbaren Trägermaterial.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zur parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Verbindungen der Formel I sind verwendbar in Verfahren zur Behandlung der oben genannten Krankheitszustände, insbesondere solcher, die auf eine Hemmung von Proteinkinasen ansprechen. Die Verbindungen der vorliegenden Erfindung können prophylaktisch oder therapeutisch verabreicht werden, vorzugsweise in einer gegen die genannten Krankheiten wirksamen Menge an einen Warmblüter, z. B. Menschen, der einer derartigen Behandlung bedarf, wobei man sie vorzugsweise in Form von pharmazeutischen Präparaten verwendet. Dabei wird bei einem Körpergewicht von etwa 70 kg eine tägliche Dosis von etwa 0,1 g bis etwa 5 g, vorzugsweise von etwa 0,5 g bis etwa 2 g, einer Verbindung der vorliegenden Erfindung verabreicht.

Die nachfolgenden Beispiele illustrieren die vorliegende Erfindung; Temperaturen werden in Grad Celsius angegeben. Folgende Abkürzungen werden verwendet: Ether ≙ Diethylether; Ethylacetat ≙ Essigsäureethylester, THF = Tetrahydrofuran; DMF = N,N-Dimethylformamid; RT ≙ Raumtemperatur; MS ≙ Massenspektrum; FAB ≙ Fast Atom Bombardment.

### Beispiel 1: 4,5-Bis(anilino)phthalimid

Eine Suspension von 230 mg (0,7 mmol) 4,5-Bis(anilino)phthalsäuredimethylester in 23 ml Ethylenglykol wird auf 120° erwärmt; unter Rühren wird während 24 h Ammoniak-Gas durchgeleitet. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan/Methanol 40:1 an Kieselgel chromatographiert, die Produktfraktionen vereinigt und eingedampft. Auf diese Weise wird die Titelverbindung in Form gelber Kristalle erhalten, Smp. 215-217°, FAB-MS: 330 [M⁺+H].

### a) 4,5-Bis(trimethylsilyloxy)cyclohexa-1,4-dien-1,2-dicarbonsäuredimethylester

Unter Argon wird eine Lösung von 7,1 g (50 mmol) Acetylendicarbonsäuredimethylester in 30 ml Toluol zu 12,5 g (50 mmol) 2,3-Bis(trimethylsilyloxy)-1,3-butadien (95%) getropft und anschliessend 19 h am Rückfluss gekocht. Das Reaktionsgemisch wird abgekühlt, das Lösungsmittel abgedampft und der Rückstand im Hochvakuum (0,1 mbar, 124-127°) destilliert. Auf diese Weise wird die Titelverbindung als gelbes, hochviskoses Oel erhalten, ¹H-NMR (CDCl₃): δ = 0.18 (s, 18H), 3.09 (s, 4H), 3.78 (s, 6H).

### b) 4,5-Bis(anilino)phthalsäuredimethylester

Eine Lösung von 5,6 g (15 mmol) 4,5-Bis(trimethylsilyloxy)cyclohexa-1,4-dien--1,2-dicarbonsäuredimethylester und 5,5 ml (60 mmol) Anilin in 60 ml Eisessig wird während 4 h am Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Das Rohprodukt wird aus Ethanol umkristallisiert. Auf diese Weise wird die Titelverbindung in Form gelber Kristalle erhalten, Smp. 178°, FAB-MS: 377 [M⁺+H].

### Beispiel 2: 5,8-Diphenyl-5,8-diaza-5,6,7,8-tetrahydro-naphthalin-2,3-dicarbonsäureimid

Analog zu Beispiel 1 werden 40 mg (0.1 mmol) 5,8-Diphenyl-5,8-diaza-5,6,7,8-tetrahydro-naphthalin-2,3-dicarbonsäuredimethylester in 4 ml Ethylenglykol auf 120° erwärmt, wobei unter Rühren während 24 h Ammoniak-Gas durchgeleitet wird. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan/Methanol 20:1 an Kieselgel chromatographiert, die Produktfraktionen vereinigt und eingedampft. Auf diese Weise wird die Titelverbindung in Form gelber Kristalle erhalten, FAB-MS: 356 [M⁺+H].

### a) 5,8-Diphenyl-5,8-diaza-5,6,7,8-tetrahydro-naphthalin-2,3-dicarbonsäuredimethylester

Eine Lösung von 2,24 g (6 mmol) 4,5-Bis(trimethylsilyloxy)cyclohexa-1,4-dien--1,2-dicarbonsäuredimethylester (Beispiel 1a) und 5,1 g (24 mmol) N,N'-Diphenylethylendiamin in 24 ml Eisessig werden während 2 h am Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Das Rohprodukt wird mit Hexan/Ethylacetat 3:1 an Kieselgel chromatographiert, die Produktfraktionen eingedampft und der Rückstand aus Ethanol umkristallisiert. Auf diese Weise wird die Titelverbindung in Form oranger Kristalle erhalten, FAB-MS: 402 [M⁺], 403 [M⁺+H].

### Beispiel 3: 4,5-Bis(4-fluoranilino)phthalimid

Analog zu Beispiel 1 werden 290 mg (0,7 mmol) 4,5-Bis(4-fluoranilino)phthalsäuredimethylester in 22 ml Ethylenglykol auf 120° erwärmt und unter Rühren während 18 h Ammoniak-Gas durchgeleitet. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Hexan/Ethylacetat 1:1 an Kieselgel chromatographiert, die Produktfraktionen vereinigt und eingedampft. Auf diese Weise wird die Titelverbindung in Form oranger Kristalle erhalten, Smp. > 220°C, FAB-MS: 366 [M⁺+H].

### a) 4,5 -Bis(4-fluoranilino)phthalsäuredimethylester

Eine Lösung von 2,4 g (6 mmol) 4,5-Bis(trimethylsilyloxy)cyclohexa-1,4-dien- -1,2-dicarbonsäuredimethylester (Beispiel 1a) und 2,3 ml (24 mmol) 4-Fluoranilin in 60 ml Eisessig wird während 2 h am Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Der Eindampfrückstand wird mit Ethylacetat/Hexan 2:1 an Kieselgel chromatographiert, die Produktfraktionen eingedampft und aus Ethylacetat/Hexan umkristallisiert. Auf diese Weise wird die Titelverbindung in Form gelber Kristalle erhalten, ¹H-NMR (CDCl₃): δ = 7.40 (s, 2H), 7.10-6.80 (m, 8H), 5.70 (br s, 2H), 3.83 (s, 6H).

### Beispiel 4: 4,5-Bis(4-benzyloxy-anilino)phthalimid

Analog zu Beispiel 1 werden 294,4 mg (0.5 mmol) 4,5-Bis(4-benzyloxy-anilino)phthalsäuredimethylester in 22 ml Ethylenglykol auf 120° erwärmt, wobei unter Rühren während 16 h Ammoniak-Gas durchgeleitet wird. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan/Methanol 50:1 an Kieselgel chromatographiert, die Produktfraktionen vereinigt und eingedampft. Auf diese Weise wird die Titelverbindung in Form roter Kristalle erhalten, Smp. 187-9°C, FAB-MS: 542 [M⁺+H].

### a) 4,5-Bis(4-benzyloxy-anilino)phthalsäuredimethylester

Eine Lösung von 2,4 g (6 mmol) 4,5-Bis(trimethylsilyloxy)cyclohexa-1,4-dien--1,2-dicarbonsäuredimethylester (Beispiel 1a) und 4,8 g (24 mmol) 4-Benzyloxy-anilin in 24 ml Eisessig wird während 2 h am Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Der Eindampfrückstand wird heiss in Ethylacetat gelöst und filtriert, dann lässt man ihn bei 0° kristallisieren. Der kristalline Rückstand wird mit Ethylacetat/Hexan 3:2 an Kieselgel chromatographiert, die Produktfraktionen eingedampft und aus Ethylacetat/Hexan umkristallisiert. Auf diese Weise wird die Titelverbindung in Form beiger Kristalle erhalten, FAB-MS: 589 [M⁺+H].

### Beispiel 5: 4,5-Bis[4-(N,N-diethylamino)-anilino]phthalimid-bishydrochlorid

Analog zu Beispiel 1 werden 294,4 mg (0,5 mmol) 4,5-Bis[4-(N,N-diethylamino)-anilino]phthalsäuredimethylester in 22 ml Ethylenglykol auf 120° erwärmt, wobei unter Rühren während 22 h Ammoniak-Gas durchgeleitet wird. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan/Methanol 30:1 an Kieselgel chromatographiert, die Produktfraktionen vereinigt und eingedampft. Der rote kristalline Eindampfrückstand wird in Dichlormethan gelöst und mit 4,1 N HCl (g) in Ether versetzt. Der kristalline Niederschlag wird abfiltriert und getrocknet. Auf diese Weise wird die Titelverbindung in Form gelber Kristalle erhalten, Smp. 228-30°C, FAB-MS: 472 [M⁺+H].

### a) 4,5-Bis[4-(N,N-diethylamino)-anilino]phthalsäuredimethylester

Eine Lösung von 2,4 g (6 mmol) 4,5-Bis(trimethylsilyloxy)cyclohexa-1,4-dien--1,2-dicarbonsäuredimethylester (Beispiel 1a) und 3,94 g (24 mmol) 4-(N,N-Diethylamino)-anilin in 24 ml Eisessig wird während 2 h am Rückfluss gekocht. Das Reaktionsgemisch wird abgekühlt, das Lösungsmittel abgedampft, der dunkelbraune Rückstand in Dichlormethan gelöst und die Lösung nacheinander mit 50 ml gesätt. NaHCO₃ und zweimal 20 ml Wasser gewaschen, mit Natriumsulfat getrocknet und eingedampft. Der Eindampfrückstand wird mit Dichlormethan/Methanol 400:15 an Kieselgel chromatographiert, die Produktfraktionen eingedampft und erneut mit Ethylacetat/Hexan 1:1 an Kieselgel chromatographiert. Die Produktfraktionen werden eingedampft. Auf diese Weise wird die Titelverbindung in Form grüner Kristalle erhalten, FAB-MS: 518 [M⁺], 519 [M⁺+H].

### Beispiel 6: 4,5-Bis(cyclohexylamino)phthalimid

Analog zu Beispiel 1 werden 194 mg (0,5 mmol) 4,5-Bis(cyclohexylamino)phthalsäuredimethylester in 15 ml Ethylenglykol auf 120° erwärmt, wobei unter Rühren während 12h Ammoniak-Gas durchgeleitet wird. Das Reaktionsgemisch wird abgekühlt, mit Natriumchlorid gesättigt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Hexan/Ethylacetat 3:1 an Kieselgel chromatographiert, die Produktfraktionen vereinigt und eingedampft. Auf diese Weise wird die Titelverbindung in Form oranger Kristalle erhalten, Smp. 170-5°C, FAB-MS: 342 [M⁺+H].

### a) 4,5-Bis(cyclohexylamino)phthalsäuredimethylester

Eine Lösung von 2,4 g (6 mmol) 4,5-Bis(trimethylsilyloxy)cyclohexa-1,4-dien--1,2-dicarbonsäuredimethylester (Beispiel 1a) in 21,5 ml (188 mmol) Cyclohexylamin und 4,5 ml Eisessig wird während 3,5 h am Rückfluss gekocht. Das Reaktionsgemisch wird abgekühlt, das Lösungsmittel abgedampft, der dunkelbraune Rückstand in Dichlormethan gelöst und die Lösung nacheinander mit 100 ml 2N HCl, 50 ml gesätt. NaHCO₃ und zweimal 20 ml Wasser gewaschen, mit Natriumsulfat getrocknet und eingedampft. Der Eindampfrückstand wird mit Ethylacetat/Hexan 5:12 an Kieselgel chromatographiert, die Produktfraktionen eingedampft und nochmals mit Ethylacetat/Hexan 1:4 chromatographiert. Auf diese Weise wird die Titelverbindung als gelbes Oel erhalten, FAB-MS: 388 [M⁺].

Als Nebenprodukt wird 4-Cyclohexylamino-phthalsäuredimethylester erhalten, der analog Beispiel 6 in 4-Cyclohexylamino-phthalimid umgewandelt wird. Auf diese Weise wird ein farbloses Pulver erhalten: FAB-MS: 245 [M⁺], Smp. 217-9°C.

### Beispiel 7: 4,5-Bis(4-methoxyanilino)phthalimid

Analog zu Beispiel 1 werden 393 mg (0,9 mmol) 4,5-Bis(4-methoxyanilino)phthalsäuredimethylester in 25 ml Ethylenglykol auf 120° erwärmt, wobei unter Rühren während 18h Ammoniak-Gas durchgeleitet wird. Das Reaktionsgemisch wird abgekühlt, mit Natriumchlorid gesättigt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Ethylacetat/Hexan 1:1 an Kieselgel chromatographiert, die Produktfraktionen vereinigt und eingedampft. Auf diese Weise wird die Titelverbindung in Form gelber Kristalle erhalten, Smp. 191-3°C, FAB-MS: 390 [M⁺+H].

### a) 4,5-Bis(4-methoxyanilino)phthalsäuredimethylester

Eine Lösung von 2,4 g (6 mmol) 4,5-Bis(trimethylsilyloxy)cyclohexa-1,4-dien--1,2-dicarbonsäuredimethylester (Beispiel 1a) und 3,0 g (24 mmol) 4-Anisidin in 24 ml Eisessig wird während 2h am Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Der Eindampfrückstand wird mit Ethylacetat/Hexan 1:1 an Kieselgel chromatographiert und die Produktfraktionen eingedampft. Auf diese Weise wird die Titelverbindung als gelber Schaum erhalten, FAB-MS: 437 [M⁺+H].

### Beispiel 8: 4,5-Bis(2-iodanilino)phthalimid

Analog zu Beispiel 1 werden 1.48 g (2.36 mmol) 4,5-Bis(2-iodanilino)phthalsäuredimethylester in 25 ml Ethylenglykol auf 120° erwärmt, und unter Rühren wird während 19 h Ammoniak-Gas durchgeleitet. Das Reaktionsgemisch wird abgekühlt, mit Sole verdünnt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der nach dem Eindampfen erhaltene Rückstand wird mit Dichlormethan durch Kieselgel filtriert, und die Produktfraktionen werden vereinigt und eingedampft. Der nach dem Eindampfen erhaltene Rückstand wird aus siedendem Dichlormethan kristallisiert. Auf diese Weise wird die Titelverbindung in Form gelber Kristalle erhalten, Smp. 108-10°C, FAB-MS: 582 [M⁺+H].

### a) 4,5-Bis(2-iodanilino)phthalsäuredimethylester

Eine Lösung von 2,4 g (6 mmol) 4,5-Bis(trimethylsilyloxy)cyclohexa-1,4-dien--1,2-dicarbonsäuredimethylester (Beispiel 1a) und 5,3 g (24 mmol) 2-Iodanilin in 24 ml Eisessig wird während 2 h am Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Der nach dem Eindampfen erhaltene Rückstand wird mit Ethylacetat/Hexan 2:1 an Kieselgel chromatographiert und die Produktfraktionen eingedampft. Auf diese Weise wird die Titelverbindung in Form eines gelben Schaums erhalten: ¹H-NMR (DMSO-d₆):δ = 7.91 (dxd, J₁=8, J₂=1, 2H), 7.39 (dxdxd, J₁=10, J₂=8, J₃=1, 2H), 7.32 (br s, 2H), 7.22-7.05 (m, 2H), 7.01 (s, 2H), 6.88 (txd, Jₜ=8, J_{d}=1, 2H), 3.73 (s, 6H).

### Beispiel 9: 4,5-Bis(2-cyananilino)phthalimid

Eine Lösung von 581 mg (1 mmol) 4,5-Bis(2-iodanilino)phthalimid und 197 mg (2.2 mmol) Kupfer(I)cyanid in DMF wird während 6 Stunden bei 130-140°C gerührt, wobei die dunkelbraune Lösung in eine dunkelgelbe Suspension übergeht. Das Reaktionsgemisch wird auf 80°C abgekühlt und mit 8 ml Essigester verdünnt. Nach weiterem Abkühlen auf 60-70°C wird eine Lösung von 467 mg (2.88 mmol) Eisen(III)chlorid in 1.6 ml Wasser und 300 »l konz. Salzsäure zugetropft und 30 min. nachgerührt. Das Reaktionsgemisch wird mit 2 ml Wasser und Hyflo Super Cel® (Kieselgur der Firma Fluka, Buchs, Schweiz) versetzt und durch Hyflo Super Cel® filtriert, und die Phasen werden getrennt. Die wässrige Phase wird einmal mit Essigester extrahiert, die vereinigten organischen Phasen werden zweimal mit Wasser, einmal mit gesätt. Natriumbicarbonat-Lösung und erneut zweimal mit Wasser gewaschen, über Magnesiumsulfat getrocknet und eingedampft. Das resultierende ölige braune Kristallgemisch wird mit Ethylacetat/Hexan 2:3 an Kieselgel chromatographiert. Die Produktfraktionen werden eingedampft und aus siedendem Dichlormethan kristallisiert. Auf diese Weise wird die Titelverbindung in Form blassgelber Kristalle erhalten: FAB-MS: 380 (M⁺+H), Smp.: 279-80°C.

Als Nebenprodukt wird 5-Anilino-4-(2-cyananilino)phthalimid in Form gelber Kristalle erhalten: FAB-MS: 355 (M⁺+H), Smp.: 115-7°C.

### Beispiel 10: 4,5-Bis(2-nitroanilino)phthalimd, 4-(4-Nitroanilino-5-(2,4-dinitroanilino)phthalimid und 4,5-Bis(4-Nitroanilino)phthalimid

Zu einer Mischung von 12 mmol Acetanhydrid und 24 mmol Eisessig und 7.5 mmol Salpetersäure (100%) werden bei Temperaturen unter 20°C 990 mg (3 mmol) 4,5-Bis(anilino)phthalimid zugegeben. Nach 20 min Rühren wird das Reaktionsgemisch auf Eis gegossen und mit Essigester extrahiert. Die organischen Phasen werden einmal mit gesätt. NaHCO₃-Lösung und einmal mit Wasser gewaschen, über Natriumsulfat getrocknet und filtriert. Durch zweimaliges Chromatographieren an Kieselgel mit einem Gradienten von Dichlormethan/Essigester 1:1 bis 3:1 werden die folgenden amorphen Verbindungen erhalten: 4,5-Bis(2-nitroanilino)phthalimid in Form eines rot-schwarzen Pulvers: Smp. 87-90°C; 4-(4-Nitroanilino-5-(2,4-dinitroanilino)phthalimid in Form eines rot-schwarzen Pulvers: Smp. 176-8°C, ¹H-NMR (DMSO-d₆): 9.25 (br s, 2H), 8.85 (d, J=2.5, 1H), 8.16 (dxd, J₁=8, J₂=2.5, 1H), 8.13 (d, j=9, 2H), 7.88 (s, 1H), 7.75 (s, 1H), 7.17 (d, J=9, 2H), 6.96 (d, J=9.5, 1H); 4,5-Bis(4-nitroanilino)phthalimid in Form eines roten, glänzenden Pulvers: Smp. > 250°C, Zers. ab ∼105°C, ¹H-NMR (DMSO-d₆): 9.22 (br s, 2H), 8.12 (d, J=9.1, 4 H), 7.71 (s, 2H), 7.13 (d, J=9.1, 4H); ¹³C-NMR (DMSO-d₆): 168.9 s, 149.8 s, 139.6 s, 138.2 s, 128.2 s, 125.9 d, 116.2 d, 115.6 d.

### Beispiel 11: 4,5-Bis(4-aminoanilino)phthalimid

Eine Lösung von 38 mg (0.09 mmol) 4,5-Bis(4-nitroanilino)phthalimid in 15 ml THF wird mit 10% Raney-Nickel als Katalysator während drei Stunden bei Normaldruck und RT hydriert. Das Reaktionsgemisch wird vom Katalysator filtriert und eingedampft. Auf diese Weise wird die Titelverbindung in Form eines leicht gelblichen Pulvers erhalten: Smp. 154-7°C, FAB-MS: 360 (M⁺+H).

### Beispiel 12: Analog zu den jeweils in Klammern angegebenen Beispielen werden hergestellt:

(a) 4,5-Bis(4-iodanilino)phthalimid, FAB-MS: 582 (M⁺+H), Smp.: 246-47°C (analog Beispiel 8).
(b) 4,5-Bis(3-iodanilino)phthalimid, FAB-MS: 582 (M⁺+H), Smp.: 244-5°C (analog Beispiel 8).
(c) 4,5-Bis(2,6-dibromanilino)phthalimid, FAB-MS: 642 (M⁺+H), Smp.: 235-7°C (analog Beispiel 8).
(d) 4,5-Bis(3-methoxyanilino)phthalimid, FAB-MS: 390 (M⁺+H), Smp.: 169-71°C (analog Beispiel 7).
(e) 4,5-Bis(2-methoxyanilino)phthalimid, FAB-MS: 390 (M⁺+H), Smp.: 227-8°C (analog Beispiel 7).
(f) 4,5-Bis(4-trifluormethylanilino)phthalimid, FAB-MS: 512 (M⁺+H), ¹H-NMR (CD₃OD): 7.7 (s, 2H), 7.5 (d, 4H), 7.2 (d, 4H) (analog Beispiel 1)
(g) 4-Cyananilino-5-trifluormethylanilino-phthalimid, FAB-MS: 423 (M⁺+H), ¹H-NMR (CDCl₃): 7.7 (d, 2H), 7.6 (d, 4H), 7.1 (d, 2H), 7.0 (d, 2H), 6.2 (s, 1H), 6.1 (s, 1H) (analog Beispiel 1).
(h) 4,5-Bis(4-biphenylamino)phthalimid, FAB-MS: 482 (M⁺+H), Smp.: 230-1°C (analog Beispiel 1).
(i) 4,5-Bis(4-cyananilino)phthalimid, FAB-MS: 380 (M⁺+H), Smp.: > 250°C, ¹H-NMR (DMSO-d₆): 7.1 (d, 4H), 7.6 (d, 4H), 7.7 (s, 2H) (analog Beispiel 9).
(j) 4,5-Bis(3-cyananilino)phthalimid, FAB-MS: 380 (M⁺+H), Smp.: 255-7°C (analog Beispiel 9).
(k) 4,5-Bis(4-pyridinamino)phthalimid (analog Beispiel 1)
(l) 4,5-Bis(3-pyridinamino)phthalimid (analog Beispiel 1)
(m) 4,5-Bis(2-pyridinamino)phthalimid (analog Beispiel 1)
(n) 4,5-Bis(2-pyrimidinamino)phthalimid (analog Beispiel 1)
(o) 4,5-Bis(3-pyrimidinamino)phthalimid (analog Beispiel 1)
(p) 4,5-Bis(4-pyrimidinamino)phthalimid (analog Beispiel 1)
(q) 4,5-Bis(2-triazinamino)phthalimid (analog Beispiel 1)
(r) 4,5-Bis(3-fluoranilino)phthalimid (analog Beispiel 3)
(s) 4,5-Bis(2-fluoranilino)phthalimid (analog Beispiel 3)
(t) 4,5-Bis(pentafluoranilino)phthalimid (analog Beispiel 3)
(u) 4,5-Bis(4-hydroxyanilino)phthalimid (analog Beispiel 1)
(v) 4,5-Bis(3-hydroxyanilino)phthalimid (analog Beispiel 1)
(w) 4,5-Bis(2-hydroxyanilino)phthalimid (analog Beispiel 1)
(x) 4,5-Bis(4-ethylanilino)phthalimid (analog Beispiel 1)
(y) 4,5-Bis(3-ethylanilino)phthalimid (analog Beispiel 1)
(z) 4,5-Bis(2-ethylanilino)phthalimid (analog Beispiel 1)
(aa) 4,5-Bis(4-methylanilino)phthalimid (analog Beispiel 1)
(ab) 4,5-Bis(3-methylanilino)phthalimid (analog Beispiel 1)
(ac) 4,5-Bis(2-methylanilino)phthalimid (analog Beispiel 1)
(ad) 4,5-Bis(3-trifluormethylanilino)phthalimid (analog Beispiel 1)
(ae) 4,5-Bis(2-trifluormethylanilino)phthalimid (analog Beispiel 1)
(af) 4,5-Bis[4-(N,N-dimethylamino)-anilino]phthalimid (analog Beispiel 5)
(ag) 4,5-Bis[4-(N-acetylamino)-anilino]phthalimid (analog Beispiel 1)
(ah) 4,5-Bis(3-biphenylylamino)phthalimid (analog Beispiel 1)
(ai) 4,5-Bis(2-biphenylylamino)phthalimid (analog Beispiel 1)
(aj) 4,5-Bis(1-naphthylamino)phthalimid (analog Beispiel 1)
(ak) 4,5-Bis(2-naphthylamino)phthalimid (analog Beispiel 1)
(al) 4,5-Bis(5-tetralinylamino)phthalimid (analog Beispiel 1)
(am) 4,5-Bis(4-carboxyanilino)phthalimid (analog Beispiel 1)
(an) 4,5-Bis(3-carboxyanilino)phthalimid (analog Beispiel 1)
(ao) 4,5-Bis(2-carboxyanilino)phthalimid (analog Beispiel 1)
(ap) 4,5-Bis(4-methoxycarbonyl-anilino)phthalimid (analog Beispiel 1)
(aq) 4,5-Bis(3-methoxycarbonyl-anilino)phthalimid (analog Beispiel 1)
(ar) 4,5-Bis(2-methoxycarbonyl-anilino)phthalimid (analog Beispiel 1)
(as) 4,5-Bis(4-ethoxycarbonyl-anilino)phthalimid (analog Beispiel 1)
(at) 4,5-Bis(3-ethoxycarbonyl-anilino)phthalimid (analog Beispiel 1)
(au) 4,5-Bis(2-ethoxycarbonyl-anilino)phthalimid (analog Beispiel 1)
(av) 4,5-Bis(4-isopropyloxycarbonyl-anilino)phthalimid (analog Beispiel 1)
(aw) 4,5-Bis(4-tert.-butyloxycarbonyl-anilino)phthalimid (analog Beispiel 1)
(ax) 4,5-Bis(4-carbamoyl-anilino)phthalimid (analog Beispiel 1)
(ay) 4,5-Bis(4-N,N-dimethylcarbamoyl-anilino)phthalimid (analog Beispiel 1)
(az) 4,5-Bis(4-hydroxy-3-methylanilino)phthalimid (analog Beispiel 1)
(ba) 4,5-Bis(2-hydroxy-5-methylanilino)phthalimid (analog Beispiel 1).

### Beispiel 13: 4,5-Bis(N-methyl-N-phenylamino)phthalimid

Analog zu Beispiel 1 werden 66 mg (0.16 mmol) 4,5-Bis(N-methyl-N-phenylamino)-phthalsäuredimethylester (Beispiel 14 A)in 5 ml Ethylenglykol auf 120° erwärmt und unter Rühren während 18 h Ammoniak-Gas durchgeleitet. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Hexan/Ethylacetat 1:1 an Kieselgel chromatographiert, die Produktfraktionen vereinigt und eingedampft. Auf diese Weise wird die Titelverbindung in Form schwach gelber Kristalle erhalten, FAB-MS: 358 [M⁺+H], ¹H-NMR (CDCl₃): 3.05 (s, 6H).

### Beispiel 14: 4-(N-Methyl-N-phenylamino)-5-anilino-phthalimid

Analog zu Beispiel 1 werden 160 mg (0.41 mmol) 4-(N-methyl-N-phenylamino)-5-anilinophthalsäuredimethylester in 12 ml Ethylenglykol auf 120° erwärmt und unter Rühren während 18 h Ammoniak-Gas durchgeleitet. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Hexan/Ethylacetat 1:1 an Kieselgel chromatographiert, die Produktfraktionen vereinigt und eingedampft. Auf diese Weise wird die Titelverbindung in Form schwach gelber Kristalle erhalten, FAB-MS: 344 [M⁺+H], ¹H-NMR (CDCl₃): 3.28 (s, 3H).

### a) 4,5-Bis(N-methyl-N-phenylamino)phthalsäuredimethylester (A) und 4-(N-Methyl- -N-phenylamino)-5-anilino-phthalsäuredimethylester (B)

Eine Lösung von 564 mg (1.5 mmol) 4,5-Bis(anilino)phthalsäuredimethylester (Beispiel 1b) in 5 ml Acetonitril wird mit 0.93 ml (15 mmol) Methyliodid und 442 mg (3.2 mmol) wasserfreiem Kaliumkarbonat im Bombenrohr während 16 Stunden auf 80°C erhitzt. Das Reaktionsgemisch wird zur Trockne eingedampft, der Rückstand zweimal in Dichlormethan digeriert, filtriert und das Filtrat eingedampft. Mehrmalige Chromatographie mit Hexan/Ehtylacetat an Kieselgel lieferte die Titelverbindungen in Form leicht gelblicher Pulver. (A): FAB-MS: 405 [M⁺+H]; (B): FAB-MS: 391 [M⁺+H].

### Beispiel 15: 4,5-Bis(anilino)-N-methyl-phthalimid

Analog zu Beispiel 1 werden 376 mg (1 mmol) 4,5-Bis(anilino)phthalsäuredimethylester in 33 ml Ethylenglykol auf 120° erwärmt und unter Rühren während 18 h Methylamin durchgeleitet. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Hexan/Ethylacetat 1:1 an Kieselgel chromatographiert, die Produktfraktionen vereinigt und eingedampft. Auf diese Weise wird die Titelverbindung in Form schwach gelber Kristalle erhalten, FAB-MS: 344 [M⁺+H], Smp. 195-6°C.

### Beispiel 16: 4,5-Bis(anilino)-thiophthalimid [= 5,6-Bis(anilino)-isoindol-1-on-3-thion]

Eine Lösung von 100 mg (0.3 mmol) 4,5-Bis(anilino)phthalimid (Beispiel 1) in 15 ml Dichlormethan wird mit 138 mg (0.36 mmol) Lawesson-Reagens [= 2,4-Bis-(4-methoxyphenyl)-2,4-dithioxo-1,3,2,4-dithiaphosphetan] versetzt und 4 Stunden am Rückfluss gekocht. Das Reaktionsgemisch wird eingedampft und direkt an Kieselgel mit Hexan/Ethylacetat 2:1 chromatographiert. Die Produktfraktionen werden eingedampft. Auf diese Weise erhält man die Titelverbindung in Form gelber Kristalle, FAB-MS: 346 [M⁺+H].

### Beispiel 17: 4,5-Bis(anilino)-N⁴,N⁵-propan-1,3-diyl-phthalimid

In einem Autoklaven werden 457 mg (1 mmol) 4,5-Bis(anilino)-N⁴,N⁵-propan-1,3-diyl--phthalsäuredimethylester in 5 ml Methanol gelöst und 15 ml Ammoniak zur Amidbildung eingesetzt. Der geschlossene Autoklav wird während 24 Stunden auf 120°C geheizt, darauf abgekühlt, geöffnet und der Ammoniak mit Stickstoff abgeblasen. Der Rückstand wird mit Essigester ausgespült, filtriert und das Filtrat an Kieselgel mit Hexan/Essigester 3:1 chromatographiert. Die Produktfraktionen werden eingedampft und aus Hexan/Essigester kristallisiert. Auf diese Weise wird die Titelverbindung in Form gelber Kristalle erhalten, FAB-MS: 370 [M⁺+H].

### a) 4,5-Bis(anilino)-N⁴,N⁵-propandiyl-phthalsäuredimethylester und 4,5-Bis(N-allylanilino)phthalsäuredimethylester

Eine Lösung von 3.76 g (10 mmol) 4,5-Bis(anilino)phthalsäuredimethylester (Beispiel 1b) in 15 ml HMPT (Hexamethylphosphorsäuretriamid) oder DMPU wird bei RT unter Argon mit 0.6 g (15.4 mmol) Natriumamid versetzt und während 30 min. auf 60°C erwärmt. Die tiefrote Lösung wird auf RT gekühlt und für 5 min evakuiert (1 Torr). Darauf wird eine Lösung von 1.5 ml (15.2 mmol) 1-Brom-3-chlorpropan in 2 ml THF zugetropft und das Reaktionsgemisch 18 Std. bei RT gerührt. Das Reaktionsgemisch wird auf Eiswasser gegossen, mit Essigester extrahiert, die organischen Phasen vereinigt und ausgiebig mit Wasser gewaschen, über Natriumsulfat getrocknet und eingedampft. Der Eindampfrückstand wird an Kieselgel mit Hexan/Essigester 5:1 chromatographiert. Auf diese Weise werden die Titelverbindungen erhalten: 4,5-Bis(anilino)-N⁴,N⁵-propandiyl--phthalsäuredimethylester in Form farbloser Kristalle, FAB-MS: 417 [M⁺+H]; und 4,5-Bis(N-allylanilino)phthalsäuredimethylester in Form eines farblosen Oels, FAB-MS: 457 [M⁺+H].

### Beispiel 18: Analog zu den Beispielen 13-17 werden hergestellt:

(a) 4-(N-Acetyl-N-phenyl)amino-5-anilino-phthalimid, FAB-MS: 372 [M⁺+H], ¹H-NMR (DMSO-d₆): 2.05 (s, 3H) (analog Beispiel 14).
(b) 4,5-Bis(anilino)-N-benzyl-phthalimid (analog Beispiel 15)
(c) 4,5-Bis(anilino)-N-amino-phthalimid (analog Beispiel 15)
(d) 4,5-Bis(anilino)-N-hydroxy-phthalimid (analog Beispiel 15)
(e) 4,5-Bis(N-allylanilino)phthalimid, FAB-MS: 410 (analog Beispiel 17)

### Beispiel 19: Es werden 5000 Kapseln hergestellt, die je 0,25 g Wirkstoff, z.B. eine der in den Beispielen 1-16 hergestellten Verbindungen, enthalten:

### Zusammensetzung

- Wirkstoff: 1250 g
- Talk: 180 g
- Weizenstärke: 120 g
- Magnesiumstearat: 80 g
- Laktose: 20 g

Verfahren: Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

### Beispiel 20: 4-Anilino-5-(4-hydroxy-anilino)phthalimid

Zu einer Lösung von 359,4 mg (1 mmol) 4-Anilino-5-(4-methoxy-anilino)phthalimid in 5 ml Chloroform wird bei - 40°C bis -30°C eine Lösung von 186 »l (2 mmol) Bortribromid in 5 ml Chloroform zugetropft. Das Reaktionsgemisch wird während 5 Stunden bei -30°C gerührt und danach mit 5 ml Wasser bei -30°C gequencht. Das Reaktionsgemisch wird auf RT aufgewärmt und die Phasen werden getrennt. Die organische Phase wird zweimal mit Wasser gewaschen, mit Magnesiumsulfat getrocknet und eingedampft. Der Eindampfrückstand wird mit Ethylacetat/Hexan 1:1 an einer mit Eiswasser (Doppelmantel) gekühlten Kieselgelsäule unter Lichtausschluss chromatographiert, die Produktfraktionen vereinigt und eingedampft. Man erhält die Titelverbindung in Form gelber Kristalle, FAB-MS: 346 [M⁺+H].

### a) 4-Anilino-5-(4-methoxy-anilino)phthalimid

Analog zu Beispiel 1 werden 0,7 g (1,7 mmol) 4-Anilino-5-(4-methoxy-anilino)phthalsäuredimethylester in 20 ml Ethylenglykol auf 120° erwärmt, wobei unter Rühren während 18 h Ammoniak-Gas durchgeleitet wird. Das Reaktionsgemisch wird abgekühlt und mit Ethylacetat extrahiert. Die Ethylacetat-Phasen werden nacheinander zweimal mit Wasser und einmal mit gesätt. Kochsalzlösung gewaschen, mit Magnesiumsulfat getrocknet und eingedampft. Der Eindampfrückstand wird mit Ethylacetat/Hexan 1:1 an Kieselgel chromatographiert, die Produktfraktionen vereinigt und eingedampft. Man erhält die Titelverbindung in Form gelber Kristalle, Smp. 266-7°C, FAB-MS: 360 [M⁺+H].

### b) 4-Anilino-5-(4-methoxy-anilino)phthalsäuredimethylester und 4,5-Bis(4-methoxyoxy-anilino)phthalsäuredimethylester

Eine Lösung von 4,8 g (12 mmol) 4,5-Bis(trimethylsilyloxy)cyclohexa-1,4-dien--1,2-dicarbonsäuredimethylester (Beispiel 1a), 2,6 g (24 mmol) p-Anisidin und 2,2 ml (24 mmol) Anilin in 48 ml Eisessig wird während 2 h am Rückfluss gekocht. Das Reaktionsgemisch wird abgekühlt, das Lösungsmittel abgedampft, der dunkelbraune Rückstand in Ethylacetat gelöst und die Lösung nacheinander mit 40 ml 1N HCl, 100 ml gesätt. NaHCO₃ und zweimal 100 ml Wasser gewaschen, mit Magnesiumsulfat getrocknet und eingedampft. Der Eindampfrückstand wird mit Ethylacetat/Hexan 1:3 an Kieselgel chromatographiert und die Produktfraktionen eingedampft. Aus den ersten Produktfraktionen wird auf diese Weise 4,5-Bis(4-methoxy-anilino)phthalsäuredimethylester in Form eines gelben Schaums erhalten: FAB-MS: 437 [M⁺+H]. Der Eindampfrückstand der nachfolgenden Produktfraktionen wird aus Ethylacetat/Hexan umkristallisiert und 4-Anilino-5-(4-methoxy-anilino)phthalsäuredimethylester in Form gelber Kristalle erhalten, Smp. 122-4°C, FAB-MS: 407 [M⁺+H].

## Patentansprüche (Patentansprüche für folgende(n) Vertragsstaat(en): AT, BE, CH, DE, DK, FR, GB, IT, LI, LU, MC, NL, PT, SE)

1. Verbindungen der Formel I, 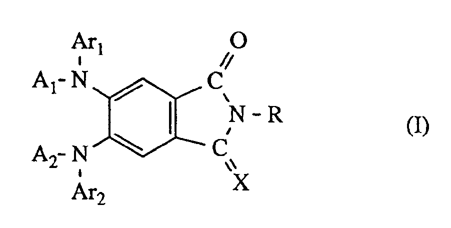 worin A₁ und A₂ unabhängig voneinander für Wasserstoff, C₁-C₇-Alkyl, C₂-C₇-Alkenyl, C₂-C₇-Alkinyl, Phenyl oder Naphthyl, welches unsubstituiert oder durch einen oder mehrere Substituenten aus der Gruppe bestehend aus C₁-C₇-Alkyl, C₂-C₇-Alkenyl, C₂-C₇-Alkinyl, C₃-C₄-Alkylen (angeknüpft an zwei benachbarten C-Atomen), C₃-C₈-Cycloalkyl, Phenyl-C₁-C₇-alkyl, Phenyl; C₁-C₇-Alkyl, das durch Hydroxy, C₁-C₇-Alkoxy, Phenyl-C₁-C₇-alkoxy, C₁-C₇-Alkanoyloxy, Halogen, Amino, C₁-C₇-Alkylamino, Di-C₁-C₇-alkylamino, Mercapto, C₁-C₇-Alkylthio, C₁-C₇-Alkylsulfinyl, C₁-C₇-Alkylsulfonyl, Carboxy, C₁-C₇-Alkoxycarbonyl, Carbamoyl, N-C₁-C₇-Alkylcarbamoyl, N,N-Di-C₁-C₇-alkylcarbamoyl und/oder Cyano substituiert ist; Hydroxy, C₁-C₇-Alkoxy, Halogen-C₁-C₇-alkoxy, Phenyl-C₁-C₇-alkoxy, Phenyloxy, C₂-C₇-Alkenyloxy, Halogen-C₂-C₇-alkenyloxy, C₂-C₇-Alkinyloxy, C₁-C₂-Alkylendioxy (angeknüpft an zwei benachbarten C-Atomen), C₁-C₇-Alkanoyloxy, Phenyl-C₁-C₇-alkanoyloxy, Phenylcarbonyloxy, Mercapto, C₁-C₇-Alkylthio, Phenyl-C₁-C₇-alkylthio, Phenylthio, C₁-C₇-Alkylsulfinyl, Phenyl-C₁-C₇-alkylsulfinyl, Phenylsulfinyl, C₁-C₇-Alkylsulfonyl, Phenyl-C₁-C₇-alkylsulfonyl, Phenylsulfonyl, Halogen, Nitro, Amino, C₁-C₇-Alkylamino, C₃-C₈-Cycloalkylamino, Phenyl-C₁-C₇-alkylamino, Phenylamino, Di-C₁-C₇-Alkylamino, N-C₁-C₇-Alkyl-N-phenylamino, N-C₁-C₇-Alkyl-N-phenyl-C₁-C₇-alkylamino, C₄-C₇-Alkylenamino, durch -O-, -S- oder -NR˝- unterbrochenes C₄-C₇-Alkylenamino (worin R˝ für Wasserstoff, C₁-C₇-Alkyl oder C₁-C₇-Alkanoyl steht), C₁-C₇-Alkanoylamino, Phenyl-C₁-C₇-alkanoylamino, Phenylcarbonylamino, C₁-C₇-Alkanoyl, Phenyl-C₁-C₇-alkanoyl, Phenylcarbonyl, Carboxy, C₁-C₇-Alkoxycarbonyl, Carbamoyl, C₁-C₇-Alkylcarbamoyl, N,N-Di-C₁-C₇-alkylcarbamoyl, N-Hydroxycarbamoyl, N-Phenylcarbamoyl, Cyano, Sulfo, C₁-C₇-Alkoxysulfonyl, Sulfamoyl, N-C₁-C₇-Alkylsulfamoyl, N,N-Di-C₁-C₇-alkylsulfamoyl oder N-Phenylsulfamoyl substituiert ist, wobei in den Substituenten vorkommende Phenylgruppen jeweils unsubstituiert oder durch C₁-C₇-Alkyl, C₁-C₇-Alkoxy, Hydroxy, Halogen und/oder Trifluormethyl substituiert sind; C₁-C₇-Alkanoyl, Halogen-C₁-C₇-alkanoyl, im Phenyl- oder Naphthylrest unsubstituiertes oder wie oben substituiertes Phenyl- oder Naphthyl-C₁-C₇-alkanoyl oder im Phenyl- oder Naphthylrest unsubstituiertes oder wie oben substituiertes Phenyl- oder Naphthyl-carbonyl, C₁-C₇-Alkylsulfonyl oder Phenyl- oder Naphthylsulfonyl, worin Phenyl oder Naphthyl unsubstituiert oder wie oben substituiert sind, stehen; oder worin A₁ und A₂ zusammen unsubstituiertes oder durch C₁-C₇-Alkyl oder Hydroxy substituiertes C₁-C₄-Alkylen bedeuten; Ar₁ und Ar₂ unabhängig voneinander unsubstituiertes oder wie oben substituiertes Phenyl oder Naphthyl; Imidazolyl, Triazolyl, Pyridyl, Pyrimidinyl oder Triazinyl, welches unsubstituiert oder durch C₁-C₇-Alkyl, Hydroxy, C₁-C₇-Alkoxy, Halogen, Cyano und/oder Trifluormethyl substituiert ist; oder unsubstituiertes oder durch C₁-C₇-Alkyl oder Hydroxy substituiertes C₃-C₈-Cycloalkyl bedeuten, die Gruppe -C(=X)- für -C(=O)-, -C(=S)-, -CH₂- oder -C(=CR₁R₂)- steht, wobei R₁ und R₂ unabhängig voneinander Wasserstoff oder C₁-C₇-Alkyl bedeuten, und R Wasserstoff, C₁-C₇-Alkyl, Aryl-C₁-C₇-alkyl oder Aryl, worin Aryl Phenyl oder Napthyl bedeutet, welches unsubstituiert oder wie oben substituiert ist, Amino, Hydroxy oder C₁-C₇-Alkoxy bedeutet, mit der Massgabe, dass R von der Bedeutung Phenyl verschieden ist, wenn A₁ und A₂ für Wasserstoff stehen, Ar₁ und Ar₂ Phenyl bedeuten und die Gruppe -C(=X)- für -C(=O)- steht, und Salze davon, sofern salzbildende Gruppen vorliegen.

2. Verbindungen der Formel I gemäss Anspruch 1, worin A₁ und A₂ unabhängig voneinander für Wasserstoff, Niederalkyl; C₂-C₇-Alkenyl; Phenyl oder 1- oder 2-Naphthyl, wobei die drei letztgenannten Reste unsubstituiert oder durch Niederalkyl, Niederalkoxy, Hydroxy, Halogen und/oder Trifluormethyl substituiert sind; Niederalkanoyl, Niederalkylsulfonyl oder Phenylsulfonyl, worin die Phenylgruppe unsubstituiert oder durch Niederalkyl, Niederalkoxy, Hydroxy, Halogen und/oder Trifluormethyl substituiert ist, stehen; oder worin A₁ und A₂ zusammen C₁-C₄-Alkylen bedeuten; worin Ar₁ und Ar₂ unabhängig voneinander Phenyl oder Naphthyl, welches jeweils unsubstituiert oder durch einen oder mehrere Substituenten aus der Gruppe bestehend aus Niederalkyl, C₂-C₇-Alkenyl, C₂-C₇-Alkinyl, C₃-C₄-Alkylen (angeknüpft an zwei benachbarten C-Atomen), C₃-C₈-Cycloalkyl, Phenylniederalkyl, Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy, Niederalkoxy, Halogenniederalkoxy, Phenylniederalkoxy, Phenyloxy, C₂-C₇-Alkenyloxy, Halogen-C₂-C₇-alkenyloxy, C₂-C₇-Alkinyloxy, C₁-C₂-Alkylendioxy (angeknüpft an zwei benachbarten C-Atomen), Niederalkanoyloxy, Phenylniederalkanoyloxy, Phenylcarbonyloxy, Mercapto, Niederalkylthio, Phenylniederalkylthio, Phenylthio, Niederalkylsulfinyl, Phenylniederalkylsulfinyl, Phenylsulfinyl, Niederalkylsulfonyl, Phenylniederalkylsulfonyl, Phenylsulfonyl, Halogen, Nitro, Amino, Niederalkylamino, C₃-C₈-Cycloalkylamino, Phenylniederalkylamino, Phenylamino, Diniederalkylamino, N-Niederalkyl-N-phenylamino, N-Niederalkyl-N-phenylniederalkylamino, C₄-C₇-Alkylenamino, durch -O-, -S- oder -NR˝- unterbrochenes C₄-C₇-Alkylenamino (worin R˝ für Wasserstoff, Niederalkyl oder Niederalkanoyl steht), Niederalkanoylamino, Phenylniederalkanoylamino, Phenylcarbonylamino, Niederalkanoyl, Phenylniederalkanoyl, Phenylcarbonyl, Carboxy, Niederalkoxycarbonyl, Carbamoyl, N-Niederalkylcarbamoyl, N,N-Diniederalkylcarbamoyl, N-Hydroxycarbamoyl, N-Phenylcarbamoyl, Cyano, Sulfo, Niederalkoxysulfonyl, Sulfamoyl, N-Niederalkylsulfamoyl, N,N-Diniederalkylsulfamoyl und N-Phenylsulfamoyl substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; Pyridyl oder Pyrimidinyl, das unsubstituiert oder durch Niederalkyl, Hydroxy, Niederalkoxy, Halogen, Cyano und/oder Trifluormethyl substituiert ist; oder C₃-C₈-Cycloalkyl bedeuten; die Gruppe -C(=X)- für -C(=O)-, -C(=S)-, -CH₂- oder -C(=CR₁R₂)- steht, wobei R₁ und R₂ unabhängig voneinander Wasserstoff oder Niederalkyl bedeuten, und R Wasserstoff, Niederalkyl; Phenylniederalkyl, Phenyl, 1- oder 2-Naphthyl, wobei in den vier letztgenannten Resten die Phenyl- bzw. Naphthylgruppe jeweils unsubstituiert oder durch Niederalkyl, Niederalkoxy, Hydroxy, Halogen und/oder Trifluormethyl substituiert ist; Amino, Hydroxy oder Niederalkoxy bedeutet, mit der Massgabe, dass R von der Bedeutung Phenyl verschieden ist, wenn A₁ und A₂ für Wasserstoff stehen, Ar₁ und Ar₂ Phenyl bedeuten und die Gruppe -C(=X)- für -C(=O)- steht, und Salze davon, sofern salzbildende Gruppen vorliegen; wobei in den vorgenannten Resten Nieder für C₁-C₇- und nieder für -C₁-C₇- steht.

3. Verbindungen der Formel I gemäss Anspruch 2, worin R die genannten Bedeutungen ausser Phenyl hat, und die übrigen Reste die genannten Bedeutungen haben, und Salze davon, sofern salzbildende Gruppen vorliegen.

4. Verbindungen der Formel I gemäss Anspruch 1, worin A₁ und A₂ unabhängig voneinander für Wasserstoff, Niederalkyl; Phenyl oder 1- oder 2-Naphthyl, wobei die drei letztgenannten Reste unsubstituiert oder durch Niederalkyl, Niederalkoxy, Hydroxy, Halogen und/oder Trifluormethyl substituiert sind; Niederalkanoyl, Niederalkylsulfonyl oder Phenylsulfonyl, worin die Phenylgruppe unsubstituiert oder durch Niederalkyl, Niederalkoxy, Hydroxy, Halogen und/oder Trifluormethyl substituiert ist, stehen; oder worin A₁ und A₂ zusammen C₁-C₄-Alkylen bedeuten; worin Ar₁ und Ar₂ unabhängig voneinander Phenyl oder Naphthyl, welches jeweils unsubstituiert oder durch einen oder mehrere Substituenten aus der Gruppe bestehend aus Niederalkyl, C₂-C₇-Alkenyl, C₂-C₇-Alkinyl, C₃-C₄-Alkylen (angeknüpft an zwei benachbarten C-Atomen), C₃-C₈-Cycloalkyl, Phenylniederalkyl, Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy, Niederalkoxy, Halogenniederalkoxy, Phenylniederalkoxy, Phenyloxy, C₂-C₇-Alkenyloxy, Halogen-C₂-C₇-alkenyloxy, C₂-C₇-Alkinyloxy, C₁-C₂-Alkylendioxy (angeknüpft an zwei benachbarten C-Atomen), Niederalkanoyloxy, Phenylniederalkanoyloxy, Phenylcarbonyloxy, Mercapto, Niederalkylthio, Phenylniederalkylthio, Phenylthio, Niederalkylsulfinyl, Phenylniederalkylsulfinyl, Phenylsulfinyl, Niederalkylsulfonyl, Phenylniederalkylsulfonyl, Phenylsulfonyl, Halogen, Nitro, Amino, Niederalkylamino, C₃-C₈-Cycloalkylamino, Phenylniederalkylamino, Phenylamino, Diniederalkylamino, N-Niederalkyl-N-phenylamino, N-Niederalkyl-N-phenylniederalkylamino, C₄-C₇-Alkylenamino, durch -O-, -S- oder -NR˝- unterbrochenes C₄-C₇-Alkylenamino (worin R˝ für Wasserstoff, Niederalkyl oder Niederalkanoyl steht), Niederalkanoylamino, Phenylniederalkanoylamino, Phenylcarbonylamino, Niederalkanoyl, Phenylniederalkanoyl, Phenylcarbonyl, Carboxy, Niederalkoxycarbonyl, Carbamoyl, N-Niederalkylcarbamoyl, N,N-Diniederalkylcarbamoyl, N-Hydroxycarbamoyl, N-Phenylcarbamoyl, Cyano, Sulfo, Niederalkoxysulfonyl, Sulfamoyl, N-Niederalkylsulfamoyl, N,N-Diniederalkylsulfamoyl und N-Phenylsulfamoyl substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; Pyridyl, das unsubstituiert oder durch Niederalkyl, Hydroxy, Niederalkoxy, Halogen, Cyano und/oder Trifluormethyl substituiert ist; oder C₃-C₈-Cycloalkyl bedeuten; die Gruppe -C(=X)- für -C(=O)-, -C(=S)-, -CH₂- oder -C(=CR₁R₂)- steht, wobei R₁ und R₂ unabhängig voneinander Wasserstoff oder Niederalkyl bedeuten, und R Wasserstoff, Niederalkyl; Phenylniederalkyl, Phenyl, 1- oder 2-Naphthyl, wobei in den vier letztgenannten Resten die Phenyl- bzw. Naphthylgruppe jeweils unsubstituiert oder durch Niederalkyl, Niederalkoxy, Hydroxy, Halogen und/oder Trifluormethyl substituiert ist; Amino, Hydroxy oder Niederalkoxy bedeutet, mit der Massgabe, dass R von der Bedeutung Phenyl verschieden ist, wenn A₁ und A₂ für Wasserstoff stehen, Ar₁ und Ar₂ Phenyl bedeuten und die Gruppe -C(=X)- für -C(=O)- steht, und Salze davon, sofern salzbildende Gruppen vorliegen; wobei in den vorgenannten Resten Nieder für C₁-C₇- und nieder für -C₁-C₇- steht.

5. Verbindungen der Formel I gemäss Anspruch 2, worin A₁ und A₂ unabhängig voneinander Wasserstoff oder Niederalkyl bedeuten, die Gruppe -C(=X)- für -C(=O)-, -C(=S)- oder -C(=CH₂)- steht, und R Wasserstoff oder Niederalkyl bedeutet, und Salze davon, sofern salzbildende Gruppen vorliegen; wobei in den vorgenannten Resten Nieder für C₁-C₇- steht.

6. Verbindungen der Formel I gemäss Anspruch 1, worin A₁ und A₂ unabhängig voneinander für Wasserstoff; Niederalkyl; C₂-C₇-Alkenyl; oder Niederalkanoyl stehen; oder worin A₁ und A₂ zusammen C₁-C₄-Alkylen bedeuten; worin Ar₁ und Ar₂ unabhängig voneinander Phenyl oder Naphthyl, welches jeweils unsubstituiert oder durch einen oder mehrere Substituenten aus der Gruppe bestehend aus Niederalkyl, C₃-C₄-Alkylen (angeknüpft an zwei benachbarten C-Atomen), Hydroxy, Phenyloxy, Halogen, Nitro, Amino, Niederalkylamino, Diniederalkylamino, Niederalkanoylamino, Carboxy, Niederalkoxycarbonyl, Carbamoyl, N-Niederalkylcarbamoyl, N,N-Diniederalkylcarbamoyl und Cyano substituiert ist; Pyridyl; Pyrimidinyl; oder C₃-C₈-Cycloalkyl bedeuten; die Gruppe -C(=X)- für -C(=O)- oder -C(=S)- steht, und R Wasserstoff, Niederalkyl; Phenylniederalkyl, Amino oder Hydroxy bedeutet, und Salze davon, sofern salzbildende Gruppen vorliegen; wobei in den vorgenannten Resten Nieder für C₁-C₇- und nieder für C₁-C₇- steht.

7. Verbindungen der Formel I gemäss Anspruch 1, worin A₁ und A₂ unabhängig voneinander für Wasserstoff oder Methyl stehen; oder worin A₁ und A₂ zusammen -(CH₂)₂- oder -(CH₂)₃- bedeuten; Ar₁ und Ar₂ unabhängig voneinander für Phenyl oder Naphthyl, welches jeweils unsubstituiert oder durch einen oder mehrere Substituenten aus der Gruppe bestehend aus Niederalkyl, Niederalkoxy, Phenylniederalkoxy, Hydroxy, Niederalkanoyloxy, Nitro, Amino, Niederalkylamino, Diniederalkylamino, Niederalkanoylamino, Halogen, Trifluormethyl, Carboxy, Niederalkoxycarbonyl, Carbamoyl, N-Niederalkylcarbamoyl, N,N-Diniederalkylcarbamoyl, Cyano, Niederalkanoyl, Benzoyl, Niederalkoxysulfonyl und Sulfamoyl, N-Niederalkylsulfamoyl und N,N-Diniederalkylsulfamoyl substituiert ist; oder für Cyclopentyl, Cyclohexyl oder Pyridyl stehen; die Gruppe -C(=X)- für -C(=O)-, -C(=S)- oder -C(=CH₂)- steht, und R Wasserstoff oder Niederalkyl bedeutet, und pharmazeutisch anwendbare Salze davon; wobei in den vorgenannten Resten Nieder für C₁-C₇- und nieder für -C₁-C₇- steht.

8. Verbindungen der Formel I gemäss Anspruch 1, worin A₁ und A₂ für Wasserstoff stehen; Ar₁ und Ar₂ unabhängig voneinander Phenyl, das unsubstituiert oder durch Niederalkyl, Trifluormethyl, Phenyl, Hydroxy, Niederalkoxy, Benzyloxy, Amino, Diniederalkylamino, Niederalkanoylamino, Halogen, Carboxy, Niederalkoxycarbonyl, Carbamoyl, N,N-Diniederalkylcarbamoyl oder Cyano substituiert ist; oder Cyclohexyl bedeuten; die Gruppe -C(=X)- für -C(=O)-, -C(=S)- oder -C(=CH₂)- steht, und R Wasserstoff bedeutet, und pharmazeutisch anwendbare Salze davon; wobei in den vorgenannten Resten Nieder für C₁-C₇- und nieder für -C₁-C₇- steht.

9. 4,5-Bis(anilino)phthalimid gemäss Anspruch 1.

10. 4,5-Bis(4-fluoranilino)phthalimid gemäss Anspruch 1.

11. 4,5-Bis(cyclohexylamino)phthalimid gemäss Anspruch 1 oder ein pharmazeutisch anwendbares Salz davon.

12. 5,8-Diphenyl-5,8-diaza-5,6,7,8-tetrahydro-naphthalin-2,3-dicarbonsäureamid gemäss Anspruch 1.

13. 4,5-Bis(2-nitroanilino)phthalimid gemäss Anspruch 1.

14. 4,5-Bis-(4-aminoanilino)phthalimid gemäss Anspruch 1 oder ein pharmazeutisch annehmbares Salz davon.

15. 4,5-Bis(2-pyridinamino)phthalimid gemäss Anspruch 1 oder ein pharmazeutisch annehmbares Salz davon.

16. 4,5-Bis(2-pyrimidinamino)phthalimid gemäss Anspruch 1 oder ein pharmazeutisch annehmbares Salz davon.

17. 4-(N-Methyl-N-phenylamino)-5-anilino-phthalimid gemäss Anspruch 1.

18. 4,5-Bis(anilino)-N⁴,N⁵-propan-1,3-diyl-phthalimid gemäss Anspruch 1.

19. 4,5-Bis(N-allylanilino)phthalimid gemäss Anspruch 1.

20. Pharmazeutische Präparate enthaltend eine Verbindung der Formel I gemäss einem der Ansprüche 1 bis 19 und mindestens ein pharmazeutisch verwendbares Trägermaterial.

21. Eine Verbindung der Formel I gemäss einem der Ansprüche 1 bis 19 zur Anwendung in einem Verfahren zur therapeutischen Behandlung des tierischen oder menschlichen Körpers.

22. Verwendung einer Verbindung gemäss einem der Ansprüche 1 bis 19 zur Herstellung pharmazeutischer Präparate für die Behandlung von Krankheiten, die auf eine Hemmung von Protein-Tyrosinkinasen oder Serin/Threonin-Kinasen ansprechen.

23. Verfahren zur Herstellung einer Verbindung der Formel I gemäss Anspruch 1, dadurch gekennzeichnet, dass man
(a) eine Verbindung der Formel II, 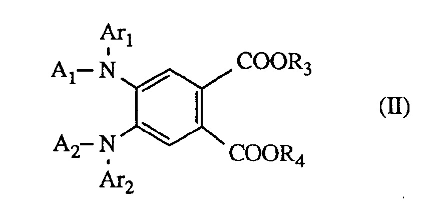 worin Ar₁, Ar₂, A₁ und A₂ die unter Formel I angegebene Bedeutung haben und R₃ und R₄ unabhängig voneinander Aryl oder C₁-C₇Alkyl bedeuten, mit einer Verbindung der Formel III,
H₂N-R (III)
worin R die unter Formel I angegebene Bedeutung hat, umsetzt, oder
(b) eine Verbindung der Formel IV, 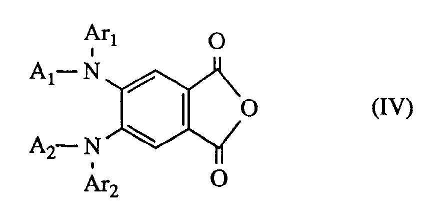 worin Ar₁, Ar₂, A₁ und A₂ die unter Formel I angegebene Bedeutung haben, mit einer Verbindung der Formel III,
H₂N-R (III)
worin R die unter Formel I angegebene Bedeutung hat, umsetzt;
und gewünschtenfalls eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I in ein Salz umwandelt und/oder ein erhaltenes Gemisch von isomeren Verbindungen der Formel I in die einzelnen Isomeren auftrennt.

## Patentansprüche (Patentansprüche für folgende(n) Vertragsstaat(en): ES, GR)

1. Verfahren zur Herstellung einer Verbindung der Formel I, 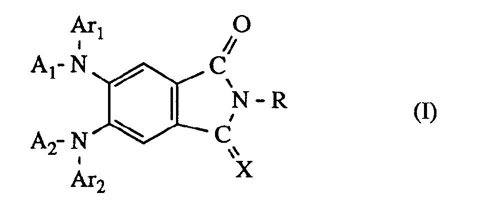 worin A₁ und A₂ unabhängig voneinander für Wasserstoff, C₁-C₇-Alkyl, C₂-C₇-Alkenyl, C₂-C₇-Alkinyl, Phenyl oder Naphthyl, welches unsubstituiert oder durch einen oder mehrere Substituenten aus der Gruppe bestehend aus C₁-C₇-Alkyl, C₂-C₇-Alkenyl, C₂-C₇-Alkinyl, C₃-C₄-Alkylen (angeknüpft an zwei benachbarten C-Atomen), C₃-C₈-Cycloalkyl, Phenyl-C₁-C₇-alkyl, Phenyl; C₁-C₇-Alkyl, das durch Hydroxy, C₁-C₇-Alkoxy, Phenyl-C₁-C₇-alkoxy, C₁-C₇-Alkanoyloxy, Halogen, Amino, C₁-C₇-Alkylamino, Di-C₁-C₇-alkylamino, Mercapto, C₁-C₇-Alkylthio, C₁-C₇-Alkylsulfinyl, C₁-C₇-Alkylsulfonyl, Carboxy, C₁-C₇-Alkoxycarbonyl, Carbamoyl, N-C₁-C₇-Alkylcarbamoyl, N,N-Di-C₁-C₇-alkylcarbamoyl und/oder Cyano substituiert ist; Hydroxy, C₁-C₇-Alkoxy, Halogen-C₁-C₇-alkoxy, Phenyl-C₁-C₇-alkoxy, Phenyloxy, C₂-C₇-Alkenyloxy, Halogen-C₂-C₇-alkenyloxy, C₂-C₇-Alkinyloxy, C₁-C₂-Alkylendioxy (angeknüpft an zwei benachbarten C-Atomen), C₁-C₇-Alkanoyloxy, Phenyl-C₁-C₇-alkanoyloxy, Phenylcarbonyloxy, Mercapto, C₁-C₇-Alkylthio, Phenyl-C₁-C₇-alkylthio, Phenylthio, C₁-C₇-Alkylsulfinyl, Phenyl-C₁-C₇-alkylsulfinyl, Phenylsulfinyl, C₁-C₇-Alkylsulfonyl, Phenyl-C₁-C₇-alkylsulfonyl, Phenylsulfonyl, Halogen, Nitro, Amino, C₁-C₇-Alkylamino, C₃-C₈-Cycloalkylamino, Phenyl-C₁-C₇-alkylamino, Phenylamino, Di-C₁-C₇-Alkylamino, N-C₁-C₇-Alkyl-N-phenylamino, N-C₁-C₇-Alkyl-N-phenyl-C₁-C₇-alkylamino, C₄-C₇-Alkylenamino, durch -O-, -S- oder -NR˝- unterbrochenes C₄-C₇-Alkylenamino (worin R˝ für Wasserstoff, C₁-C₇-Alkyl oder C₁-C₇-Alkanoyl steht), C₁-C₇-Alkanoylamino, Phenyl-C₁-C₇-alkanoylamino, Phenylcarbonylamino, C₁-C₇-Alkanoyl, Phenyl-C₁-C₇-alkanoyl, Phenylcarbonyl, Carboxy, C₁-C₇-Alkoxycarbonyl, Carbamoyl, C₁-C₇-Alkylcarbamoyl, N,N-Di-C₁-C₇-alkylcarbamoyl, N-Hydroxycarbamoyl, N-Phenylcarbamoyl, Cyano, Sulfo, C₁-C₇-Alkoxysulfonyl, Sulfamoyl, N-C₁-C₇-Alkylsulfamoyl, N,N-Di-C₁-C₇-alkylsulfamoyl oder N-Phenylsulfamoyl substituiert ist, wobei in den Substituenten vorkommende Phenylgruppen jeweils unsubstituiert oder durch C₁-C₇-Alkyl, C₁-C₇-Alkoxy, Hydroxy, Halogen und/oder Trifluormethyl substituiert sind; C₁-C₇-Alkanoyl, Halogen-C₁-C₇-alkanoyl, im Phenyl- oder Naphthylrest unsubstituiertes oder wie oben substituiertes Phenyl- oder Naphthyl-C₁-C₇-alkanoyl oder im Phenyl- oder Naphthylrest unsubstituiertes oder wie oben substituiertes Phenyl- oder Naphthyl-carbonyl, C₁-C₇-Alkylsulfonyl oder Phenyl- oder Naphthylsulfonyl, worin Phenyl oder Naphthyl unsubstituiert oder wie oben substituiert sind, stehen; oder worin A₁ und A₂ zusammen unsubstituiertes oder durch C₁-C₇-Alkyl oder Hydroxy substituiertes C₁-C₄-Alkylen bedeuten; Ar₁ und Ar₂ unabhängig voneinander unsubstituiertes oder wie oben substituiertes Phenyl oder Naphthyl; Imidazolyl, Triazolyl, Pyridyl, Pyrimidinyl oder Triazinyl, welches unsubstituiert oder durch C₁-C₇-Alkyl, Hydroxy, C₁-C₇-Alkoxy, Halogen, Cyano und/oder Trifluormethyl substituiert ist; oder unsubstituiertes oder durch C₁-C₇-Alkyl oder Hydroxy substituiertes C₃-C₈-Cycloalkyl bedeuten, die Gruppe -C(=X)- für -C(=O)-, -C(=S)-, -CH₂- oder -C(=CR₁R₂)- steht, wobei R₁ und R₂ unabhängig voneinander Wasserstoff oder C₁-C₇-Alkyl bedeuten, und R Wasserstoff, C₁-C₇-Alkyl, Aryl-C₁-C₇-alkyl oder Aryl, worin Aryl Phenyl oder Napthyl bedeutet, welches unsubstituiert oder wie oben substituiert ist, Amino, Hydroxy oder C₁-C₇-Alkoxy bedeutet, mit der Massgabe, dass R von der Bedeutung Phenyl verschieden ist, wenn A₁ und A₂ für Wasserstoff stehen, Ar₁ und Ar₂ Phenyl bedeuten und die Gruppe -C(=X)- für -C(=O)- steht, und von Salzen davon, sofern salzbildende Gruppen vorliegen, dadurch gekennzeichnet, dass man
(a) eine Verbindung der Formel II, 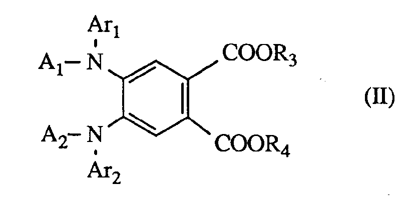 worin Ar₁, Ar₂, A₁ und A₂ die unter Formel I angegebene Bedeutung haben und R₃ und R₄ unabhängig voneinander Aryl oder C₁-C₇-Alkyl bedeuten, mit einer Verbindung der Formel III,
H₂N-R (III)
worin R die unter Formel I angegebene Bedeutung hat, umsetzt, oder
(b) eine Verbindung der Formel IV, 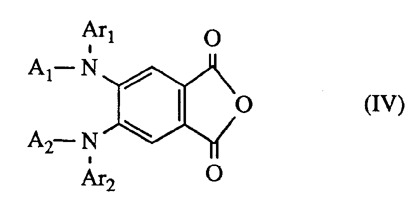 worin Ar₁, Ar₂, A₁ und A₂ die unter Formel I angegebene Bedeutung haben, mit einer Verbindung der Formel III,
H₂N-R (III)
worin R die unter Formel I angegebene Bedeutung hat, umsetzt;
und gewünschtenfalls eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I in ein Salz umwandelt und/oder ein erhaltenes Gemisch von isomeren Verbindungen der Formel I in die einzelnen Isomeren auftrennt.

2. Verfahren gemäss Anspruch 1 zur Herstellung einer Verbindung der Formel I, worin A₁ und A₂ unabhängig voneinander für Wasserstoff, Niederalkyl; C₂-C₇-Alkenyl; Phenyl oder 1- oder 2-Naphthyl, wobei die drei letztgenannten Reste unsubstituiert oder durch Niederalkyl, Niederalkoxy, Hydroxy, Halogen und/oder Trifluormethyl substituiert sind; Niederalkanoyl, Niederalkylsulfonyl oder Phenylsulfonyl, worin die Phenylgruppe unsubstituiert oder durch Niederalkyl, Niederalkoxy, Hydroxy, Halogen und/oder Trifluormethyl substituiert ist, stehen; oder worin A₁ und A₂ zusammen C₁-C₄-Alkylen bedeuten; worin Ar₁ und Ar₂ unabhängig voneinander Phenyl oder Naphthyl, welches jeweils unsubstituiert oder durch einen oder mehrere Substituenten aus der Gruppe bestehend aus Niederalkyl, C₂-C₇-Alkenyl, C₂-C₇-Alkinyl, C₃-C₄-Alkylen (angeknüpft an zwei benachbarten C-Atomen), C₃-C₈-Cycloalkyl, Phenylniederalkyl, Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy, Niederalkoxy, Halogenniederalkoxy, Phenylniederalkoxy, Phenyloxy, C₂-C₇-Alkenyloxy, Hälogen-C₂-C₇-alkenyloxy, C₂-C₇-Alkinyloxy, C₁-C₂-Alkylendioxy (angeknüpft an zwei benachbarten C-Atomen), Niederalkanoyloxy, Phenylniederalkanoyloxy, Phenylcarbonyloxy, Mercapto, Niederalkylthio, Phenylniederalkylthio, Phenylthio, Niederalkylsulfinyl, Phenylniederalkylsulfinyl, Phenylsulfinyl, Niederalkylsulfonyl, Phenylniederalkylsulfonyl, Phenylsulfonyl, Halogen, Nitro, Amino, Niederalkylamino, C₃-C₈-Cycloalkylamino, Phenylniederalkylamino, Phenylamino, Diniederalkylamino, N-Niederalkyl-N-phenylamino, N-Niederalkyl-N-phenylniederalkylamino, C₄-C₇-Alkylenamino, durch -O-, -S- oder -NR˝- unterbrochenes C₄-C₇-Alkylenamino (worin R˝ für Wasserstoff, Niederalkyl oder Niederalkanoyl steht), Niederalkanoylamino, Phenylniederalkanoylamino, Phenylcarbonylamino, Niederalkanoyl, Phenylniederalkanoyl, Phenylcarbonyl, Carboxy, Niederalkoxycarbonyl, Carbamoyl, N-Niederalkylcarbamoyl, N,N-Diniederalkylcarbamoyl, N-Hydroxycarbamoyl, N-Phenylcarbamoyl, Cyano, Sulfo, Niederalkoxysulfonyl, Sulfamoyl, N-Niederalkylsulfamoyl, N,N-Diniederalkylsulfamoyl und N-Phenylsulfamoyl substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; Pyridyl oder Pyrimidinyl, das unsubstituiert oder durch Niederalkyl, Hydroxy, Niederalkoxy, Halogen, Cyano und/oder Trifluormethyl substituiert ist; oder C₃-C₈-Cycloalkyl bedeuten; die Gruppe -C(=X)- für -C(=O)-, -C(=S)-, -CH₂- oder -C(=CR₁R₂)- steht, wobei R₁ und R₂ unabhängig voneinander Wasserstoff oder Niederalkyl bedeuten, und R Wasserstoff, Niederalkyl; Phenylniederalkyl, Phenyl, 1- oder 2-Naphthyl, wobei in den vier letztgenannten Resten die Phenyl- bzw. Naphthylgruppe jeweils unsubstituiert oder durch Niederalkyl, Niederalkoxy, Hydroxy, Halogen und/oder Trifluormethyl substituiert ist; Amino, Hydroxy oder Niederalkoxy bedeutet, mit der Massgabe, dass R von der Bedeutung Phenyl verschieden ist, wenn A₁ und A₂ für Wasserstoff stehen, Ar₁ und Ar₂ Phenyl bedeuten und die Gruppe -C(=X)- für -C(=O)- steht, und von Salzen davon, sofern salzbildende Gruppen vorliegen; wobei in den vorgenannten Resten Nieder für C₁-C₇- und nieder für -C₁-C₇- steht; dadurch gekennzeichnet, dass man die entsprechend substituierten Ausgangsmaterialien verwendet.

3. Verfahren gemäss Anspruch 2 zur Herstellung einer Verbindung der Formel I gemäss Anspruch 1, worin R die genannten Bedeutungen ausser Phenyl hat, und die übrigen Reste die genannten Bedeutungen haben, und von Salzen davon, sofern salzbildende Gruppen vorliegen, dadurch gekennzeichnet, dass man die entsprechend substituierten Ausgangsmaterialien verwendet.

4. Verfahren gemäss Anspruch 1 zur Herstellung einer Verbindung der Formel I gemäss Anspruch 1, worin A₁ und A₂ unabhängig voneinander für Wasserstoff, Niederalkyl; Phenyl oder 1- oder 2-Naphthyl, wobei die drei letztgenannten Reste unsubstituiert oder durch Niederalkyl, Niederalkoxy, Hydroxy, Halogen und/oder Trifluormethyl substituiert sind; Niederalkanoyl, Niederalkylsulfonyl oder Phenylsulfonyl, worin die Phenylgruppe unsubstituiert oder durch Niederalkyl, Niederalkoxy, Hydroxy, Halogen und/oder Trifluormethyl substituiert ist, stehen; oder worin A₁ und A₂ zusammen C₁-C₄-Alkylen bedeuten; worin Ar₁ und Ar₂ unabhängig voneinander Phenyl oder Naphthyl, welches jeweils unsubstituiert oder durch einen oder mehrere Substituenten aus der Gruppe bestehend aus Niederalkyl, C₂-C₇-Alkenyl, C₂-C₇-Alkinyl, C₃-C₄-Alkylen (angeknüpft an zwei benachbarten C-Atomen), C₃-C₈-Cycloalkyl, Phenylniederalkyl, Phenyl; Niederalkyl, das durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy, Niederalkoxy, Halogenniederalkoxy, Phenylniederalkoxy, Phenyloxy, C₂-C₇-Alkenyloxy, Halogen-C₂-C₇-alkenyloxy, C₂-C₇-Alkinyloxy, C₁-C₂-Alkylendioxy (angeknüpft an zwei benachbarten C-Atomen), Niederalkanoyloxy, Phenylniederalkanoyloxy, Phenylcarbonyloxy, Mercapto, Niederalkylthio, Phenylniederalkylthio, Phenylthio, Niederalkylsulfinyl, Phenylniederalkylsulfinyl, Phenylsulfinyl, Niederalkylsulfonyl, Phenylniederalkylsulfonyl, Phenylsulfonyl, Halogen, Nitro, Amino, Niederalkylamino, C₃-C₈-Cycloalkylamino, Phenylniederalkylamino, Phenylamino, Diniederalkylamino, N-Niederalkyl-N-phenylamino, N-Niederalkyl-N-phenylniederalkylamino, C₄-C₇-Alkylenamino, durch -O-, -S- oder -NR˝- unterbrochenes C₄-C₇-Alkylenamino (worin R˝ für Wasserstoff, Niederalkyl oder Niederalkanoyl steht), Niederalkanoylamino, Phenylniederalkanoylamino, Phenylcarbonylamino, Niederalkanoyl, Phenylniederalkanoyl, Phenylcarbonyl, Carboxy, Niederalkoxycarbonyl, Carbamoyl, N-Niederalkylcarbamoyl, N,N-Diniederalkylcarbamoyl, N-Hydroxycarbamoyl, N-Phenylcarbamoyl, Cyano, Sulfo, Niederalkoxysulfonyl, Sulfamoyl, N-Niederalkylsulfamoyl, N,N-Diniederalkylsulfamoyl und N-Phenylsulfamoyl substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; Pyridyl, das unsubstituiert oder durch Niederalkyl, Hydroxy, Niederalkoxy, Halogen, Cyano und/oder Trifluormethyl substituiert ist; oder C₃-C₈-Cycloalkyl bedeuten; die Gruppe -C(=X)- für -C(=O)-, -C(=S)-, -CH₂- oder -C(=CR₁R₂)- steht, wobei R₁ und R₂ unabhängig voneinander Wasserstoff oder Niederalkyl bedeuten, und R Wasserstoff, Niederalkyl; Phenylniederalkyl, Phenyl, 1- oder 2-Naphthyl, wobei in den vier letztgenannten Resten die Phenyl- bzw. Naphthylgruppe jeweils unsubstituiert oder durch Niederalkyl, Niederalkoxy, Hydroxy, Halogen und/oder Trifluormethyl substituiert ist; Amino, Hydroxy oder Niederalkoxy bedeutet, mit der Massgabe, dass R von der Bedeutung Phenyl verschieden ist, wenn A₁ und A₂ für Wasserstoff stehen, Ar₁ und Ar₂ Phenyl bedeuten und die Gruppe -C(=X)- für -C(=O)- steht, und von Salzen davon, sofern salzbildende Gruppen vorliegen; wobei in den vorgenannten Resten Nieder für C₁-C₇- und nieder für -C₁-C₇- steht,
dadurch gekennzeichnet, dass man die entsprechend substituierten Ausgangsmaterialien verwendet.

5. Verfahren gemäss Anspruch 4 zur Herstellung von einer Verbindung der Formel I, worin A₁ und A₂ unabhängig voneinander Wasserstoff oder Niederalkyl bedeuten, die Gruppe -C(=X)- für -C(=O)-, -C(=S)- oder -C(=CH₂)- steht, und R Wasserstoff oder Niederalkyl bedeutet, und von Salzen davon, sofern salzbildende Gruppen vorliegen; wobei in den vorgenannten Resten Nieder für C₁-C₇- steht, dadurch gekennzeichnet, dass man die entsprechend substituierten Ausgangsmaterialien verwendet.

6. Verfahren gemäss Anspruch 1 zur Herstellung einer Verbindung der Formel I, worin A₁ und A₂ unabhängig voneinander für Wasserstoff; Niederalkyl; C₂-C₇-Alkenyl; oder Niederalkanoyl stehen; oder worin A₁ und A₂ zusammen C₁-C₄-Alkylen bedeuten; worin Ar₁ und Ar₂ unabhängig voneinander Phenyl oder Naphthyl, welches jeweils unsubstituiert oder durch einen oder mehrere Substituenten aus der Gruppe bestehend aus Niederalkyl, C₃-C₄-Alkylen (angeknüpft an zwei benachbarten C-Atomen), Hydroxy, Phenyloxy, Halogen, Nitro, Amino, Niederalkylamino, Diniederalkylamino, Niederalkanoylamino, Carboxy, Niederalkoxycarbonyl, Carbamoyl, N-Niederalkylcarbamoyl, N,N-Diniederalkylcarbamoyl und Cyano substituiert ist; Pyridyl; Pyrimidinyl; oder C₃-C₈-Cycloalkyl bedeuten; die Gruppe -C(=X)- für -C(=O)- oder -C(=S)- steht, und R Wasserstoff, Niederalkyl; Phenylniederalkyl, Amino oder Hydroxy bedeutet, und von Salzen davon, sofern salzbildende Gruppen vorliegen; wobei in den vorgenannten Resten Nieder für C₁-C₇- und nieder für -C₁-C₇- steht, dadurch gekennzeichnet, dass man die entsprechend substituierten Ausgangsmaterialien verwendet.

7. Verfahren gemäss Anspruch 1 zur Herstellung einer Verbindung der Formel I gemäss Anspruch 1, worin A₁ und A₂ unabhängig voneinander für Wasserstoff oder Methyl stehen; oder worin A₁ und A₂ zusammen -(CH₂)₂- oder -(CH₂)₃- bedeuten; Ar₁ und Ar₂ unabhängig voneinander für Phenyl oder Naphthyl, welches jeweils unsubstituiert oder durch einen oder mehrere Substituenten aus der Gruppe bestehend aus Niederalkyl, Niederalkoxy, Phenylniederalkoxy, Hydroxy, Niederalkanoyloxy, Nitro, Amino, Niederalkylamino, Diniederalkylamino, Niederalkanoylamino, Halogen, Trifluormethyl, Carboxy, Niederalkoxycarbonyl, Carbamoyl, N-Niederalkylcarbamoyl, N,N-Diniederalkylcarbamoyl, Cyano, Niederalkanoyl, Benzoyl, Niederalkoxysulfonyl und Sulfamoyl, N-Niederalkylsulfamoyl und N,N-Diniederalkylsulfamoyl substituiert ist; oder für Cyclopentyl, Cyclohexyl oder Pyridyl stehen; die Gruppe -C(=X)- für -C(=O)-, -C(=S)- oder -C(=CH₂)- steht, und R Wasserstoff oder Niederalkyl bedeutet, und von pharmazeutisch anwendbaren Salzen davon; wobei in den vorgenannten Resten Nieder für C₁-C₇- und nieder für -C₁-C₇- steht, dadurch gekennzeichnet, dass man die entsprechend substituierten Ausgangsmaterialien verwendet.

8. Verfahren gemäss Anspruch 1 zur Herstellung einer Verbindung der Formel I gemäss Anspruch 1, worin A₁ und A₂ für Wasserstoff stehen; Ar₁ und Ar₂ unabhängig voneinander Phenyl, das unsubstituiert oder durch Niederalkyl, Trifluormethyl, Phenyl, Hydroxy, Niederalkoxy, Benzyloxy, Amino, Diniederalkylamino, Niederalkanoylamino, Halogen, Carboxy, Niederalkoxycarbonyl, Carbamoyl, N,N-Diniederalkylcarbamoyl oder Cyano substituiert ist; oder Cyclohexyl bedeuten; die Gruppe -C(=X)- für -C(=O)-, -C(=S)- oder -C(=CH₂)- steht, und R Wasserstoff bedeutet, und von pharmazeutisch anwendbaren Salzen davon; wobei in den vorgenannten Resten Nieder für C₁-C₇- und nieder für -C₁-C₇- steht, dadurch gekennzeichnet, dass man die entsprechend substituierten Ausgangsmaterialien verwendet.

9. Verfahren gemäss Anspruch 1 zur Herstellung von 4,5-Bis(anilino)phthalimid gemäss Anspruch 1, dadurch gekennzeichnet, dass man die entsprechend substituierten Ausgangsmaterialien verwendet.

10. Verfahren gemäss Anspruch 1 zur Herstellung von 4,5-Bis(4-fluoranilino)phthalimid gemäss Anspruch 1, dadurch gekennzeichnet, dass man die entsprechend substituierten Ausgangsmaterialien verwendet.

11. Verfahren gemäss Anspruch 1 zur Herstellung von 4,5-Bis(cyclohexylamino)phthalimid gemäss Anspruch 1 oder einem pharmazeutisch anwendbaren Salz davon, dadurch gekennzeichnet, dass man die entsprechend substituierten Ausgangsmaterialien verwendet.

12. Verfahren gemäss Anspruch 1 zur Herstellung von 5,8-Diphenyl-5,8-diaza-5,6,7,8-tetrahydro-naphthalin -2,3-dicarbonsäureamid gemäss Anspruch 1, dadurch gekennzeichnet, dass man die entsprechend substituierten Ausgangsmaterialien verwendet.

13. Verfahren gemäss Anspruch 1 zur Herstellung von 4,5-Bis(2-nitroanilino)phthalimid gemäss Anspruch 1, dadurch gekennzeichnet, dass man die entsprechend substituierten Ausgangsmaterialien verwendet.

14. Verfahren gemäss Anspruch 1 zur Herstellung von 4,5-Bis-(4-aminoanilino)-phthalimid gemäss Anspruch 1 oder einem pharmazeutisch annehmbaren Salz davon, dadurch gekennzeichnet, dass man die entsprechend substituierten Ausgangsmaterialien verwendet.

15. Verfahren gemäss Anspruch 1 zur Herstellung von 4,5-Bis(2-pyridinamino)phthalimid gemäss Anspruch 1 oder einem pharmazeutisch annehmbaren Salz davon, dadurch gekennzeichnet, dass man die entsprechend substituierten Ausgangsmaterialien verwendet.

16. Verfahren gemäss Anspruch 1 zur Herstellung von 4,5-Bis(2-pyrimidinamino)-phthalimid gemäss Anspruch 1 oder einem pharmazeutisch annehmbaren Salz davon, dadurch gekennzeichnet, dass man die entsprechend substituierten Ausgangsmaterialien verwendet

17. Verfahren gemäss Anspruch 1 zur Herstellung von 4-(N-Methyl-N-phenylamino)-5-anilino-phthalimid gemäss Anspruch 1, dadurch gekennzeichnet, dass man die entsprechend substituierten Ausgangsmaterialien verwendet.

18. Verfahren gemäss Anspruch 1 zur Herstellung von 4,5-Bis(anilino)-N⁴,N⁵-propan-1,3-diyl-phthalimid gemäss Anspruch 1, dadurch gekennzeichnet, dass man die entsprechend substituierten Ausgangsmaterialien verwendet.

19. Verfahren gemäss Anspruch 1 zur Herstellung von 4,5-Bis(N-allylanilino)phthalimid gemäss Anspruch 1, dadurch gekennzeichnet, dass man die entsprechend substituierten Ausgangsmaterialien verwendet.

20. Verfahren zur Herstellung eines pharmazeutischen Präparates enthaltend eine gemäss einem der Ansprüche 1 bis 19 hergestellte Verbindung der Formel I, oder ein pharmazeutisch annehmbares Salz davon, sofern salzbildende Gruppen vorliegen, dadurch gekennzeichnet, dass man die Verbindung der Formel I mit mindestens einem pharmazeutisch annehmbaren Trägermaterial versetzt.

## Claims (Claims for the following Contracting State(s): AT, BE, CH, DE, DK, FR, GB, IT, LI, LU, MC, NL, PT, SE)

1. A compound of formula I 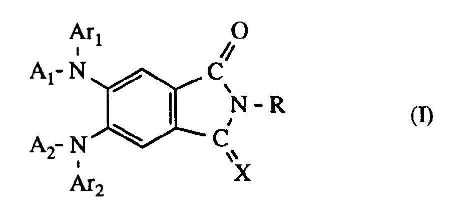 wherein
A₁ and A₂ are each independently of the other hydrogen, C₁-C₇alkyl, C₂-C₇alkenyl, C₂-C₇alkynyl, phenyl or naphthyl that is unsubstituted or substituted by one or more substituents from the group consisting of: C₁-C₇alkyl, C₂-C₇alkenyl, C₂-C₇alkynyl, C₃-C₄-alkylene (linked to two adjacent carbon atoms), C₃-C₈cycloalkyl, phenyl-C₁-C₇alkyl, phenyl; C₁-C₇alkyl substituted by hydroxy, C₁-C₇alkoxy, phenyl-C₁-C₇alkoxy, C₁-C₇-alkanoyloxy, halogen, amino, C₁-C₇alkylamino, di-C₁-C₇alkylamino, mercapto, C₁-C₇-alkylthio, C₁-C₇alkylsulfinyl, C₁-C₇alkylsulfonyl, carboxy, C₁-C₇alkoxycarbonyl, carbamoyl, N-C₁-C₇alkylcarbamoyl, N,N-di-C₁-C₇alkylcarbamoyl and/or by cyano; hydroxy, C₁-C₇alkoxy, halo-C₁-C₇alkoxy, phenyl-C₁-C₇alkoxy, phenoxy, C₂-C₇alkenyloxy, halo-C₂-C₇alkenyloxy, C₂-C₇alkynyloxy, C₁-C₂alkylenedioxy (linked to two adjacent carbon atoms), C₁-C₇alkanoyloxy, phenyl-C₁-C₇alkanoyloxy, phenylcarbonyloxy, mercapto, C₁-C₇alkylthio, phenyl-C₁-C₇alkylthio, phenylthio, C₁-C₇alkylsulfinyl, phenyl-C₁-C₇alkylsulfinyl, phenylsulfinyl, C₁-C₇alkylsulfonyl, phenyl-C₁-C₇alkylsulfonyl, phenylsulfonyl, halogen, nitro, amino, C₁-C₇alkylamino, C₃-C₈cycloalkylamino, phenyl-C₁-C₇alkylamino, phenylamino, di-C₁-C₇alkylamino, N-C₁-C₇alkyl-N-phenylamino, N-C₁-C₇alkyl-N-phenyl-C₁-C₇alkylamino, C₄-C₇alkyleneamino, C₄-C₇alkyleneamino interrupted by -O-, -S- or -NR˝- (wherein R˝ is hydrogen, C₁-C₇alkyl or C₁-C₇alkanoyl), C₁-C₇alkanoylamino, phenyl-C₁-C₇alkanoylamino, phenylcarbonylamino, C₁-C₇alkanoyl, phenyl-C₁-C₇alkanoyl, phenylcarbonyl, carboxy, C₁-C₇alkoxycarbonyl, carbamoyl, C₁-C₇alkylcarbamoyl, N,N-di-C₁-C₇alkylcarbamoyl, N-hydroxycarbamoyl, N-phenylcarbamoyl, cyano, sulfo, C₁-C₇alkoxysulfonyl, sulfamoyl, N-C₁-C₇alkylsulfamoyl, N,N-di-C₁-C₇alkylsulfamoyl and N-phenylsulfamoyl (phenyl groups occurring in the substituents each being unsubstituted or substituted by C₁-C₇alkyl, C₁-C₇alkoxy, hydroxy, halogen and/or by trifluoromethyl); C₁-C₇alkanoyl, halo-C₁-C₇alkanoyl, phenyl- or naphthyl-C₁-C₇alkanoyl that in the phenyl or naphthyl radical is unsubstituted or substituted as above or phenyl- or naphthyl-carbonyl that in the phenyl or naphthyl radical is unsubstituted or substituted as above, C₁-C₇alkylsulfonyl or phenyl- or naphthyl-sulfonyl wherein phenyl or naphthyl is unsubstituted or substituted as above; or wherein
A₁ and A₂ together form unsubstituted or C₁-C₇alkyl- or hydroxy-substituted C₁-C₄-alkylene ;
Ar₁ and Ar₂ are each independently of the other phenyl or naphthyl that is unsubstituted or substituted as above; imidazolyl, triazolyl, pyridyl, pyrimidinyl or triazinyl that is unsubstituted or substituted by C₁-C₇alkyl, hydroxy, C₁-C₇alkoxy, halogen, cyano and/or by trifluoromethyl; or C₃-C₈cycloalkyl that is unsubstituted or substituted by C₁-C₇alkyl or by hydroxy;
the group -C(=X)- is -C(=O)-, -C(=S)-, -CH₂- or -C(=CR₁R₂)- wherein R₁ and R₂ are each independently of the other hydrogen or C₁-C₇alkyl; and
R is hydrogen, C₁-C₇alkyl, aryl-C₁-C₇alkyl or aryl wherein aryl is phenyl or naphthyl that is unsubstituted or substituted as above; amino, hydroxy or C₁-C₇alkoxy; with the proviso that R is other than phenyl when A₁ and A₂ are hydrogen, Ar₁ and Ar₂ are phenyl and the group -C(=X)- is -C(=O)-; or a salt thereof when salt-forming groups are present.

2. A compound of formula I according to claim 1 wherein
A₁ and A₂ are each independently of the other hydrogen, lower alkyl; C₂-C₇alkenyl; phenyl or 1-naphthyl or 2-naphthyl, the three last-mentioned radicals being unsubstituted or substituted by lower alkyl, lower alkoxy, hydroxy, halogen and/or by trifluoromethyl; lower alkanoyl, lower alkylsulfonyl or phenylsulfonyl wherein the phenyl group is unsubstituted or substituted by lower alkyl, lower alkoxy, hydroxy, halogen and/or by trifluoromethyl; or wherein
A₁ and A₂ together are C₁-C₄alkylene; wherein
Ar₁ and Ar₂ are each independently of the other phenyl or naphthyl, each of which is unsubstituted or substituted by one or more substituents from the group consisting of: lower alkyl, C₂-C₇alkenyl, C₂-C₇alkynyl, C₃-C₄alkylene (linked to two adjacent carbon atoms), C₃-C₈cycloalkyl, phenyl-lower alkyl, phenyl; lower alkyl substituted by hydroxy, lower alkoxy, phenyl-lower alkoxy, lower alkanoyloxy, halogen, amino, lower alkylamino, dilower alkylamino, mercapto, lower alkylthio, lower alkylsulfinyl, lower alkylsulfonyl, carboxy, lower alkoxycarbonyl, carbamoyl, N-lower alkylcarbamoyl, N,N-di-lower alkylcarbamoyl and/or by cyano; hydroxy, lower alkoxy, halo-lower alkoxy, phenyl-lower alkoxy, phenoxy, C₂-C₇alkenyloxy, halo-C₂-C₇alkenyloxy, C₂-C₇alkynyloxy, C₁-C₂-alkylenedioxy (linked to two adjacent carbon atoms), lower alkanoyloxy, phenyl-lower alkanoyloxy, phenylcarbonyloxy, mercapto, lower alkylthio, phenyl-lower alkylthio, phenylthio, lower alkylsulfinyl, phenyl-lower alkylsulfinyl, phenylsulfinyl, lower alkylsulfonyl, phenyl-lower alkylsulfonyl, phenylsulfonyl, halogen, nitro, amino, lower alkylamino, C₃-C₈cycloalkylamino, phenyl-lower alkylamino, phenylamino, di-lower alkylamino, N-lower alkyl-N-phenylamino, N-lower alkyl-N-phenyl-lower alkylamino, C₄-C₇-alkyleneamino, C₄-C₇alkyleneamino interrupted by -O-, -S- or -NR˝- (wherein R˝ is hydrogen, lower alkyl or lower alkanoyl), lower alkanoylamino, phenyl-lower alkanoylamino, phenylcarbonylamino, lower alkanoyl, phenyl-lower alkanoyl, phenylcarbonyl, carboxy, lower alkoxycarbonyl, carbamoyl, N-lower alkylcarbamoyl, N,N-di-lower alkylcarbamoyl, N-hydroxycarbamoyl, N-phenylcarbamoyl, cyano, sulfo, lower alkoxysulfonyl, sulfamoyl, N-lower alkylsulfamoyl, N,N-di-lower alkylsulfamoyl and N-phenylsulfamoyl (phenyl groups occurring in the substituents each being unsubstituted or substituted by lower alkyl, lower alkoxy, hydroxy, halogen and/or by trifluoromethyl); pyridyl or pyrimidinyl that is unsubstituted or substituted by lower alkyl, hydroxy, lower alkoxy, halogen, cyano and/or by trifluoromethyl; or C₃-C₈cycloalkyl;
the group -C(=X)- is -C(=O)-, -C(=S)-, -CH₂- or -C(=CR₁R₂)- wherein R₁ and R₂ are each independently of the other hydrogen or lower alkyl, and
R is hydrogen, lower alkyl; phenyl-lower alkyl, phenyl, 1-naphthyl or 2-naphthyl, in the four last-mentioned radicals the phenyl or naphthyl group being unsubstituted or substituted by lower alkyl, lower alkoxy, hydroxy, halogen and/or by trifluoromethyl; or R is amino, hydroxy or lower alkoxy; with the proviso that R is other than phenyl when A₁ and A₂ are hydrogen, Ar₁ and Ar₂ are phenyl and the group -C(=X)- is -C(=O)-; or a salt thereof when salt-forming groups are present; in the above-mentioned radicals "lower" representing "C₁-C₇-" and "-lower" representing "-C₁-C₇-".

3. A compound of formula I according to claim 2 wherein R is as defined except for phenyl, and the other radicals are as defined, or a salt thereof when salt-forming groups are present.

4. A compound of formula I according to claim 1 wherein
A₁ and A₂ are each independently of the other hydrogen, lower alkyl; phenyl or 1-naphthyl or 2-naphthyl, the three last-mentioned radicals being unsubstituted or substituted by lower alkyl, lower alkoxy, hydroxy, halogen and/or by trifluoromethyl; lower alkanoyl, lower alkylsulfonyl or phenylsulfonyl wherein the phenyl group is unsubstituted or substituted by lower alkyl, lower alkoxy, hydroxy, halogen and/or by trifluoromethyl; or wherein
A₁ and A₂ together are C₁-C₄alkylene; wherein
Ar₁ and Ar₂ are each independently of the other phenyl or naphthyl, each of which is unsubstituted or substituted by one or more substituents from the group consisting of: lower alkyl, C₂-C₇alkenyl, C₂-C₇alkynyl, C₃-C₄alkylene (linked to two adjacent carbon atoms), C₃-C₈cycloalkyl, phenyl-lower alkyl, phenyl; lower alkyl substituted by hydroxy, lower alkoxy, phenyl-lower alkoxy, lower alkanoyloxy, halogen, amino, lower alkylamino, di-lower alkylamino, mercapto, lower alkylthio, lower alkylsulfinyl, lower alkylsulfonyl, carboxy, lower alkoxycarbonyl, carbamoyl, N-lower alkylcarbamoyl, N,N-di-lower alkylcarbamoyl and/or by cyano; hydroxy, lower alkoxy, halo-lower alkoxy, phenyl-lower alkoxy, phenoxy, C₂-C₇alkenyloxy, halo-C₂-C₇alkenyloxy, C₂-C₇alkynyloxy, C₁-C₂alkylenedioxy (linked to two adjacent carbon atoms), lower alkanoyloxy, phenyl-lower alkanoyloxy, phenylcarbonyloxy, mercapto, lower alkylthio, phenyl-lower alkylthio, phenylthio, lower alkylsulfinyl, phenyl-lower alkylsulfinyl, phenylsulfinyl, lower alkylsulfonyl, phenyl-lower alkylsulfonyl, phenylsulfonyl, halogen, nitro, amino, lower alkylamino, C₃-C₈cycloalkylamino, phenyl-lower alkylamino, phenylamino, di-lower alkylamino, N-lower alkyl-N-phenylamino, N-lower alkyl-N-phenyl-lower alkylamino, C₄-C₇alkyleneamino, C₄-C₇alkyleneamino interrupted by -O-, -S- or -NR˝- (wherein R˝ is hydrogen, lower alkyl or lower alkanoyl), lower alkanoylamino, phenyl-lower alkanoylamino, phenylcarbonylamino, lower alkanoyl, phenyl-lower alkanoyl, phenylcarbonyl, carboxy, lower alkoxycarbonyl, carbamoyl, N-lower alkylcarbamoyl, N,N-di-lower alkylcarbamoyl, N-hydroxycarbamoyl, N-phenylcarbamoyl, cyano, sulfo, lower alkoxysulfonyl, sulfamoyl, N-lower alkylsulfamoyl, N,N-di-lower alkylsulfamoyl and N-phenylsulfamoyl (phenyl groups occurring in the substituents each being unsubstituted or substituted by lower alkyl, lower alkoxy, hydroxy, halogen and/or by trifluoromethyl); pyridyl that is unsubstituted or substituted by lower alkyl, hydroxy, lower alkoxy, halogen, cyano and/or by trifluoromethyl; or C₃-C₈cycloalkyl;
the group -C(=X)- is -C(=O)-, -C(=S)-, -CH₂- or -C(=CR₁R₂)- wherein R₁ and R₂ are each independently of the other hydrogen or lower alkyl, and
R is hydrogen, lower alkyl; phenyl-lower alkyl, phenyl, 1-naphthyl or 2-naphthyl, in the four last-mentioned radicals the phenyl or naphthyl group being unsubstituted or substituted by lower alkyl, lower alkoxy, hydroxy, halogen and/or by trifluoromethyl; or R is amino, hydroxy or lower alkoxy; with the proviso that R is other than phenyl when A₁ and A₂ are hydrogen, Ar₁ and Ar₂ are phenyl and the group -C(=X)- is -C(=O)-; or a salt thereof when salt-forming groups are present; in the above-mentioned radicals "lower" representing "C₁-C₇-" and "-lower" representing "-C₁-C₇-".

5. A compound of formula I according to claim 2 wherein A₁ and A₂ are each independently of the other hydrogen or lower alkyl, the group -C(=X)- is -C(=O)-, -C(=S)- or -C(=CH₂)-, and R is hydrogen or lower alkyl; or a salt thereof when salt-forming groups are present; in the above-mentioned radicals "lower" representing "C₁-C₇-".

6. A compound of formula I according to claim 1 wherein A₁ and A₂ are each independently of the other hydrogen; lower alkyl; C₂-C₇alkenyl; or lower alkanoyl; or wherein A₁ and A₂ together are C₁-C₄alkylene; wherein Ar₁ and Ar₂ are each independently of the other phenyl or naphthyl, each of which is unsubstituted or substituted by one or more substituents from the group consisting of: lower alkyl, C₃-C₄alkylene (linked to two adjacent carbon atoms), hydroxy, phenoxy, halogen, nitro, amino, lower alkylamino, di-lower alkylamino, lower alkanoylamino, carboxy, lower alkoxycarbonyl, carbamoyl, N-lower alkylcarbamoyl, N,N-di-lower alkylcarbamoyl and cyano; pyridyl; pyrimidinyl; or C₃-C₈cycloalkyl; the group -C(=X)- is -C(=O)- or -C(=S)-; and R is hydrogen, lower alkyl, phenyl-lower alkyl, amino or hydroxy; or a salt thereof when salt-forming groups are present; in the above-mentioned radicals "lower" representing "C₁-C₇-" and "-lower" representing "-C₁-C₇-".

7. A compound of formula I according to claim 1 wherein A₁ and A₂ are each independently of the other hydrogen or methyl; or wherein A₁ and A₂ together are -(CH₂)₂- or -(CH₂)₃-; Ar₁ and Ar₂ are each independently of the other phenyl or naphthyl, each of which is unsubstituted or substituted by one or more substituents from the group consisting of: lower alkyl, lower alkoxy, phenyl-lower alkoxy, hydroxy, lower alkanoyloxy, nitro, amino, lower alkylamino, di-lower alkylamino, lower alkanoylamino, halogen, trifluoromethyl, carboxy, lower alkoxycarbonyl, carbamoyl, N-lower alkylcarbamoyl, N,N-di-lower alkylcarbamoyl, cyano, lower alkanoyl, benzoyl, lower alkoxysulfonyl and sulfamoyl, N-lower alkylsulfamoyl and N,N-di-lower alkylsulfamoyl; or cyclopentyl; cyclohexyl; or pyridyl; the group -C(=X)- is -C(=O)-, -C(=S)- or -C(=CH₂)-, and R is hydrogen or lower alkyl; or a pharmaceutically acceptable salt thereof; in the above-mentioned radicals "lower" representing "C₁-C₇-" and "-lower" representing "-C₁-C₇-".

8. A compound of formula I according to claim 1 wherein A₁ and A₂ are hydrogen; Ar₁ and Ar₂ are each independently of the other phenyl that is unsubstituted or substituted by lower alkyl, trifluoromethyl, phenyl, hydroxy, lower alkoxy, benzyloxy, amino, di-lower alkylamino, lower alkanoylamino, halogen, carboxy, lower alkoxycarbonyl, carbamoyl, N,N-di-lower alkylcarbamoyl or by cyano; or cyclohexyl; the group -C(=X)- is -C(=O)-, -C(=S)- or -C(=CH₂)-, and R is hydrogen; or a pharmaceutically acceptable salt thereof; in the above-mentioned radicals "lower" representing "C₁-C₇-" and "-lower" representing "-C₁-C₇-".

9. 4,5-Bis(anilino)phthalimide according to claim 1.

10. 4,5-Bis(4-fluoroanilino)phthalimide according to claim 1.

11. 4,5-Bis(cyclohexylamino)phthalimide according to claim 1 or a pharmaceutically acceptable salt thereof.

12. 5,8-Diphenyl-5,8-diaza-5,6,7,8-tetrahydronaphthalene-2,3-dicarboxylic acid amide according to claim 1.

13. 4,5-Bis(2-nitroanilino)phthalimide according to claim 1.

14. 4,5-Bis(4-aminoanilino)phthalimide according to claim 1 or a pharmaceutically acceptable salt thereof.

15. 4,5-Bis(2-pyridineamino)phthalimide according to claim 1 or a pharmaceutically acceptable salt thereof.

16. 4,5-Bis(2-pyrimidineamino)phthalimide according to claim 1 or a pharmaceutically acceptable salt thereof.

17. 4-(N-Methyl-N-phenylamino)-5-anilinophthalimide according to claim 1.

18. 4,5-Bis(anilino)-N⁴,N⁵-propane-1,3-diyl-phthalimide according to claim 1.

19. 4,5-Bis(N-allylanilino)phthalimide according to claim 1.

20. A pharmaceutical composition comprising a compound of formula I according to any one of claims 1 to 19 and at least one pharmaceutically acceptable carrier.

21. A compound of formula I according to any one of claims 1 to 19 for use in a method for the therapeutic treatment of the animal or human body.

22. The use of a compound according to any one of claims 1 to 19 for the preparation of a pharmaceutical composition for the treatment of diseases responsive to the inhibition of tyrosine protein kinases or serine/threonine kinases.

23. A process for the preparation of a compound of formula I according to claim 1, which process comprises
(a) reacting a compound of formula II 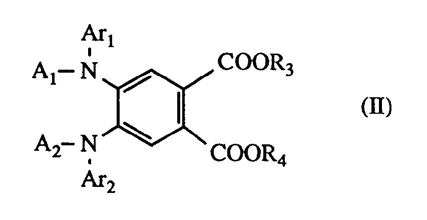 wherein Ar₁, Ar₂, A₁ and A₂ are as defined under formula I and R₃ and R₄ are each independently of the other aryl or C₁-C₇alkyl, with a compound of formula III
H₂N-R (III)
wherein R is as defined under formula I, or
(b) reacting a compound of formula IV 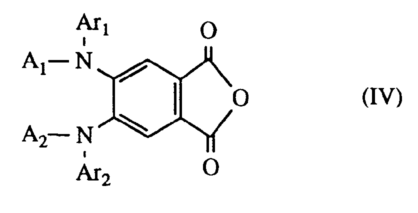 wherein Ar₁, Ar₂, A₁ and A₂ are as defined under formula I, with a compound of formula III
H₂N-R (III)
wherein R is as defined under formula I;
and, if desired, converting a resulting compound of formula I into a different compound of formula I, and/or converting a resulting salt into the free compound or into a different salt, and/or converting a resulting free compound of formula I into a salt and/or separating a resulting mixture of isomeric compounds of formula I into the individual isomers.

## Claims (Claims for the following Contracting State(s): ES, GR)

1. A process for the preparation of a compound of formula I 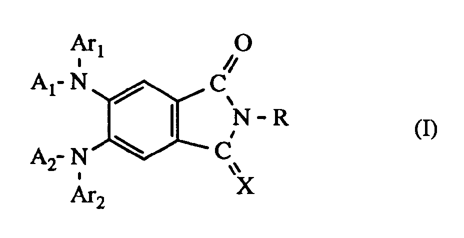 wherein
A₁ and A₂ are each independently of the other hydrogen, C₁-C₇alkyl, C₂-C₇alkenyl, C₂-C₇alkynyl, phenyl or naphthyl that is unsubstituted or substituted by one or more substituents from the group consisting of: C₁-C₇alkyl, C₂-C₇alkenyl, C₂-C₇alkynyl, C₃-C₄-alkylene (linked to two adjacent carbon atoms), C₃-C₈cycloalkyl, phenyl-C₁-C₇alkyl, phenyl; C₁-C₇alkyl substituted by hydroxy, C₁-C₇alkoxy, phenyl-C₁-C₇alkoxy, C₁-C₇-alkanoyloxy, halogen, amino, C₁-C₇alkylamino, di-C₁-C₇alkylamino, mercapto, C₁-C₇-alkylthio, C₁-C₇alkylsulfinyl, C₁-C₇alkylsulfonyl, carboxy, C₁-C₇alkoxycarbonyl, carbamoyl, N-C₁-C₇alkylcarbamoyl, N,N-di-C₁-C₇alkylcarbamoyl and/or by cyano; hydroxy, C₁-C₇alkoxy, halo-C₁-C₇alkoxy, phenyl-C₁-C₇alkoxy, phenoxy, C₂-C₇alkenyloxy, halo-C₂-C₇alkenyloxy, C₂-C₇alkynyloxy, C₁-C₂alkylenedioxy (linked to two adjacent carbon atoms), C₁-C₇alkanoyloxy, phenyl-C₁-C₇alkanoyloxy, phenylcarbonyloxy, mercapto, C₁-C₇alkylthio, phenyl-C₁-C₇alkylthio, phenylthio, C₁-C₇alkylsulfinyl, phenyl-C₁-C₇alkylsulfinyl, phenylsulfinyl, C₁-C₇alkylsulfonyl, phenyl-C₁-C₇alkylsulfonyl, phenylsulfonyl, halogen, nitro, amino, C₁-C₇alkylamino, C₃-C₈cycloalkylamino, phenyl-C₁-C₇alkylamino, phenylamino, di-C₁-C₇alkylamino, N-C₁-C₇alkyl-N-phenylamino, N-C₁-C₇alkyl-N-phenyl-C₁-C₇alkylamino, C₄-C₇alkyleneamino, C₄-C₇alkyleneamino interrupted by -O-, -S- or -NR˝- (wherein R˝ is hydrogen, C₁-C₇alkyl or C₁-C₇alkanoyl), C₁-C₇alkanoylamino, phenyl-C₁-C₇alkanoylamino, phenylcarbonylamino, C₁-C₇alkanoyl, phenyl-C₁-C₇alkanoyl, phenylcarbonyl, carboxy, C₁-C₇alkoxycarbonyl, carbamoyl, C₁-C₇alkylcarbamoyl, N,N-di-C₁-C₇alkylcarbamoyl, N-hydroxycarbamoyl, N-phenylcarbamoyl, cyano, sulfo, C₁-C₇alkoxysulfonyl, sulfamoyl, N-C₁-C₇alkylsulfamoyl, N,N-di-C₁-C₇alkylsulfamoyl and N-phenylsulfamoyl (phenyl groups occurring in the substituents each being unsubstituted or substituted by C₁-C₇alkyl, C₁-C₇alkoxy, hydroxy, halogen and/or by trifluoromethyl); C₁-C₇alkanoyl, halo-C₁-C₇alkanoyl, phenyl- or naphthyl-C₁-C₇alkanoyl that in the phenyl or naphthyl radical is unsubstituted or substituted as above or phenyl- or naphthyl-carbonyl that in the phenyl or naphthyl radical is unsubstituted or substituted as above, C₁-C₇alkylsulfonyl or phenyl- or naphthyl-sulfonyl wherein phenyl or naphthyl is unsubstituted or substituted as above; or wherein
A₁ and A₂ together form unsubstituted or C₁-C₇alkyl- or hydroxy-substituted C₁-C₄-alkylene ;
Ar₁ and Ar₂ are each independently of the other phenyl or naphthyl that is unsubstituted or substituted as above; imidazolyl, triazolyl, pyridyl, pyrimidinyl or triazinyl that is unsubstituted or substituted by C₁-C₇alkyl, hydroxy, C₁-C₇alkoxy, halogen, cyano and/or by trifluoromethyl; or C₃-C₈cycloalkyl that is unsubstituted or substituted by C₁-C₇alkyl or by hydroxy;
the group -C(=X)- is -C(=O)-, -C(=S)-, -CH₂- or -C(=CR₁R₂)- wherein R₁ and R₂ are each independently of the other hydrogen or C₁-C₇alkyl; and
R is hydrogen, C₁-C₇alkyl, aryl-C₁-C₇alkyl or aryl wherein aryl is phenyl or naphthyl that is unsubstituted or substituted as above; amino, hydroxy or C₁-C₇alkoxy; with the proviso that R is other than phenyl when A₁ and A₂ are hydrogen, Ar₁ and Ar₂ are phenyl and the group -C(=X)- is -C(=O)-; or a salt thereof when salt-forming groups are present, which process comprises
(a) reacting a compound of formula II 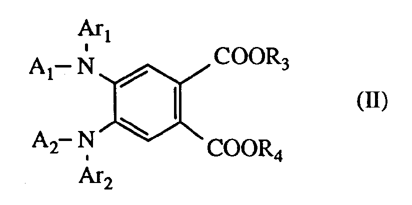 wherein Ar₁, Ar₂, A₁ and A₂ are as defined under formula I and R₃ and R₄ are each independently of the other aryl or C₁-C₇alkyl, with a compound of formula III
H₂N-R (III)
wherein R is as defined under formula I, or
(b) reacting a compound of formula IV 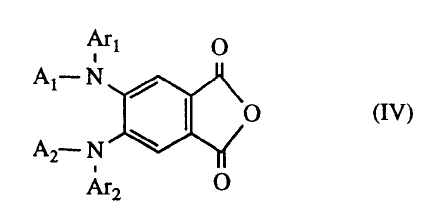 wherein Ar₁, Ar₂, A₁ and A₂ are as defined under formula I, with a compound of formula III
H₂N-R (III)
wherein R is as defined under formula I;
and, if desired, converting a resulting compound of formula I into a different compound of formula I, and/or converting a resulting salt into the free compound or into a different salt, and/or converting a resulting free compound of formula I into a salt and/or separating a resulting mixture of isomeric compounds of formula I into the individual isomers.

2. A process according to claim 1 for the preparation of a compound of formula I wherein A₁ and A₂ are each independently of the other hydrogen, lower alkyl; C₂-C₇alkenyl; phenyl or 1-naphthyl or 2-naphthyl, the three last-mentioned radicals being unsubstituted or substituted by lower alkyl, lower alkoxy, hydroxy, halogen and/or by trifluoromethyl; lower alkanoyl, lower alkylsulfonyl or phenylsulfonyl wherein the phenyl group is unsubstituted or substituted by lower alkyl, lower alkoxy, hydroxy, halogen and/or by trifluoromethyl; or wherein
A₁ and A₂ together are C₁-C₄alkylene; wherein
Ar₁ and Ar₂ are each independently of the other phenyl or naphthyl, each of which is unsubstituted or substituted by one or more substituents from the group consisting of: lower alkyl, C₂-C₇alkenyl, C₂-C₇alkynyl, C₃-C₄alkylene (linked to two adjacent carbon atoms), C₃-C₈cycloalkyl, phenyl-lower alkyl, phenyl; lower alkyl substituted by hydroxy, lower alkoxy, phenyl-lower alkoxy, lower alkanoyloxy, halogen, amino, lower alkylamino, di-lower alkylamino, mercapto, lower alkylthio, lower alkylsulfinyl, lower alkylsulfonyl, carboxy, lower alkoxycarbonyl, carbamoyl, N-lower alkylcarbamoyl, N,N-di-lower alkylcarbamoyl and/or by cyano; hydroxy, lower alkoxy, halo-lower alkoxy, phenyl-lower alkoxy, phenoxy, C₂-C₇alkenyloxy, halo-C₂-C₇alkenyloxy, C₂-C₇alkynyloxy, C₁-C₂-alkylenedioxy (linked to two adjacent carbon atoms), lower alkanoyloxy, phenyl-lower alkanoyloxy, phenylcarbonyloxy, mercapto, lower alkylthio, phenyl-lower alkylthio, phenylthio, lower alkylsulfinyl, phenyl-lower alkylsulfinyl, phenylsulfinyl, lower alkylsulfonyl, phenyl-lower alkylsulfonyl, phenylsulfonyl, halogen, nitro, amino, lower alkylamino, C₃-C₈cycloalkylamino, phenyl-lower alkylamino, phenylamino, di-lower alkylamino, N-lower alkyl-N-phenylamino, N-lower alkyl-N-phenyl-lower alkylamino, C₄-C₇-alkyleneamino, C₄-C₇alkyleneamino interrupted by -O-, -S- or -NR˝- (wherein R˝ is hydrogen, lower alkyl or lower alkanoyl), lower alkanoylamino, phenyl-lower alkanoylamino, phenylcarbonylamino, lower alkanoyl, phenyl-lower alkanoyl, phenylcarbonyl, carboxy, lower alkoxycarbonyl, carbamoyl, N-lower alkylcarbamoyl, N,N-di-lower alkylcarbamoyl, N-hydroxycarbamoyl, N-phenylcarbamoyl, cyano, sulfo, lower alkoxysulfonyl, sulfamoyl, N-lower alkylsulfamoyl, N,N-di-lower alkylsulfamoyl and N-phenylsulfamoyl (phenyl groups occurring in the substituents each being unsubstituted or substituted by lower alkyl, lower alkoxy, hydroxy, halogen and/or by trifluoromethyl); pyridyl or pyrimidinyl that is unsubstituted or substituted by lower alkyl, hydroxy, lower alkoxy, halogen, cyano and/or by trifluoromethyl; or C₃-C₈cycloalkyl;
the group -C(=X)- is -C(=O)-, -C(=S)-, -CH₂- or -C(=CR₁R₂)- wherein R₁ and R₂ are each independently of the other hydrogen or lower alkyl, and
R is hydrogen, lower alkyl; phenyl-lower alkyl, phenyl, 1-naphthyl or 2-naphthyl, in the four last-mentioned radicals the phenyl or naphthyl group being unsubstituted or substituted by lower alkyl, lower alkoxy, hydroxy, halogen and/or by trifluoromethyl; or R is amino, hydroxy or lower alkoxy; with the proviso that R is other than phenyl when A₁ and A₂ are hydrogen, Ar₁ and Ar₂ are phenyl and the group -C(=X)- is -C(=O)-; or a salt thereof when salt-forming groups are present; in the above-mentioned radicals "lower" representing "C₁-C₇-" and "-lower" representing "-C₁-C₇-",
wherein the appropriately substituted starting materials are used.

3. A process according to claim 2 for the preparation of a compound of formula I according to claim 1 wherein R is as defined except for phenyl, and the other radicals are as defined, or a salt thereof when salt-forming groups are present, wherein the appropriately substituted starting materials are used.

4. A process according to claim 1 for the preparation of a compound of formula I according to claim 1 wherein
A₁ and A₂ are each independently of the other hydrogen, lower alkyl; phenyl or 1-naphthyl or 2-naphthyl, the three last-mentioned radicals being unsubstituted or substituted by lower alkyl, lower alkoxy, hydroxy, halogen and/or by trifluoromethyl; lower alkanoyl, lower alkylsulfonyl or phenylsulfonyl wherein the phenyl group is unsubstituted or substituted by lower alkyl, lower alkoxy, hydroxy, halogen and/or by trifluoromethyl; or wherein
A₁ and A₂ together are C₁-C₄alkylene; wherein
Ar₁ and Ar₂ are each independently of the other phenyl or naphthyl, each of which is unsubstituted or substituted by one or more substituents from the group consisting of: lower alkyl, C₂-C₇alkenyl, C₂-C₇alkynyl, C₃-C₄alkylene (linked to two adjacent carbon atoms), C₃-C₈cycloalkyl, phenyl-lower alkyl, phenyl; lower alkyl substituted by hydroxy, lower alkoxy, phenyl-lower alkoxy, lower alkanoyloxy, halogen, amino, lower alkylamino, di-lower alkylamino, mercapto, lower alkylthio, lower alkylsulfinyl, lower alkylsulfonyl, carboxy, lower alkoxycarbonyl, carbamoyl, N-lower alkylcarbamoyl, N,N-di-lower alkylcarbamoyl and/or by cyano; hydroxy, lower alkoxy, halo-lower alkoxy, phenyl-lower alkoxy, phenoxy, C₂-C₇alkenyloxy, halo-C₂-C₇alkenyloxy, C₂-C₇alkynyloxy, C₁-C₂alkylenedioxy (linked to two adjacent carbon atoms), lower alkanoyloxy, phenyl-lower alkanoyloxy, phenylcarbonyloxy, mercapto, lower alkylthio, phenyl-lower alkylthio, phenylthio, lower alkylsulfinyl, phenyl-lower alkylsulfinyl, phenylsulfinyl, lower alkylsulfonyl, phenyl-lower alkylsulfonyl, phenylsulfonyl, halogen, nitro, amino, lower alkylamino, C₃-C₈cycloalkylamino, phenyl-lower alkylamino, phenylamino, di-lower alkylamino, N-lower alkyl-N-phenylamino, N-lower alkyl-N-phenyl-lower alkylamino, C₄-C₇alkyleneamino, C₄-C₇alkyleneamino interrupted by -O-, -S- or -NR˝- (wherein R˝ is hydrogen, lower alkyl or lower alkanoyl), lower alkanoylamino, phenyl-lower alkanoylamino, phenylcarbonylamino, lower alkanoyl, phenyl-lower alkanoyl, phenylcarbonyl, carboxy, lower alkoxycarbonyl, carbamoyl, N-lower alkylcarbamoyl, N,N-di-lower alkylcarbamoyl, N-hydroxycarbamoyl, N-phenylcarbamoyl, cyano, sulfo, lower alkoxysulfonyl, sulfamoyl, N-lower alkylsulfamoyl, N,N-di-lower alkylsulfamoyl and N-phenylsulfamoyl (phenyl groups occurring in the substituents each being unsubstituted or substituted by lower alkyl, lower alkoxy, hydroxy, halogen and/or by trifluoromethyl); pyridyl that is unsubstituted or substituted by lower alkyl, hydroxy, lower alkoxy, halogen, cyano and/or by trifluoromethyl; or C₃-C₈cycloalkyl;
the group -C(=X)- is -C(=O)-, -C(=S)-, -CH₂- or -C(=CR₁R₂)- wherein R₁ and R₂ are each independently of the other hydrogen or lower alkyl, and
R is hydrogen, lower alkyl; phenyl-lower alkyl, phenyl, 1-naphthyl or 2-naphthyl, in the four last-mentioned radicals the phenyl or naphthyl group being unsubstituted or substituted by lower alkyl, lower alkoxy, hydroxy, halogen and/or by trifluoromethyl; or R is amino, hydroxy or lower alkoxy; with the proviso that R is other than phenyl when A₁ and A₂ are hydrogen, Ar₁ and Ar₂ are phenyl and the group -C(=X)- is -C(=O)-; or a salt thereof when salt-forming groups are present; in the above-mentioned radicals "lower" representing "C₁-C₇-" and "-lower" representing "-C₁-C₇-", wherein the appropriately substituted starting materials are used.

5. A process according to claim 4 for the preparation of a compound of formula I wherein A₁ and A₂ are each independently of the other hydrogen or lower alkyl, the group -C(=X)- is -C(=O)-, -C(=S)- or -C(=CH₂)-, and R is hydrogen or lower alkyl; or a salt thereof when salt-forming groups are present; in the above-mentioned radicals "lower" representing "C₁-C₇-", wherein the appropriately substituted starting materials are used.

6. A process according to claim 1 for the preparation of a compound of formula I wherein A₁ and A₂ are each independently of the other hydrogen; lower alkyl; C₂-C₇alkenyl; or lower alkanoyl; or wherein A₁ and A₂ together are C₁-C₄alkylene; wherein Ar₁ and Ar₂ are each independently of the other phenyl or naphthyl, each of which is unsubstituted or substituted by one or more substituents from the group consisting of: lower alkyl, C₃-C₄-alkylene (linked to two adjacent carbon atoms), hydroxy, phenoxy, halogen, nitro, amino, lower alkylamino, di-lower alkylamino, lower alkanoylamino, carboxy, lower alkoxycarbonyl, carbamoyl, N-lower alkylcarbamoyl, N,N-di-lower alkylcarbamoyl and cyano; pyridyl; pyrimidinyl; or C₃-C₈cycloalkyl; the group -C(=X)- is -C(=O)- or -C(=S)-; and R is hydrogen, lower alkyl, phenyl-lower alkyl, amino or hydroxy; or a salt thereof when salt-forming groups are present; in the above-mentioned radicals "lower" representing "C₁-C₇" and "-lower" representing "-C₁-C₇-", wherein the appropriately substituted starting materials are used.

7. A process according to claim 1 for the preparation of a compound of formula I according to claim 1 wherein A₁ and A₂ are each independently of the other hydrogen or methyl; or wherein A₁ and A₂ together are -(CH₂)₂- or -(CH₂)₃-; Ar₁ and Ar₂ are each independently of the other phenyl or naphthyl, each of which is unsubstituted or substituted by one or more substituents from the group consisting of: lower alkyl, lower alkoxy, phenyl-lower alkoxy, hydroxy, lower alkanoyloxy, nitro, amino, lower alkylamino, di-lower alkylamino, lower alkanoylamino, halogen, trifluoromethyl, carboxy, lower alkoxycarbonyl, carbamoyl, N-lower alkylcarbamoyl, N,N-di-lower alkylcarbamoyl, cyano, lower alkanoyl, benzoyl, lower alkoxysulfonyl and sulfamoyl, N-lower alkylsulfamoyl and N,N-di-lower alkylsulfamoyl; or cyclopentyl; cyclohexyl; or pyridyl; the group -C(=X)- is -C(=O)-, -C(=S)- or -C(=CH₂)-, and R is hydrogen or lower alkyl; or a pharmaceutically acceptable salt thereof; in the above-mentioned radicals "lower" representing "C₁-C₇-" and "-lower" representing "-C₁-C₇-", wherein the appropriately substituted starting materials are used.

8. A process according to claim 1 for the preparation of a compound of formula I according to claim 1 wherein A₁ and A₂ are hydrogen; Ar₁ and Ar₂ are each independently of the other phenyl that is unsubstituted or substituted by lower alkyl, trifluoromethyl, phenyl, hydroxy, lower alkoxy, benzyloxy, amino, di-lower alkylamino, lower alkanoylamino, halogen, carboxy, lower alkoxycarbonyl, carbamoyl, N,N-di-lower alkylcarbamoyl or by cyano; or cyclohexyl; the group -C(=X)- is -C(=O)-, -C(=S)- or -C(=CH₂)-, and R is hydrogen; or a pharmaceutically acceptable salt thereof; in the above-mentioned radicals "lower" representing "C₁-C₇-" and "-lower" representing "-C₁-C₇-", wherein the appropriately substituted starting materials are used.

9. A process according to claim 1 for the preparation of 4,5-bis(anilino)phthalimide according to claim 1, wherein the appropriately substituted starting materials are used.

10. A process according to claim 1 for the preparation of 4,5-bis(4-fluoroanilino)phthalimide according to claim 1, wherein the appropriately substituted starting materials are used.

11. A process according to claim 1 for the preparation of 4,5-bis(cyclohexylamino)phthalimide according to claim 1 or a pharmaceutically acceptable salt thereof, wherein the appropriately substituted starting materials are used.

12. A process according to claim 1 for the preparation of 5,8-diphenyl-5,8-diaza-5,6,7,8-tetrahydronaphthalene-2,3-dicarboxylic acid amide according to claim 1, wherein the appropriately substituted starting materials are used.

13. A process according to claim 1 for the preparation of 4,5-bis(2-nitroanilino)phthalimide according to claim 1, wherein the appropriately substituted starting materials are used.

14. A process according to claim 1 for the preparation of 4,5-bis(4-aminoanilino)phthalimide according to claim 1 or a pharmaceutically acceptable salt thereof, wherein the appropriately substituted starting materials are used.

15. A process according to claim 1 for the preparation of 4,5-bis(2-pyridineamino)phthalimide according to claim 1 or a pharmaceutically acceptable salt thereof, wherein the appropriately substituted starting materials are used.

16. A process according to claim 1 for the preparation of 4,5-bis(2-pyrimidineamino)-phthalimide according to claim 1 or a pharmaceutically acceptable salt thereof, wherein the appropriately substituted starting materials are used.

17. A process according to claim 1 for the preparation of 4-(N-methyl-N-phenylamino)-5-anilinophthalimide according to claim 1, wherein the appropriately substituted starting materials are used.

18. A process according to claim 1 for the preparation of 4,5-bis(anilino)-N⁴,N⁵-propane-1,3-diyl-phthalimide according to claim 1, wherein the appropriately substituted starting materials are used.

19. A process according to claim 1 for the preparation of 4,5-bis(N-allylanilino)phthalimide according to claim 1, wherein the appropriately substituted starting materials are used.

20. A process for the preparation of a pharmaceutical composition comprising a compound of formula I prepared according to any one of claims 1 to 19, or a pharmaceutically acceptable salt thereof when salt-forming groups are present, wherein at least one pharmaceutically acceptable carrier is added to the compound of formula I.

## Revendications (Revendications pour l'(les) Etat(s) contractant(s) suivant(s): AT, BE, CH, DE, DK, FR, GB, IT, LI, LU, MC, NL, PT, SE)

1. Composés de formule I 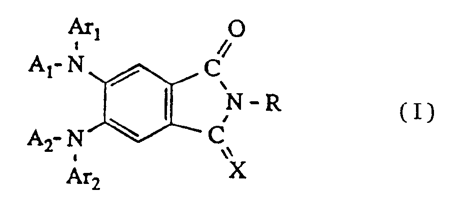 dans laquelle A₁ et A₂ représentent chacun, indépendamment l'un de l'autre, l'hydrogène, un groupe alkyle en C 1-C 7, alcényle en C 2-C 7, alcynyle en C 2-C 7, phényle ou naphtyle, non substitué ou portant un ou plusieurs substituants choisis parmi les groupes alkyle en C 1-C 7, alcényle en C 2-C 7, alcynyle en C 2-C 7, alkylène en C 3-C 4 (relié à 2 atomes de carbone voisins), cycloalkyle en C 3-C 8, phényl-alkyle en C 1-C 7, phényle, alkyle en C 1-C 7 substitué par des groupes hydroxy, alcoxy en C 1-C 7, phényl-alcoxy en C 1-C 7, alcanoyloxy en C 1-C 7, des halogènes, des groupes amino, alkylamino en C 1-C 7, di-(alkyle en C 1-C 7)-amino, mercapto, alkylthio en C 1-C 7, alkylsulfinyle en C 1-C 7, alkylsulfonyle en C 1-C 7, carboxy, (alcoxy en C 1-C 7)-carbonyle, carbamoyle, N-(alkyle en C 1-C 7)-carbamoyle, N,N-di-(alkyle en C 1-C 7)-carbamoyle et/ou cyano; les groupes hydroxy, alcoxy en C 1-C 7, halogénoalcoxy en C 1-C 7, phényl-alcoxy en C 1-C 7, phényloxy, alcényloxy en C 2-C 7, halogénoalcényloxy en C 2-C 7, alcynyloxy en C 2-C 7, (alkylène en C 1-C 2)-dioxy (relié à 2 atomes de carbone voisins), alcanoyloxy en C 1-C 7, phényl-alcanoyloxy en C 1-C 7, phénylcarbonyloxy, mercapto, alkylthio en C 1-C 7, phényl-alkylthio en C 1-C 7, phénylthio, alkylsulfinyle en C 1-C 7, phénylalkylsulfinyle en C 1-C 7, phénylsulfinyle, alkylsulfonyle en C 1-C 7, phényl-alkylsulfonyle en C 1-C 7, phénylsulfonyle, les halogènes, les groupes nitro, amino, alkylamino en C 1-C 7, cycloalkylamino en C 3-C 8, phényl-alkylamino en C 1-C 7, phénylamino, di-(alkyle en C 1-C 7)-amino, N-(alkyle en C 1-C 7)-N-phénylamino, N-(alkyle en C 1-C 7)-N-phénylalkylamino en C 1-C 7, (alkylène en C 4-C 7)-amino, (alkylène en C 4-C 7)-amino interrompu par -O-, -S- ou -NR˝- (dans lequel R˝ représente l'hydrogène, un groupe alkyle en C 1-C 7 ou alcanoyle en C 1-C 7), alcanoylamino en C 1-C 7, phényl-alcanoylamino en C 1-C 7, phénylcarbonylamino, alcanoyle en C 1-C 7, phényl-alcanoyle en C 1-C 7, phénylcarbonyle, carboxy, (alcoxy en C 1-C 7)-carbonyle, carbamoyle, (alkyle en C 1-C 7)-carbamoyle, N,N-di-(alkyle en C 1-C 7)-carbamoyle, N-hydroxycarbamoyle, N-phénylcarbamoyle, cyano, sulfo, alcoxysulfonyle en C 1-C 7, sulfamoyle, N-(alkyle en C 1-C 7)-sulfamoyle, N,N-di-(alkyle en C 1-C 7)-sulfamoyle ou N-phénylsulfamoyle, les groupes phényle existant dans les substituants pouvant chacun être non substitué ou substitué par des groupes alkyle en C 1-C 7, alcoxy en C 1-C 7, hydroxy, des halogènes et/ou des groupes trifluorométhyle; un groupe alcanoyle en C 1-C 7, halogénoalcanoyle en C 1-C 7, phényl- ou naphtylalcanoyle en C 1-C 7 non substitué ou substitué dans la partie phényle ou naphtyle comme on vient de l'indiquer, ou bien phényl- ou naphtyl-carbonyle non substitué ou substitué comme on vient de l'indiquer dans la partie phényle ou naphtyle, alkylsulfonyle en C 1-C 7, ou phényl- ou naphtyl-sulfonyle, dans lequel la partie phényle ou naptyle est non substituée ou substituée comme on vient de l'indiquer; ou bien A₁ et A₂ forment ensemble un groupe alkylène en C 1-C 4 non substitué ou substitué par un groupe alkyle en C 1-C 7 ou hydroxy; Ar₁ et Ar₂ représentent chacun, indépendamment l'un de l'autre, un groupe phényle ou naphtyle non substitué ou substitué comme on vient de l'indiquer; un groupe imidazolyle, triazolyle, pyridyle, pyrimidinyle ou triazinyle, non substitué ou substitué par des groupes alkyle en C 1-C 7, hydroxy, alcoxy en C 1-C 7, des halogènes, des groupes cyano et/ou trifluorométhyle; ou bien un groupe cycloalkyle en C 3-C 8 non substitué ou substitué par des groupes alkyle en C 1-C 7 ou hydroxy, le groupe -C(=X)- représente -C(=O)-, -C(=S)-, -CH₂- ou -C(=CR₁R₂)- dans lequel R₁ et R₂ représentent chacun, indépendamment l'un de l'autre, l'hydrogène ou un groupe alkyle en C 1-C 7, et R représente l'hydrogène, un groupe alkyle en C 1-C 7, aryl-alkyle en C 1-C 7 ou aryle, lequel consiste en un groupe phényle ou naphtyle non substitué ou substitué comme indiqué ci-dessus, un groupe amino, hydroxy ou alcoxy en C 1-C 7, sous réserve que R ne peut représenter un groupe phényle lorsque A₁ et A₂ représentent l'hydrogène, Ar₁ et Ar₂ des groupes phényle et le groupe -C(=X)- le groupe -C(=O)-, et leurs sels lorsqu'il y a des groupes salifiables.

2. Composés de formule I selon revendication 1, dans laquelle A₁ et A₂ représentent chacun, indépendamment l'un de l'autre, l'hydrogène, un groupe alkyle inférieur; un groupe alcényle en C 2-C 7; un groupe phényle ou 1- ou 2-naphtyle, ces trois derniers groupes pouvant être non substitués ou substitués par des groupes alkyle inférieur, alcoxy inférieur, hydroxy, des halogènes et/ou des groupes trifluorométhyle; un groupe alcanoyle inférieur, alkylsulfonyle inférieur, phénylsulfonyle dans lequel le groupe phényle est non substitué ou substitué par des groupes alkyle inférieur, alcoxy inférieur, hydroxy, des halogènes et/ou des groupes trifluorométhyle; ou bien A₁ et A₂ forment ensemble un groupe alkylène en C 1-C 4; dans laquelle Ar₁ et Ar₂ représentent chacun, indépendamment l'un de l'autre, un groupe phényle ou naphtyle non substitué ou portant un ou plusieurs substituants choisis parmi les groupes alkyle inférieur, alcényle en C 2-C 7, alcynyle en C 2-C 7, alkylène en C 3-C 4 (relié à deux atomes de carbone voisins), cycloalkyle en C 3-C 8, phényl-alkyle inférieur, phényle; alkyle inférieur substitué par des groupes hydroxy, alcoxy inférieur, phényl-alcoxy inférieur, alcanoyloxy inférieur, des halogènes, des groupes amino, alkylamino inférieur, di-(alkyle inférieur)-amino, mercapto, alkylthio inférieur, alkylsulfinyle inférieur, alkylsulfonyle inférieur, carboxy, (alcoxy inférieur)-carbonyle, carbamoyle, N-(alkyle inférieur)-carbamoyle, N,N-di-(alkyle inférieur)-carbamoyle et/ou cyano; hydroxy, alcoxy inférieur, halogénoalcoxy inférieur, phényl-alcoxy inférieur, phényloxy, alcényloxy en C 2-C 7, halogénoalcényloxy en C 2-C 7, alcynyloxy en C 2-C 7, (alkylène en C 1-C 2)-dioxy (relié à deux atomes de carbone voisins), alcanoyloxy inférieur, phényl-alcanoyloxy inférieur, phénylcarbonyloxy, mercapto, alkylthio inférieur, phényl-alkylthio inférieur, phénylthio, alkylsulfinyle inférieur, phényl-alkylsulfinyle inférieur, phénylsulfinyle, alkylsulfonyle inférieur, phényl-alkylsulfonyle inférieur, phénylsulfonyle, des halogènes, des groupes nitro, amino, alkylamino inférieur, cycloalkylamino en C 3-C 8, phényl-alkylamino inférieur, phénylamino, di-(alkyle inférieur)-amino, N-(alkyle inférieur)-N-phénylamino, N-(alkyle inférieur)-N-phényl-alkylamino inférieur, (alkylène en C 4-C 7)-amino, (alkylène en C 4-C 7)-amino interrompu par -O-, -S- ou -NR˝- (dans lequel R˝ représente l'hydrogène, un groupe alkyle inférieur ou alcanoyle inférieur), alcanoylamino inférieur, phényl-alcanoylamino inférieur, phénylcarbonylamino, alcanoyle inférieur, phényl-alcanoyle inférieur, phénylcarbonyle, carboxy, (alcoxy inférieur)-carbonyle, carbamoyle, N-(alkyle inférieur)-carbamoyle, N,N-di-(alkyle inférieur)-carbamoyle, N-hydroxycarbamoyle, N-phénylcarbamoyle, cyano, sulfo, alcoxysulfonyle inférieur, sulfamoyle, N-(alkyle inférieur)-sulfamoyle, N,N-di-(alkyle inférieur)-sulfamoyle et N-phénylsulfamoyle; les groupes phényle existant dans les substituants pouvant eux-mêmes être non substitués ou substitués par des groupes alkyle inférieur, alcoxy inférieur, hydroxy, des halogènes et/ou des groupes trifluorométhyle; un groupe pyridyle ou pyrimidinyle, non substitué ou substitué par des groupes alkyle inférieur, hydroxy, alcoxy inférieur, des halogènes, des groupes cyano et/ou trifluorométhyle; ou bien cycloalkyle en C 3-C 8; le groupe -C(=X)- représente -C(=O)-, -C(=S)-, CH₂- ou -C(=CR₁R₂)- dans lequel R₁ et R₂ représentent chacun, indépendamment l'un de l'autre, l'hydrogène ou un groupe alkyle inférieur, et R représente l'hydrogène, un groupe alkyle inférieur; phényl-alkyle inférieur, phényle, 1- ou 2-naphtyle, les groupes phényle et naphtyle de ces quatre derniers substituants pouvant eux-mêmes être non substitués ou substitués par des groupes alkyle inférieur, alcoxy inférieur, hydroxy, des halogènes et/ou des groupes trifluorométhyle; amino, hydroxy ou alcoxy inférieur, sous réserve que R ne peut représenter un groupe phényle lorsque A₁ et A₂ représentent l'hydrogène, Ar₁ et Ar₂ des groupes phényle et le groupe -C(=X)- un groupe -C(=O)-, et leurs sels lorsqu'ils ont des groupes salifiables; l'expression inférieur, appliquée aux groupes mentionnés, indiquant qu'ils contiennent de 1 à 7 atomes de carbone.

3. Composés de formule I de la revendication 2, dans laquelle R a les significations indiquées sauf le groupe phényle, et les autres symboles ont les significations indiquées, et leurs sels lorsqu'ils ont des groupes salifiables.

4. Composés de formule I selon revendication 1, dans laquelle A₁ et A₂ représentent chacun, indépendamment l'un de l'autre, l'hydrogène, un groupe alkyle inférieur; un groupe phényle ou 1- ou 2- naphtyle, ces trois derniers groupes pouvant être non substitués ou substitués par des groupes alkyle inférieur, alcoxy inférieur, hydroxy, des halogènes et/ou des groupes trifluorométhyle; un groupe alcanoyle inférieur, alkylsulfonyle inférieur ou phénylsulfonyle dans lequel le groupe phényle est non substitué ou substitué par des groupes alkyle inférieur, alcoxy inférieur, hydroxy, des halogènes et/ou des groupes trifluorométhyle; ou bien A₁ et A₂ forment ensemble un groupe alkylène en C 1-C 4; Ar₁ et Ar₂ représentent chacun, indépendamment l'un de l'autre, un groupe phényle ou naphtyle qui peut être non substitué ou porter un ou plusieurs substituants choisis parmi les groupes alkyle inférieur, alcényle en C 2-C 7, alcynyle en C 2-C 7, alkylène en C 3-C 4 (relié à deux atomes de carbone voisins), cycloalkyle en C 3-C 8, phényl-alkyle inférieur, phényle; alkyle inférieur substitué par des groupes hydroxy, alcoxy inférieur, phényl-alcoxy inférieur, alcanoyloxy inférieur, des halogènes, des groupes amino, alkylamino inférieur, di-(alkyle inférieur)-amino, mercapto, alkylthio inférieur, alkylsulfinyle inférieur, alkylsulfonyle inférieur, carboxy, (alcoxy inférieur)-carbonyle, carbamoyle, N-(alkyle inférieur)-carbamoyle, N,N-di-(alkyle inférieur)-carbamoyle et/ou cyano; hydroxy, alcoxy inférieur, halogénoalcoxy inférieur, phényl-alcoxy inférieur, phényloxy, alcényloxy en C 2-C 7, halogénoalcényloxy en C 2-C 7, alcynyloxy en C 2-C 7, (alkylène en C 1-C 2)-dioxy (relié à deux atomes de carbone voisins), alcanoyloxy inférieur, phényl-alcanoyloxy inférieur, phénylcarbonyloxy, mercapto, alkylthio inférieur, phényl-alkylthio inférieur, phénylthio, alkylsulfinyle inférieur, phényl-alkylsulfinyle inférieur, phénylsulfinyle, alkylsulfonyle inférieur, phényl-alkylsulfonyle inférieur, phénylsulfonyle, les halogènes, les groupes nitro, amino, alkylamino inférieur, cycloalkylamino en C 3-C 8, phényl-alkylamino inférieur, phénylamino, di-(alkyle inférieur)-amino, N-(alkyle inférieur)-N-phénylamino, N-(alkyle inférieur)-N-phényl-alkylamino inférieur, (alkylène en C 4-C 7)-amino, alkylène en C 4-C 7)-amino interrompu par -O-, -S- ou -NR˝- (dans lequel R˝ représente l'hdrogène, un groupe alkyle inférieur ou alcanoyle inférieur), alcanoylamino inférieur, phényl-alcanoylamino inférieur, phénylcarbonylamino, alcanoyle inférieur, phényl-alcanoyle inférieur, phénylcarbonyle, carboxy, (alcoxy inférieur)-carbonyle, carbamoyle, N-(alkyle inférieur)-carbamoyle, N,N-di-(alkyle inférieur)-carbamoyle, N-hydroxycarbamoyle, N-phénylcarbamoyle, cyano, sulfo, alcoxysulfonyle inférieur, sulfamoyle, N-(alkyle inférieur)-sulfamoyle, N,N-di-(alkyle inférieur)-sulfamoyle et N-phénylsulfamoyle; les groupes phényle existant dans les substituants pouvant eux-mêmes être non substitués ou substitués par des groupes alkyle inférieur, alcoxy inférieur, hydroxy, des halogènes et/ou des groupes trifluorométhyle; pyridyle non substitué ou substitué par des groupes alkyle inférieur, hydroxy, alcoxy inférieur, des halogènes, des groupes cyano et/ou trifluorométhyle; ou bien un groupe cycloalkyle en C 3-C 8; le groupe -C(=X)- représente -C(=O)-, -C(=S)-, -CH₂- ou -C(=CR₁R₂)- dans lequel R₁ et R₂ représentent chacun, indépendamment l'un de l'autre, l'hydrogène ou un groupe alkyle inférieur, et R représente l'hydrogène, un groupe alkyle inférieur, phényl-alkyle inférieur, phényle, 1- ou 2-naphtyle, le groupe phényle ou naphtyle de ces quatre derniers substituants pouvant lui-même être non substitué ou substitué par des groupes alkyle inférieur, alcoxy inférieur, hydroxy, des halogènes et/ou des groupes trifluorométhyle; amino, hydroxy ou alcoxy inférieur, sous réserve que R ne peut représenter un groupe phényle lorsque A₁ et A₂ représentent l'hydrogène, Ar₁ et Ar₂ des groupes phényle et le groupe -C(=X)- un groupe -C(=O)-, et leurs sels lorsqu'ils ont des groupes salifiables; l'expression "inférieur" s'appliquant à des groupes en C 1-C 7.

5. Composés de formule I selon revendication 2, dans laquelle A₁ et A₂ représentent chacun, indépendamment l'un de l'autre, l'hydrogène ou un groupe alkyle inférieur, -C(=X)- représente -C(=O)-, -C(=S)- ou -C(=CH₂)- et R représente l'hydrogène ou un groupe alkyle inférieur, et leurs sels lorsqu'ils ont des groupes salifiables; l'expression inférieur s'appliquant à des substituants en C 1-C 7.

6. Composés de formule I selon revendication 1, dans laquelle A₁ et A₂ représentent chacun, indépendamment l'un de l'autre, l'hydrogène; un groupe alkyle inférieur; alcényle en C 2-C 7; ou alcanoyle inférieur; ou bien A₁ et A₂ forment ensemble un groupe alkylène en C 1-C 4; Ar₁ et Ar₂ représentent chacun, indépendamment l'un de l'autre, un groupe phényle ou naphtyle non substitué ou portant un ou plusieurs substituants choisis parmi les groupes alkyle inférieur, alkylène en C 3-C 4 (relié à deux atomes de carbone voisins), hydroxy, phényloxy, les halogènes, les groupes nitro, amino, alkylamino inférieur, di-(alkyle inférieur)-amino, alcanoylamino inférieur, carboxy, (alcoxy inférieur)-carbonyle, carbamoyle, N-(alkyle inférieur)-carbamoyle, N,N-di-(alkyle inférieur)-carbamoyle et cyano; un groupe pyridyle; un groupe pyrimidinyle; ou un groupe cycloalkyle en C 3-C 8; -C(=X)- représente -C(=O)- ou -C(=S)-, et R représente l'hydrogène, un groupe alkyle inférieur; phényl-alkyle inférieur, amino ou hydroxy, et leurs sels lorsqu'ils ont des groupes salifiables; l'expression inférieur s'appliquant à des groupes en C 1-C 7.

7. Composés de formule I selon revendication 1, dans laquelle A₁ et A₂ représentent chacun, indépendamment l'un de l'autre, l'hydrogène ou un groupe méthyle; ou bien A₁ et A₂ forment ensemble un groupe -(CH₂)₂- ou -(CH₂)₃-; Ar₁ et Ar₂ représentent chacun, indépendamment l'un de l'autre, un groupe phényle ou naphtyle qui peut être non substitué ou porter un ou plusieurs substituants choisis parmi les groupes alkyle inférieur, alcoxy inférieur, phénylalcoxy inférieur, hydroxy, alcanoyloxy inférieur, nitro, amino, alkylamino inférieur, di-(alkyle inférieur)-amino, alcanoylamino inférieur, les halogènes, les groupes trifluorométhyle, carboxy, (alcoxy inférieur)-carbonyle, carbamoyle, N-(alkyle inférieur)-carbamoyle, N,N-di-(alkyle inférieur)-carbamoyle, cyano, alcanoyle inférieur, benzoyle, alcoxysulfonyle inférieur et sulfamoyle, N-(alkyle inférieur)-sulfamoyle et N,N-di-(alkyle inférieur)-sulfamoyle; ou bien un groupe cyclopentyle, cyclohexyle ou pyridyle; -C(=X)- représente -C(=O)-, -C(=S)- ou -C(=CH₂)-, et R représente l'hydrogène ou un groupe alkyle inférieur, et leurs sels acceptables pour l'usage pharmaceutique; l'expression inférieur s'appliquant à des groupes en C 1-C 7.

8. Composés de formule I selon revendication 1, dans laquelle A₁ et A₂ représentent l'hydrogène; Ar₁ et Ar₂ représentent chacun, indépendamment l'un de l'autre, un groupe phényle non substitué ou substitué par des groupes alkyle inférieur, trifluorométhyle, phényle, hydroxy, alcoxy inférieur, benzyloxy, amino, di-(alkyle inférieur)-amino, alcanoylamino inférieur, des halogènes, des groupes carboxy, (alcoxy inférieur)-carbonyle, carbamoyle, N,N-di-(alkyle inférieur)-carbamoyle, ou cyano; ou bien un groupe cyclohexyle; -C(=X)-représente -C(=O)-, -C(=S)- ou -C(=CH₂)-, et R représente l'hydrogène, et leurs sels acceptables pour l'usage pharmaceutique; l'expression inférieur s'appliquant à des substituants en C 1-C 7.

9. Le 4,5-bis-(4-fluoranilino)-phtalimide selon revendication 1.

10. Le 4,5-bis-(anilino)-phtalimide selon revendication 1.

11. Le 4,5-bis-(cyclohexylamino)-phtalimide selon revendication 1, ou un sel de ce composé acceptable pour l'usage pharmaceutique.

12. Le 5,8-diphényl-5,8-diaza-5,6,7,8-tétrahydro-naphtalène-2,3-dicarboxamide selon revendication 1.

13. Le 4,5-bis-(2-nitroanilino)-phtalimide selon revendication 1.

14. Le 4,5-bis-(4-aminoanilino)-phtalimide selon revendication 1, ou l'un de ses sels acceptables pour l'usage pharmaceutique.

15. Le 4,5-bis-(2-pyridinamino)-phtalimide selon revendication 1, ou l'un de ses sels acceptables pour l'usage pharmaceutique.

16. Le 4,5-bis-(2-pyrimidinamino)-phtalimide selon revendication 1, ou l'un de ses sels acceptables pour l'usage pharmaceutique.

17. Le 4-(N-méthyl-N-phénylamino)-5-anilino-phtalimide selon revendication 1.

18. Le 4,5-bis-(anilino)-N⁴,N⁵-propane-1,3-diyl-phtalimide selon revendication 1.

19. Le 4,5-bis-(N-allylanilino)-phtalimide selon revendication 1.

20. Compositions pharmaceutiques contenant un composé de formule I selon l'une des revendications 1 à 19 et au moins un véhicule acceptable pour l'usage pharmaceutique.

21. Un composé de formule I selon l'une des revendications 1 à 19, pour l'utilisation dans un procédé pour le traitement thérapeutique de l'organisme humain ou animal.

22. Utilisation d'un composé selon l'une des revendications 1 à 19 pour la préparation de compositions pharmaceutiques pour le traitement de maladies nécessitant une inhibition des protéine-tyrosine-kinases et des sérine/thréonine-kinases.

23. Procédé de préparation d'un composé de formule I selon revendication 1, caractérisé en ce que:
a) on fait réagir un composé de formule II 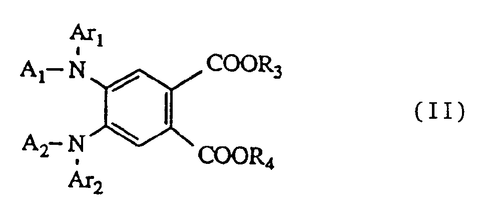 dans laquelle Ar₁, Ar₂, A₁ et A₂ ont les significations indiquées en référence à la formule I, et R₃ et R₄ représentent chacun, indépendamment l'un de l'autre, un groupe aryle ou alkyle en C 1-C 7, avec un composé de formule III
H₂N-R (III)
dans laquelle R a les significations indiquées en référence à la formule I, ou bien
b) on fait réagir un composé de formule IV 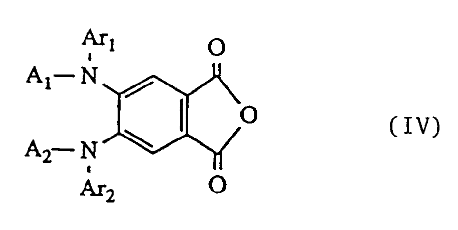 dans laquelle Ar₁, Ar₂, A₁ et A₂ ont les significations indiquées en référence à la formule I, avec un composé de formule III
H₂N-R (III)
dans laquelle R a les significations indiquées en référence à la formule I,
et, si on le désire, on convertit un composé de formule I ainsi obtenu en un autre composé de formule I et/ou un sel ainsi obtenu en le composé libre ou en un autre sel et/ou un composé de formule I obtenu à l'état libre en un sel et/ou on sépare un mélange d'isomères de formule I ainsi obtenu en les isomères individuels.

## Revendications (Revendications pour l'(les) Etat(s) contractant(s) suivant(s): ES, GR)

1. Procédé de préparation d'un composé de formule I 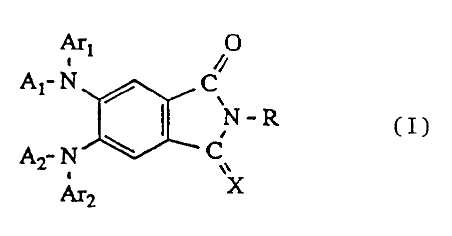 dans laquelle A₁ et A₂ représentent chacun, indépendamment l'un de l'autre, l'hydrogène, un groupe alkyle en C 1-C 7, alcényle en C 2-C 7, alcynyle en C 2-C 7, phényle ou naphtyle, non substitué ou portant un ou plusieurs substituants choisis par les groupes alkyle en C 1-C 7, alcényle en C 2-C 7, alcynyle en C 2-C 7, alkylène en C 3-C 4 (relié à 2 atomes de carbone voisins), cycloalkyle en C 3-C 8, phényl-alkyle en C 1-C 7, phényle, alkyle en C 1-C 7 substitué par des groupes hydroxy, alcoxy en C 1-C 7, phényl-alcoxy en C 1-C 7, alcanoyloxy en C 1-C 7, des halogènes, des groupes amino, alkylamino en C 1-C 7, di-(alkyle en C 1-C 7)-amino, mercapto, alkylthio en C 1-C 7, alkylsulfinyle en C 1-C 7, alkylsulfonyle en C 1-C 7, carboxy, (alcoxy en C 1-C 7)-carbonyle, carbamoyle, N-(alkyle en C 1-C 7)-carbamoyle, N,N-di-(alkyle en C 1-C 7)-carbamoyle et/ou cyano; les groupes hydroxy, alcoxy en C 1-C 7, halogénoalcoxy en C 1-C 7, phényl-alcoxy en C 1-C 7, phényloxy, alcényloxy en C 2-C 7, halogénoalcényloxy en C 2-C 7, alcynyloxy en C 2-C 7, (alkylène en C 1-C 2)-dioxy (relié à 2 atomes de carbone voisins), alcanoyloxy en C 1-C 7, phényl-alcanoyloxy en C 1-C 7, phénylcarbonyloxy, mercapto, alkylthio en C 1-C 7, phényl-alkylthio en C 1-C 7, phénylthio, alkylsulfinyle en C 1-C 7, phényl-alkylsulfinyle en C 1-C 7, phénylsulfinyle, alkylsulfonyle en C 1-C 7, phényl-alkylsulfonyle en C 1-C 7, phénylsulfonyle, les halogènes, les groupes nitro, amino, alkylamino en C 1-C 7, cycloalkylamino en C 3-C 8, phényl-alkylamino en C 1-C 7, phénylamino, di-(alkyle en C 1-C 7)-amino, N-(alkyle en C 1-C 7)-N-phénylamino, N-(alkyle en C 1-C 7)-N-phénylalkylamino en C 1-C 7, (alkylène en C 4-C 7)-amino, (alkylène en C 4-C 7)-amino interrompu par -O- , -S- ou -NR˝- (dans lequel R˝ représente l'hydrogène, un groupe alkyle en C 1-C 7 ou alcanoyle en C 1-C 7), alcanoylamino en C 1-C 7, phényl-alcanoylamino en C 1-C 7, phénylcarbonylamino, alcanoyle en C 1-C 7, phényl-alcanoyle en C 1-C 7, phénylcarbonyle, carboxy, (alcoxy en C 1-C 7)-carbonyle, carbamoyle, (alkyle en C 1-C 7)-carbamoyle, N,N-di-(alkyle en C 1-C 7)-carbamoyle, N-hydroxycarbamoyle, N-phénylcarbamoyle, cyano, sulfo, alcoxysulfonyle en C 1-C 7, sulfamoyle, N-(alkyle en C 1-C 7)-sulfamoyle, N,N-di-(alkyle en C 1-C 7)- sulfamoyle ou N-phénylsulfamoyle, les groupes phényle existant dans les substituants pouvant chacun être non substitué ou substitué par des groupes alkyle en C 1-C 7, alcoxy en C 1-C 7, hydroxy, des halogènes et/ou des groupes trifluorométhyle; un groupe alcanoyle en C 1-C 7, halogénoalcanoyle en C 1-C 7, phényl- ou naphtyl-alcanoyle en C 1-C 7 non substitué ou substitué dans la partie phényle ou naphtyle comme on vient de l'indiquer, ou bien phényl- ou naphtyl-carbonyle non substitué ou substitué comme on vient de l'indiquer dans la partie phényle ou naphtyle, alkylsulfonyle en C 1-C 7, ou phényl- ou naphtyl-sulfonyle, dans lequel la partie phényle ou naphtyle est non substituée ou substitué comme on vient de l'indiquer; ou bien A₁ et A₂ forment ensemble un groupe alkylène en C 1-C 4 non substitué ou substitué par un groupe alkyle en C 1-C 7 ou hydroxy; Ar₁ et Ar₂ représentent chacun, indépendamment l'un de l'autre, un groupe phényle ou naphtyle non substitué ou substitué comme on vient de l'indiquer; un groupe imidazolyle, triazolyle, pyridyle, pyrimidinyle ou triazinyle, non substitué ou substitué par des groupes alkyle en C 1-C 7, hydroxy, alcoxy en C 1-C 7, des halogènes, des groupes cyano et/ou trifluorométhyle; ou bien un groupe cycloalkyle en C 3-C 8 non substitué ou substitué par des groupes alkyle en C 1-C 7 ou hydroxy, le groupe -C(=X)- représente -C(=O)-, -C(=S)-, -CH₂- ou -C(=CR₁R₂)- dans lequel R₁ et R₂ représentent chacun, indépendamment l'un de l'autre, l'hydrogène ou un groupe alkyle en C 1-C 7, et R représente l'hydrogène, un groupe alkyle en C 1-C 7, aryl-alkyle en C 1-C 7 ou aryle, lequel consiste en un groupe phényle ou naphtyle non substitué ou substitué comme indiqué ci-dessus, un groupe amino, hydroxy ou alcoxy en C 1-C 7, sous réserve que R ne peut représenter un groupe phényle lorsque A₁ et A₂ représentent l'hydrogène, Ar₁ et Ar₂ des groupes phényle et le groupe -C(=X)- le groupe -C(=O)-, et leurs sels lorsqu'il y a des groupes salifiables, caractérisé en ce que :
a) on fait réagir un composé de formule II 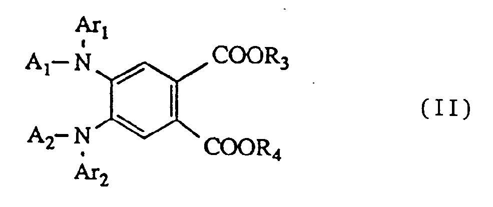 dans laquelle Ar₁, Ar₂, A₁ et A₂ ont les significations indiquées en référence à la formule I, et R₃ et R₄ représentent chacun, indépendamment l'un de l'autre, un groupe aryle ou alkyle en C 1-C 7, avec un composé de formule III
H₂N-R (III)
dans laquelle R a les significations indiquées en référence à la formule I, ou bien
b) on fait réagir un composé de formule IV 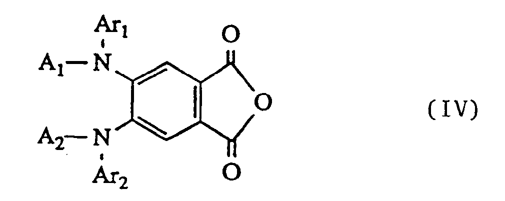 dans laquelle Ar₁, Ar₂, A₁ et A₂ ont les significations indiquées en référence à la formule I, avec un composé de formule III
H₂N-R (III)
dans laquelle R a les significations indiquées en référence à la formule I,
et, si on le désire, on convertit un composé de formule I ainsi obtenu en un autre composé de formule I et/ou un sel ainsi obtenu en le composé libre ou en un autre sel et/ou un composé de formule I obtenu à l'état libre en un sel et/ou on sépare un mélange d'isomères de formule I ainsi obtenu en les isomères individuels.

2. Procédé selon revendication 1, pour la préparation d'un composé de formule I dans laquelle A₁ et A₂ représentent chacun, indépendamment l'un de l'autre, l'hydrogène, un groupe alkyle inférieur; un groupe alcényle en C 2-C 7; un groupe phényle ou 1- ou 2-naphtyle, ces trois derniers groupes pouvant être non substitués ou substitués par des groupes alkyle inférieur, alcoxy inférieur, hydroxy, des halogènes et/ou des groupes trifluorométhyle; un groupe alcanoyle inférieur, alkylsulfonyle inférieur, phénylsulfonyle dans lequel le groupe phényle est non substitué ou substitué par des groupes alkyle inférieur, alcoxy inférieur, hydroxy, des halogènes et/ou des groupes trifluorométhyle; ou bien A₁ et A₂ forment ensemble un groupe alkylène en C 1-C 4; dans laquelle Ar₁ et Ar₂ représentent chacun, indépendamment l'un de l'autre, un groupe phényle ou naphtyle non substitué ou portant un ou plusieurs substituants choisis parmi les groupes alkyle inférieur, alcényle en C 2-C 7, alcynyle en C 2-C 7, alkylène en C 3-C 4 (relié à deux atomes de carbone voisins), cycloalkyle en C 3-C 8, phényl-alkyle inférieur, phényle; alkyle inférieur substitué par des groupes hydroxy, alcoxy inférieur, phényl-alcoxy inférieur, alcanoyloxy inférieur, des halogènes, des groupes amino, alkylamino inférieur, di-(alkyle inférieur)-amino, mercapto, alkylthio inférieur, alkylsulfinyle inférieur, alkylsulfonyle inférieur, carboxy, (alcoxy inférieur)-carbonyle, carbamoyle, N-(alkyle inférieur)-carbamoyle, N,N-di-(alkyle inférieur)-carbamoyle et/ou cyano; hydroxy, alcoxy inférieur, halogénoalcoxy inférieur, phényl-alcoxy inférieur, phényloxy, alcényloxy en C 2-C 7, halogénoalcényloxy en C 2-C 7, alcynyloxy en C 2-C 7, (alkylène en C 1-C 2)-dioxy (relié à deux atomes de carbone voisins), alcanoyloxy inférieur, phényl-alcanoyloxy inférieur, phénylcarbonyloxy, mercapto, alkylthio inférieur, phényl-alkylthio inférieur, phénylthio, alkylsulfinyle inférieur, phényl-alkylsulfinyle inférieur, phénylsulfinyle, alkylsulfonyle inférieur, phényl-alkylsulfonyle inférieur, phénylsulfonyle, des halogènes, des groupes nitro, amino, alkylamino inférieur, cycloalkylamino en C 3-C 8, phényl-alkylamino inférieur, phénylamino, di-(alkyle inférieur)-amino, N-(alkyle inférieur)-N-phénylamino, N-(alkyle inférieur)-N-phényl-alkylamino inférieur, (alkylène en C 4-C 7)-amino, (alkylène en C 4-C 7)-amino interrompu par -O-, -S- ou -NR˝- (dans lequel R˝ représente l'hydrogène, un groupe alkyle inférieur ou alcanoyle inférieur), alcanoylamino inférieur, phényl-alcanoylamino inférieur, phénylcarbonylamino, alcanoyle inférieur, phényl-alcanoyle inférieur, phénylcarbonyle, carboxy, (alcoxy inférieur)-carbonyle, carbamoyle, N-(alkyle inférieur)-carbamoyle, N,N-di-(alkyle inférieur)-carbamoyle, N-hydroxycarbamoyle, N-phénylcarbamoyle, cyano, sulfo, alcoxysulfonyle inférieur, sulfamoyle, N-(alkyle inférieur)-sulfamoyle, N,N-di-(alkyle inférieur)-sulfamoyle et N-phénylsulfamoyle; les groupes phényle existant dans les substituants pouvant eux-mêmes être non susbtitués ou substitués par des groupes alkyle inférieur, alcoxy inférieur, hydroxy, des halogènes et/ou des groupes trifluorométhyle; un groupe pyridyle ou pyrimidinyle, non substitué ou substitué par des groupes alkyle inférieur, hydroxy, alcoxy inférieur, des halogènes, des groupes cyano et/ou trifluorométhyle; ou bien cycloalkyle en C 3-C 8; le groupe -C(=X)- représente -C(=O)-, -C(=S)-, -CH₂- ou -C(=CR₁R₂)- dans lequel R₁ et R₂ représentent chacun, indépendamment l'un de l'autre, l'hydrogène ou un groupe alkyle inférieur, et R représente l'hydrogène, un groupe alkyle inférieur; phényl-alkyle inférieur, phényle, 1- ou 2-naphtyle, les groupes phényle et naphtyle de ces quatre derniers substituants pouvant eux-mêmes être non substitués ou substitués par des groupes alkyle inférieur, alcoxy inférieur, hydroxy, des halogènes et/ou des groupes trifluorométhyle; amino, hydroxy ou alcoxy inférieur, sous réserve que R ne peut représenter un groupe phényle lorsque A₁ et A₂ représentent l'hydrogène, Ar₁ et Ar₂ des groupes phényle et le groupe -C(=X)- un groupe -C(=O)-, et leurs sels lorsqu'ils ont des groupes salifiables; l'expression inférieur, appliquée aux groupes mentionnés, indiquant qu'ils contiennent de 1 à 7 atomes de carbone; caractérisé en ce que l'on utilise les produits de départ portant les substituants correspondants.

3. Procédé selon revendication 2, pour préparer un composé de formule I selon revendication 1 dans laquelle R a les significations indiquées sauf le groupe phényle, et les autres substituants ont les significations indiquées, et de leurs sels lorsqu'ils ont des groupes salifiables, caractérisé en ce que l'on utilise les produits de départ portant les substituants correspondants.

4. Procédé selon revendication 1, pour la préparation d'un composé de formule I selon revendication 1, dans laquelle A₁ et A₂ représentent chacun, indépendamment l'un de l'autre, l'hydrogène, un groupe alkyle inférieur; un groupe phényle ou 1- ou 2-naphtyle, ces trois derniers groupes pouvant être non substitués ou substitués par des groupes alkyle inférieur, alcoxy inférieur, hydroxy, des halogènes et/ou des groupes trifluorométhyle; un groupe alcanoyle inférieur, alkylsulfonyle inférieur ou phénylsulfonyle dans lequel le groupe phényle est non substitué ou substitué par des groupes alkyle inférieur, alcoxy inférieur, hydroxy, des halogènes et/ou des groupes trifluorométhyle; ou bien A₁ et A₂ forment ensemble un groupe alkylène en C 1-C 4; Ar₁ et Ar₂ représentent chacun, indépendamment l'un de l'autre, un groupe phényle ou naphtyle qui peut être non substitué ou porter un ou plusieurs substituants choisis parmi les groupes alkyle inférieur, alcényle en C 2-C 7, alcynyle en C 2-C 7, alkylène en C 3-C 4 (relié à deux atomes de carbone voisins), cycloalkyle en C 3-C 8, phényl-alkyle inférieur, phényle; alkyle inférieur substitué par des groupes hydroxy, alcoxy inférieur, phényl-alcoxy inférieur, alcanoyloxy inférieur, des halogènes, des groupes amino, alkylamino inférieur, di-(alkyle inférieur)-amino, mercapto, alkylthio inférieur, alkylsulfinyle inférieur, alkylsulfonyle inférieur, carboxy, (alcoxy inférieur)-carbonyle, carbamoyle, N-(alkyle inférieur)-carbamoyle, N,N-di-(alkyle inférieur)-carbamoyle et/ou cyano; hydroxy, alcoxy inférieur, halogénoalcoxy inférieur, phényl-alcoxy inférieur, phényloxy, alcényloxy en C 2-C 7, halogénoalcényloxy en C 2-C 7, alcynyloxy en C 2-C 7, (alkylène en C 1-C 2)-dioxy (relié à deux atomes de carbone voisins), alcanoyloxy inférieur, phényl-alcanoyloxy inférieur, phénylcarbonyloxy, mercapto, alkylthio inférieur, phényl-alkylthio inférieur, phénylthio, alkylsulfinyle inférieur, phényl-alkylsulfinyle inférieur, phénylsulfinyle, alkylsulfonyle inférieur, phényl-alkylsulfonyle inférieur, phénylsulfonyle, les halogènes, les groupes nitro, amino, alkylamino inférieur, cycloalkylamino en C 3-C 8, phényl-alkylamino inférieur, phénylamino, di-(alkyle inférieur)-amino, N-(alkyle inférieur)-N-phénylamino, N-(alkyle inférieur)-N-phényl-alkylamino inférieur, (alkylène en C 4-C 7)-amino, alkylène en C 4-C 7)-amino interrompu par -O-, -S- ou -NR˝- (dans lequel R˝ représente l'hydrogène, un groupe alkyle inférieur ou alcanoyle inférieur), alcanoylamino inférieur, phényl-alcanoylamino inférieur, phénylcarbonylamino, alcanoyle inférieur, phényl-alcanoyle inférieur, phénylcarbonyle, carboxy, (alcoxy inférieur)-carbonyle, carbamoyle, N-(alkyle inférieur)-carbamoyle, N,N-di-(alkyle inférieur)-carbamoyle, N-hydroxycarbamoyle, N-phénylcarbamoyle, cyano, sulfo, alcoxysulfonyle inférieur, sulfamoyle, N-(alkyle inférieur)-sulfamoyle, N,N-di-(alkyle inférieur)-sulfamoyle et N-phénylsulfamoyle; les groupes phényle existant dans les substituants pouvant eux-mêmes être non substitués ou substitués par des groupes alkyle inférieur, alcoxy inférieur, hydroxy, des halogènes et/ou des groupes trifluorométhyle; pyridyle non substitué ou substitué par des groupes alkyle inférieur, hydroxy, alcoxy inférieur, des halogènes, des groupes cyano et/ou trifluorométhyle; ou bien un groupe cycloalkyle en C 3-C 8; le groupe -C(=X)- représente -C(=O)-, C(=S)-, -CH₂- ou -C(=CR₁R₂)- dans lequel R₁ et R₂ représentent chacun, indépendamment l'un de l'autre, l'hydrogène ou un groupe alkyle inférieur, et R représente l'hydrogène, un groupe alkyle inférieur, phényl-alkyle inférieur, phényle, 1- ou 2-naphtyle, le groupe phényle ou naphtyle de ces quatre derniers substituants pouvant lui-même être non substitué ou substitué par des groupes alkyle inférieur, alcoxy inférieur, hydroxy, des halogènes et/ou des groupes trifluorométhyle; amino, hydroxy ou alcoxy inférieur, sous réserve que R ne peut représenter un groupe phényle lorsque A₁ et A₂ représentent l'hydrogène, Ar₁ et Ar₂ des groupes phényle et le groupe -C(=X)- un groupe -C(=O)-, et leurs sels lorsqu'ils ont des groupes salifiables; l'expression "inférieur" s'appliquant à des groupes en C 1-C 7, caractérisé en ce que l'on utilise les produits de départ portant les substituants correspondants.

5. Procédé selon revendication 4, pour la préparation d'un composé de formule I dans laquelle A₁ et A₂ représentent chacun, indépendamment l'un de l'autre, l'hydrogène ou un groupe alkyle inférieur, -C(=X)- représente -C(=O)-, -C(=S)- ou -C(=CH₂)- et R représente l'hydrogène ou un groupe alkyle inférieur, et leurs sels lorsqu'ils ont des groupes salifiables; l'expression "inférieur" s'appliquant à des substituants en C 1-C 7, caractérisé en ce que l'on utilise les produits de départ portant les substituants correspondants.

6. Procédé selon revendication 1, pour la préparation d'un composé de formule I dans laquelle A₁ et A₂ représentent chacun, indépendamment l'un de l'autre, l'hydrogène; un groupe alkyle inférieur; alcényle en C 2-C 7; ou alcanoyle inférieur; ou bien A₁ et A₂ forment ensemble un groupe alkylène en C 1-C 4; Ar₁ et Ar₂ représentent chacun, indépendamment l'un de l'autre, un groupe phényle ou naphtyle non substitué ou portant un ou plusieurs substituants choisis parmi les groupes alkyle inférieur, alkylène en C 3-C 4 (relié à deux atomes de carbone voisins), hydroxy, phényloxy, les halogènes, les groupes nitro, amino, alkylamino inférieur, di-(alkyle inférieur)-amino, alcanoylamino inférieur, carboxy, (alcoxy inférieur)-carbonyle, carbamoyle, N-(alkyle inférieur)-carbamoyle, N,N-di-(alkyle inférieur)-carbamoyle et cyano; un groupe pyridyle; un groupe pyrimidinyle; ou un groupe cycloalkyle en C 3-C 8; -C(=X)- représente -C(=O)- ou -C(=S)-, et R représente l'hydrogène, un groupe alkyle inférieur; phényl-alkyle inférieur, amino ou hydroxy, et leurs sels lorsqu'ils ont des groupes salifiables; l'expression "inférieur" s'appliquant à des groupes en C 1-C 7, caractérisé en ce que l'on utilise les produits de départ portant les substituants correspondants.

7. Procédé selon revendication 1, pour la préparation d'un composé de formule I selon revendication 1, dans laquelle A₁ et A₂ représentent chacun, indépendamment l'un de l'autre, l'hydrogène ou un groupe méthyle; ou bien A₁ et A₂ forment ensemble un groupe -(CH₂)₂- ou -(CH₂)₃-; Ar₁ et Ar₂ représentent chacun, indépendamment l'un de l'autre, un groupe phényle ou naphtyle qui peut être non substitué ou porter un ou plusieurs substituants choisis parmi les groupes alkyle inférieur, alcoxy inférieur, phényl-alcoxy inférieur, hydroxy, alcanoyloxy inférieur, nitro, amino, alkylamino inférieur, di-(alkyle inférieur)-amino, alcanoylamino inférieur, les halogènes, les groupes trifluorométhyle, carboxy, (alcoxy inférieur)-carbonyle, carbamoyle, N-(alkyle inférieur)-carbamoyle, N,N-di-(alkyle inférieur)-carbamoyle, cyano, alcanoyle inférieur, benzoyle, alcoxysulfonyle inférieur et sulfamoyle, N-(alkyle inférieur)-sulfamoyle et N,N-di-(alkyle inférieur)-sulfamoyle; ou bien un groupe cyclopentyle, cyclohexyle ou pyridyle; -C(=X)- représente -C(=O)-, -C(=S)- ou -C(=CH₂)-, et R représente l'hydrogène ou un groupe alkyle inférieur, et leurs sels acceptables pour l'usage pharmaceutique; l'expression "inférieur" s'appliquant à des groupes en C 1-C 7, caractérisé en ce que l'on utilise les produits de départ portant les substituants correspondants.

8. Procédé selon revendication 1, pour la préparation d'un composé de formule I selon revendication 1, dans laquelle A₁ et A₂ représentent l'hydrogène; Ar₁ et Ar₂ représentent chacun, indépendamment l'un de l'autre, un groupe phényle non substitué ou substitué par des groupes alkyle inférieur, trifluorométhyle, phényle, hydroxy, alcoxy inférieur, benzyloxy, amino, di-(alkyle inférieur)-amino, , alcanoylamino inférieur, des halogènes, des groupes carboxy, (alcoxy inférieur)-carbonyle, carbamoyle, N,N-di-(alkyle inférieur)-carbamoyle, ou cyano; ou bien un groupe cyclohexyle; -C(=X)- représente -C(=O)-, -C(=S)- ou -C(=CH₂)-, et R représente l'hydrogène, et leurs sels acceptables pour l'usage pharmaceutique; l'expression "inférieur" s'appliquant à des substituants en C 1-C 7, caractérisé en ce que l'on utilise les produits de départ portant les substituants correspondants.

9. Procédé selon revendication 1, pour la préparation du 4,5-bis-(anilino)-phtalimide selon revendication 1, caractérisé en ce que l'on utilise les produits de départ portant les substituants correspondants.

10. Procédé selon revendication 1, pour la préparation du 4,5-bis-(4-fluoranilino)-phtalimide selon revendication 1, caractérisé en ce que l'on utilise les composés de départ portant les substituants correspondants.

11. Procédé selon revendication 1, pour la préparation du 4,5-bis-(cyclohexylamino)-phtalimide selon revendication 1 ou de l'un de ses sels acceptables pour l'usage pharmaceutique, caractérisé en ce que l'on utilise les produits de départ portant les substituants correspondants.

12. Procédé selon revendication 1, pour la préparation du 5,8-diphényl-5,8-diaza-5,6,7,8-tétrahydro-naphtalène-2,3-dicarboxamide selon revendication 1, caractérisé en ce que l'on utilise les produits de départ portant les substituants correspondants.

13. Procédé selon revendication 1, pour la préparation du 4,5-bis-(2-nitroanilino)-phtalimide selon revendication 1, caractérisé en ce que l'on utilise les produits de départ portant les substituants correspondants.

14. Procédé selon revendication 1, pour la préparation du 4,5-bis-(4-aminoanilino)-phtalimide selon revendication 1 ou de l'un de ses sels acceptables pour l'usage pharmaceutique, caractérisé en ce que l'on utilise les produits de départ portant les substituants correspondants.

15. Procédé selon revendication 1, pour la préparation du 4,5-bis-(2-pyridinamino)-phtalimide selon revendication 1 ou de l'un de ses sels acceptables pour l'usage pharmaceutique, caractérisé en ce que l'on utilise les produits de départ portant les substituants correspondants.

16. Procédé selon revendication 1, pour la préparation du 4,5-bis-(2-pyrimidinamino)-phtalimide selon revendication 1 ou de l'un de ses sels acceptables pour l'usage pharmaceutique, caractérisé en ce que l'on utilise les prodits de départ portant les substituants correspondants.

17. Procédé selon revendication 1, pour la préparation du 4-(N-méthyl-N-phénylamino)-5-anilino-phtalimide selon revendication 1, caractérisé en ce que l'on utilise les produits de départ portant les substituants correspondants.

18. Procédé selon revendication 1, pour la préparation du 4,5-bis-(anilino)-N⁴,N⁵-propane-1,3-diyl-phtalimide selon revendication 1, caractérisé en ce que l'on utilise les produits de départ portant les substituants correspondants.

19. Procédé selon revendication 1, pour la préparation du 4,5-bis-(N-allylanilino)-phtalimide selon revendication 1, caractérisé en ce que l'on utilise les produits de départ portant les substituants correspondants.

20. Procédé de préparation d'une composition pharmaceutique contenant un composé de formule I préparé selon l'une des revendications 1 à 19 ou l'un de ses sels acceptables pour l'usage pharmaceutique lorsqu'il a des groupes salifiables, caractérisé en ce que l'on ajoute au moins un véhicule acceptable pour l'usage pharmaceutique du composé de formule I.
